(19) **Europäisches Patentamt European Patent Office Office européen des brevets**

(11) **EP 3 167 902 B1**

(12) **EUROPEAN PATENT SPECIFICATION**

(45) Date of publication and mention of the grant of the patent:
**29.07.2020 Bulletin 2020/31**

(51) Int Cl.:
*A61K 39/395* (2006.01)  *A61K 31/635* (2006.01)
*A61K 31/60* (2006.01)  *A61K 31/57* (2006.01)
*A61K 31/52* (2006.01)  *A61P 37/06* (2006.01)

(21) Application number: **16191558.2**

(22) Date of filing: **13.04.2001**

(54) **ANTIBODY BINDING ALPHA4BETA7 INTEGRIN AND ITS USE TO TREAT INFLAMMATORY BOWEL DISEASE**

ALPHA4BETA7-INTEGRIN-BINDENDER ANTIKÖRPER UND DESSEN VERWENDUNG ZUR BEHANDLUNG ENTZÜNDLICHER DARMERKRANKUNGEN

ANTICORPS A LIAISON D'INTEGRINE ALPHA4BETA7 ET SON UTILISATION POUR TRAITER LES MALADIES INTESTINALES INFLAMMATOIRES

(84) Designated Contracting States:
**AT BE CH CY DE DK ES FI FR GB GR IE IT LI LU MC NL PT SE TR**

(30) Priority: **14.04.2000 US 550082**
**27.12.2000 US 748960**

(43) Date of publication of application:
**17.05.2017 Bulletin 2017/20**

(62) Document number(s) of the earlier application(s) in accordance with Art. 76 EPC:
**10010997.4 / 2 298 348**
**01925028.1 / 1 278 543**

(73) Proprietors:
• **Millennium Pharmaceuticals, Inc.**
  **Cambridge, MA 02139 (US)**
• **GENENTECH, INC.**
  **South San Francisco, CA 94080-4990 (US)**

(72) Inventors:
• **BRETTMAN, Lee R**
  **Brookline, MA 02446 (US)**
• **FOX, Judith A.**
  **San Francisco, CA 94115 (US)**
• **ALLISON, David Edward**
  **Sunnyvale, CA 94086 (US)**

(74) Representative: **Hoffmann Eitle**
**Patent- und Rechtsanwälte PartmbB**
**Arabellastraße 30**
**81925 München (DE)**

(56) References cited:
**WO-A-98/06248**    **US-A- 5 834 021**
**US-A- 6 015 662**

• **GORDON FIONA H ET AL: "Treatment of active ulcerative colitis with a recombinant humanised antibody to alpha4 integrin (Antegren(R)).", GASTROENTEROLOGY, vol. 116, no. 4 PART 2, April 1999 (1999-04), page A726, XP001024675, Digestive Disease Week and the 100th Annual Meeting of the American Gastroenterological Association;Orlando, Florida, USA; May 16-19, 1999 ISSN: 0016-5085**
• **GORDON FIONA H ET AL: "Randomised double-blind placebo-controlled trial of recombinant humanised antibody to alpha4 integrin (Antegren(R)) in active Crohn's disease.", GASTROENTEROLOGY, vol. 116, no. 4 PART 2, April 1999 (1999-04), page A726, XP008133112, Digestive Disease Week and the 100th Annual Meeting of the American Gastroenterological Association;Orlando, Florida, USA; May 16-19, 1999 ISSN: 0016-5085**
• **LIN KO-CHUNG ET AL: "Very late antigen 4 (VLA4) antagonists as anti-inflammatory agents.", CURRENT OPINION IN CHEMICAL BIOLOGY, vol. 2, no. 4, August 1998 (1998-08), pages 453-457, XP001024679, ISSN: 1367-5931**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

EP 3 167 902 B1

- GRANT A J ET AL: "MAdCAM-1 expressed in chronic inflammatory liver disease supports mucosal lymphocyte adhesion to hepatic endothelium (MAdCAM-1 in chronic inflammatory liver disease).", HEPATOLOGY (BALTIMORE, MD.) MAY 2001, vol. 33, no. 5, May 2001 (2001-05), pages 1065-1072, ISSN: 0270-9139
- ADAMS DAVID H ET AL: "Aberrant homing of mucosal T cells and extra-intestinal manifestations of inflammatory bowel disease.", NATURE REVIEWS. IMMUNOLOGY MAR 2006, vol. 6, no. 3, March 2006 (2006-03), pages 244-251, ISSN: 1474-1733
- TANNEAU G M ET AL: "Differential recruitment of T- and IgA B-lymphocytes in the developing mammary gland in relation to homing receptors and vascular addressins.", THE JOURNAL OF HISTOCHEMISTRY AND CYTOCHEMISTRY : OFFICIAL JOURNAL OF THE HISTOCHEMISTRY SOCIETY DEC 1999, vol. 47, no. 12, December 1999 (1999-12), pages 1581-1592, ISSN: 0022-1554
- MACKAY C: "Lymphocyte migration. A new spin on lymphocyte homing.", CURRENT BIOLOGY : CB 01 JUL 1995, vol. 5, no. 7, 1 July 1995 (1995-07-01), pages 733-736, ISSN: 0960-9822
- AL-ASHQAR ROQAYA A ET AL: "The CD markers of camel (Camelus dromedarius) milk cells during mastitis: the LPAM-1 expression is an indication of possible mucosal nature of the cellular trafficking.", RESEARCH IN VETERINARY SCIENCE APR 2015, vol. 99, April 2015 (2015-04), pages 77-81, ISSN: 1532-2661

SEQUENCE LISTING

<110> Millennium Pharmaceuticals, Inc.
Genentech, Inc.
Lee R. Brettman
Judith A. Fox
David Edward Allison

<120> Method of Administering an Antibody

<130> 1855.2007003

<140> PCT/US01/12234
<141> 2001-04-13

<150> US 09/748,960
<151> 2000-12-27

<150> US 09/550,082
<151> 2000-04-14

<160> 14

<170> FastSEQ for Windows Version 4.0

<210> 1
<211> 396
<212> DNA
<213> Mus musculus

<220>
<221> CDS
<222> (1)...(396)

<400> 1

```
atg aag ttg cct gtt agg ctg ttg gtg ctt ctg ttg ttc tgg att cct    48
Met Lys Leu Pro Val Arg Leu Leu Val Leu Leu Leu Phe Trp Ile Pro
 1               5                  10                  15

gtt tcc gga ggt gat gtt gtg gtg act caa act cca ctc tcc ctg cct    96
Val Ser Gly Gly Asp Val Val Val Thr Gln Thr Pro Leu Ser Leu Pro
                20                  25                  30

gtc agc ttt gga gat caa gtt tct atc tct tgc agg tct agt cag agt   144
Val Ser Phe Gly Asp Gln Val Ser Ile Ser Cys Arg Ser Ser Gln Ser
             35                  40                  45

ctt gca aag agt tat ggg aac acc tat ttg tct tgg tac ctg cac aag   192
Leu Ala Lys Ser Tyr Gly Asn Thr Tyr Leu Ser Trp Tyr Leu His Lys
     50                  55                  60

cct ggc cag tct cca cag ctc ctc atc tat ggg att tcc aac aga ttt   240
Pro Gly Gln Ser Pro Gln Leu Leu Ile Tyr Gly Ile Ser Asn Arg Phe
 65                  70                  75                  80

tct ggg gtg cca gac agg ttc agt ggc agt ggt tca ggg aca gat ttc   288
Ser Gly Val Pro Asp Arg Phe Ser Gly Ser Gly Ser Gly Thr Asp Phe
                 85                  90                  95

aca ctc aag atc agc aca ata aag cct gag gac ttg gga atg tat tac   336


Thr Leu Lys Ile Ser Thr Ile Lys Pro Glu Asp Leu Gly Met Tyr Tyr
                100                 105                 110

tgc tta caa ggt aca cat cag ccg tac acg ttc gga ggg ggg acc aag   384
Cys Leu Gln Gly Thr His Gln Pro Tyr Thr Phe Gly Gly Gly Thr Lys
            115                 120                 125

ctg gaa ata aaa                                                    396
Leu Glu Ile Lys
            130
```

<210> 2
<211> 132
<212> PRT
<213> Mus musculus

<220>
<221> SIGNAL
<222> (1)...(20)
<223> signal peptide
<221> SITE
<222> (21)...(43)
<223> framework 1
<221> SITE
<222> (44)...(59)
<223> CDR1
<221> SITE
<222> (60)...(74)
<223> framework 2
<221> SITE
<222> (75)...(81)
<223> CDR2

<221> SITE
<222> (82)...(113)
<223> framework 3
<221> SITE
<222> (114)...(122)
<223> CDR3
<221> SITE
<222> (123)...(132)
<223> framework 4

<400> 2

```
Met Lys Leu Pro Val Arg Leu Leu Val Leu Leu Leu Phe Trp Ile Pro
 1           5                   10                  15
Val Ser Gly Gly Asp Val Val Val Thr Gln Thr Pro Leu Ser Leu Pro
            20                  25                  30
Val Ser Phe Gly Asp Gln Val Ser Ile Ser Cys Arg Ser Ser Gln Ser
            35                  40                  45
Leu Ala Lys Ser Tyr Gly Asn Thr Tyr Leu Ser Trp Tyr Leu His Lys
    50                  55                  60
Pro Gly Gln Ser Pro Gln Leu Leu Ile Tyr Gly Ile Ser Asn Arg Phe
65                  70                  75                  80
Ser Gly Val Pro Asp Arg Phe Ser Gly Ser Gly Ser Gly Thr Asp Phe
                85                  90                  95

Thr Leu Lys Ile Ser Thr Ile Lys Pro Glu Asp Leu Gly Met Tyr Tyr
            100                 105                 110
Cys Leu Gln Gly Thr His Gln Pro Tyr Thr Phe Gly Gly Gly Thr Lys
            115                 120                 125
Leu Glu Ile Lys
            130
```

<210> 3
<211> 420
<212> DNA
<213> Mus musculus

<220>
<221> CDS
<222> (1)...(420)

<400> 3

```
atg gga tgg agc tgt atc atc ctc ttc ttg gta tca aca gct aca agt    48
Met Gly Trp Ser Cys Ile Ile Leu Phe Leu Val Ser Thr Ala Thr Ser
 1               5                  10                  15

gtc cac tcc cag gtc caa ctg cag cag cct ggg gct gag ctt gtg aag    96
Val His Ser Gln Val Gln Leu Gln Gln Pro Gly Ala Glu Leu Val Lys
                20                  25                  30

cct ggg act tca gtg aag ctg tcc tgc aag ggt tat ggc tac acc ttc   144
Pro Gly Thr Ser Val Lys Leu Ser Cys Lys Gly Tyr Gly Tyr Thr Phe
            35                  40                  45

acc agc tac tgg atg cac tgg gtg aag cag agg cct gga caa ggc ctt   192
Thr Ser Tyr Trp Met His Trp Val Lys Gln Arg Pro Gly Gln Gly Leu
        50                  55                  60

gag tgg atc gga gag att gat cct tct gag agt aat act aac tac aat   240
Glu Trp Ile Gly Glu Ile Asp Pro Ser Glu Ser Asn Thr Asn Tyr Asn
 65                  70                  75                  80

caa aaa ttc aag ggc aag gcc aca ttg act gta gac att tcc tcc agc   288
Gln Lys Phe Lys Gly Lys Ala Thr Leu Thr Val Asp Ile Ser Ser Ser
                85                  90                  95

aca gcc tac atg cag ctc agc agc ctg aca tct gag gac tct gcg gtc   336
Thr Ala Tyr Met Gln Leu Ser Ser Leu Thr Ser Glu Asp Ser Ala Val
                100                 105                 110

tac tat tgt gca aga ggg ggt tac gac gga tgg gac tat gct att gac   384
Tyr Tyr Cys Ala Arg Gly Gly Tyr Asp Gly Trp Asp Tyr Ala Ile Asp
            115                 120                 125

tac tgg ggt caa ggc acc tca gtc acc gtc tcc tca                   420
Tyr Trp Gly Gln Gly Thr Ser Val Thr Val Ser Ser
        130                 135                 140
```

<210> 4
<211> 140
<212> PRT
<213> Mus musculus

<220>
<221> SIGNAL
<222> (1)...(19)
<223> signal peptide
<221> SITE
<222> (20)...(49)
<223> framework 1
<221> SITE
<222> (50)...(54)
<223> CDR1
<221> SITE
<222> (55)...(68)
<223> framework 2
<221> SITE
<222> (69)...(85)
<223> CDR2
<221> SITE
<222> (86)...(117)
<223> framework 3

<221> SITE
<222> (118)...(129)
<223> CDR3
<221> SITE
<222> (130)...(140)
<223> framework 4

<400> 4

```
      Met Gly Trp Ser Cys Ile Ile Leu Phe Leu Val Ser Thr Ala Thr Ser
       1               5                   10                  15
      Val His Ser Gln Val Gln Leu Gln Gln Pro Gly Ala Glu Leu Val Lys
                  20                  25                  30
      Pro Gly Thr Ser Val Lys Leu Ser Cys Lys Gly Tyr Gly Tyr Thr Phe
                  35                  40                  45
      Thr Ser Tyr Trp Met His Trp Val Lys Gln Arg Pro Gly Gln Gly Leu
              50                  55                  60
      Glu Trp Ile Gly Glu Ile Asp Pro Ser Glu Ser Asn Thr Asn Tyr Asn
      65                  70                  75                  80
      Gln Lys Phe Lys Gly Lys Ala Thr Leu Thr Val Asp Ile Ser Ser Ser
                          85                  90                  95
      Thr Ala Tyr Met Gln Leu Ser Ser Leu Thr Ser Glu Asp Ser Ala Val
                  100                 105                 110
      Tyr Tyr Cys Ala Arg Gly Gly Tyr Asp Gly Trp Asp Tyr Ala Ile Asp
                  115                 120                 125
      Tyr Trp Gly Gln Gly Thr Ser Val Thr Val Ser Ser
                  130                 135                 140
```

<210> 5
<211> 540
<212> DNA
<213> Artificial Sequence

<220>
<223> DNA encoding portion of the heavy chain of LDP-02 with a heavy chain signal peptide
<221> CDS
<222> (1)...(540)

<400> 5

EP 3 167 902 B1

```
atg aaa tgc acc tgg gtc att ctc ttc ttg gta tca aca gct aca agt   48
Met Lys Cys Thr Trp Val Ile Leu Phe Leu Val Ser Thr Ala Thr Ser
1               5                   10                  15

gtc cac tcc cag gtc caa cta gtg cag tct ggg gct gag gtt aag aag   96
Val His Ser Gln Val Gln Leu Val Gln Ser Gly Ala Glu Val Lys Lys
            20                  25                  30

cct ggg gct tca gtg aag gtg tcc tgc aag ggt tct ggc tac acc ttc   144
Pro Gly Ala Ser Val Lys Val Ser Cys Lys Gly Ser Gly Tyr Thr Phe
            35                  40                  45

acc agc tac tgg atg cat tgg gtg agg cag gcg cct ggc caa cgt cta   192
Thr Ser Tyr Trp Met His Trp Val Arg Gln Ala Pro Gly Gln Arg Leu
        50                  55                  60

gag tgg atc gga gag att gat cct tct gag agt aat act aac tac aat   240
Glu Trp Ile Gly Glu Ile Asp Pro Ser Glu Ser Asn Thr Asn Tyr Asn
65                  70                  75                  80

caa aaa ttc aag gga cgc gtc aca ttg act gta gac att tcc gct agc   288
Gln Lys Phe Lys Gly Arg Val Thr Leu Thr Val Asp Ile Ser Ala Ser
                85                  90                  95

aca gcc tac atg gag ctc agc agc ctg aga tct gag gac act gcg gtc   336
Thr Ala Tyr Met Glu Leu Ser Ser Leu Arg Ser Glu Asp Thr Ala Val
            100                 105                 110

tac tat tgt gca aga ggg ggt tac gac gga tgg gac tat gct att gac   384
Tyr Tyr Cys Ala Arg Gly Gly Tyr Asp Gly Trp Asp Tyr Ala Ile Asp
            115                 120                 125

tac tgg ggt caa ggc acc ctg gtc acc gtc tcc tca gcc tcc acc aag   432
Tyr Trp Gly Gln Gly Thr Leu Val Thr Val Ser Ser Ala Ser Thr Lys
        130                 135                 140

ggc cca tcg gtc ttc ccc ctg gca ccc tcc tcc aag agc acc tct ggg   480
Gly Pro Ser Val Phe Pro Leu Ala Pro Ser Ser Lys Ser Thr Ser Gly
145                 150                 155                 160

ggc aca gcg gcc ctg ggc tgc ctg gtc aag gac tac ttc ccc gaa ccg   528
Gly Thr Ala Ala Leu Gly Cys Leu Val Lys Asp Tyr Phe Pro Glu Pro
            165                 170                 175

gtg acg gtg tcg                                                    540
Val Thr Val Ser
            180
```

<210> 6
<211> 180
<212> PRT
<213> Artificial Sequence

<220>
<223> Portion of the heavy chain of LDP-02 with a heavy chain signal peptide
<221> SITE
<222> (19)...(20)
<223> signal peptide cleavage site

<400> 6

43

```
Met Lys Cys Thr Trp Val Ile Leu Phe Leu Val Ser Thr Ala Thr Ser
1               5                   10                  15
Val His Ser Gln Val Gln Leu Val Gln Ser Gly Ala Glu Val Lys Lys
            20                  25                  30
Pro Gly Ala Ser Val Lys Val Ser Cys Lys Gly Ser Gly Tyr Thr Phe
        35                  40                  45
Thr Ser Tyr Trp Met His Trp Val Arg Gln Ala Pro Gly Gln Arg Leu
    50                  55                  60
Glu Trp Ile Gly Glu Ile Asp Pro Ser Glu Ser Asn Thr Asn Tyr Asn
65                  70                  75                  80
Gln Lys Phe Lys Gly Arg Val Thr Leu Thr Val Asp Ile Ser Ala Ser
            85                  90                  95
Thr Ala Tyr Met Glu Leu Ser Ser Leu Arg Ser Glu Asp Thr Ala Val
            100                 105                 110
Tyr Tyr Cys Ala Arg Gly Gly Tyr Asp Gly Trp Asp Tyr Ala Ile Asp
            115                 120                 125
Tyr Trp Gly Gln Gly Thr Leu Val Thr Val Ser Ser Ala Ser Thr Lys
    130                 135                 140
Gly Pro Ser Val Phe Pro Leu Ala Pro Ser Ser Lys Ser Thr Ser Gly
145                 150                 155                 160
Gly Thr Ala Ala Leu Gly Cys Leu Val Lys Asp Tyr Phe Pro Glu Pro
            165                 170                 175
Val Thr Val Ser
            180
```

<210> 7
<211> 413
<212> DNA
<213> Artificial Sequence

<220>
<223> DNA encoding portion of the light chain of LDP-02 with a light chain signal peptide
<221> CDS
<222> (1)...(413)

<400> 7

```
atg aag ttg cct gtt agg ctg ttg gtg ctt ctg ttg ttc tgg att cct      48
Met Lys Leu Pro Val Arg Leu Leu Val Leu Leu Leu Phe Trp Ile Pro
1               5                   10                  15

gtt tcc gga ggt gat gtt gtg atg act caa agt cca ctc tcc ctg cct      96
Val Ser Gly Gly Asp Val Val Met Thr Gln Ser Pro Leu Ser Leu Pro
            20                  25                  30

gtc acc cct gga gaa cca gct tct atc tct tgc agg tct agt cag agt     144
Val Thr Pro Gly Glu Pro Ala Ser Ile Ser Cys Arg Ser Ser Gln Ser
            35                  40                  45

ctt gca aag agt tat ggg aac acc tat ttg tct tgg tac ctg cag aag     192
Leu Ala Lys Ser Tyr Gly Asn Thr Tyr Leu Ser Trp Tyr Leu Gln Lys
            50                  55                  60

cct ggc cag tct cca cag ctc ctc atc tat ggg att tcc aac aga ttt     240
Pro Gly Gln Ser Pro Gln Leu Leu Ile Tyr Gly Ile Ser Asn Arg Phe
65                  70                  75                  80

tct ggg gtg cca gac agg ttc agt ggc agt ggt tca ggg aca gat ttc     288
```

44

```
        Ser Gly Val Pro Asp Arg Phe Ser Gly Ser Gly Ser Gly Thr Asp Phe
                         85                  90                  95

        aca ctc aag atc tcg cga gta gag gct gag gac gtg gga gtg tat tac    336
        Thr Leu Lys Ile Ser Arg Val Glu Ala Glu Asp Val Gly Val Tyr Tyr
                    100                 105                 110

        tgc tta caa ggt aca cat cag ccg tac acg ttc gga cag ggg acc aag    384
        Cys Leu Gln Gly Thr His Gln Pro Tyr Thr Phe Gly Gln Gly Thr Lys
                    115                 120                 125

        gtg gaa ata aaa cgg gct gat gcg gcg cc                             413
        Val Glu Ile Lys Arg Ala Asp Ala Ala
                    130                 135
```

<210> 8
<211> 138
<212> PRT
<213> Artificial Sequence

<220>
<223> Portion of the light chain of LDP-02 with a light chain signal peptide
<221> SITE
<222> (20)...(21)
<223> signal peptide cleavage site

<400> 8

```
        Met Lys Leu Pro Val Arg Leu Leu Val Leu Leu Leu Phe Trp Ile Pro
        1               5                   10                  15
        Val Ser Gly Gly Asp Val Val Met Thr Gln Ser Pro Leu Ser Leu Pro
                    20                  25                  30
        Val Thr Pro Gly Glu Pro Ala Ser Ile Ser Cys Arg Ser Ser Gln Ser
                    35                  40                  45
        Leu Ala Lys Ser Tyr Gly Asn Thr Tyr Leu Ser Trp Tyr Leu Gln Lys
                50                  55                  60
        Pro Gly Gln Ser Pro Gln Leu Leu Ile Tyr Gly Ile Ser Asn Arg Phe
        65                  70                  75                  80
        Ser Gly Val Pro Asp Arg Phe Ser Gly Ser Gly Ser Gly Thr Asp Phe
                         85                  90                  95
        Thr Leu Lys Ile Ser Arg Val Glu Ala Glu Asp Val Gly Val Tyr Tyr
                    100                 105                 110
        Cys Leu Gln Gly Thr His Gln Pro Tyr Thr Phe Gly Gln Gly Thr Lys
                    115                 120                 125
        Val Glu Ile Lys Arg Ala Asp Ala Ala Pro
                130                 135
```

<210> 9
<211> 16
<212> PRT
<213> Mus musculus

<220>
<221> SITE
<222> (1)...(16)
<223> CDR1 of the light chain of antibodies Act-1 and LDP-02

<400> 9

```
Arg Ser Ser Gln Ser Leu Ala Lys Ser Tyr Gly Asn Thr Tyr Leu Ser

       1              5              10              15
```

<210> 10
<211> 7
<212> PRT
<213> Mus musculus

<220>
<221> SITE
<222> (1)...(7)
<223> CDR2 of the light chain of antibodies Act-1 and LDP-02

<400> 10

```
                        Gly Ile Ser Asn Arg Phe Ser
                         1              5
```

<210> 11
<211> 9
<212> PRT
<213> Mus musculus

<220>
<221> SITE
<222> (1)...(9)
<223> CDR3 of the light chain of antibodies Act-1 and LDP-02

<400> 11

```
                        Leu Gln Gly Thr His Gln Pro Tyr Thr
                         1              5
```

<210> 12
<211> 5
<212> PRT
<213> Mus musculus

<220>
<221> SITE
<222> (1)...(5)
<223> CDR1 of the heavy chain of antibodies Act-1 and LDP-02

<400> 12

```
                        Ser Tyr Trp Met His
                         1              5
```

<210> 13
<211> 17
<212> PRT
<213> Mus musculus

<220>
<221> SITE

<222> (1)...(17)
<223> CDR2 of the heavy chain of antibodies Act-1 and LDP-02

<400> 13

```
        Glu Ile Asp Pro Ser Glu Ser Asn Thr Asn Tyr Asn Gln Lys Phe Lys
         1                   5                   10                  15
        Gly
```

<210> 14
<211> 12
<212> PRT
<213> Mus musculus

<220>
<221> SITE
<222> (1)...(12)
<223> CDR3 of the heavy chain of antibodies Act-1 and LDP-02

<400> 14

```
        Gly Gly Tyr Asp Gly Trp Asp Tyr Ala Ile Asp Tyr
         1                   5                   10
```

**Description**

BACKGROUND OF THE INVENTION

Integrin receptors are important for regulating both lymphocyte recirculation and recruitment to sites of inflammation (Carlos, T.M. and Harlan, J.M., Blood, 84:2068-2101 (1994)). The human $\alpha4\beta7$ integrin has several ligands, one of which is the mucosal vascular addressin MAdCAM-1 (Berlin, C., et al., Cell 74:185-195 (1993); Erle, D.J., et al., J. Immunol. 153:517-528 (1994)) expressed on high endothelial venules in mesenteric lymph nodes and Peyer's patches (Streeter, P.R., et al., Nature 331:41-46 (1988)). As such, the $\alpha4\beta7$ integrin acts as a homing receptor that mediates lymphocyte migration to intestinal mucosal lymphoid tissue (Schweighoffer, T., et al., J. Immunol. 151:717-729 (1993)). In addition, the $\alpha4\beta7$ integrin interacts with fibronectin and vascular cell adhesion molecule-1 (VCAM-1).

Inflammatory bowel disease (IBD), such as ulcerative colitis and Crohn's disease, for example, can be a debilitating and progressive disease involving inflammation of the gastrointestinal tract. Affecting an estimated two million people in the United States alone, symptoms include abdominal pain, cramping, diarrhea and rectal bleeding. IBD treatments have included anti-inflammatory drugs (such as, corticosteroids and sulfasalazine), immunosuppressive drugs (such as, 6-mercaptopurine, cyclosporine and azathioprine) and surgery (such as, colectomy). Podolsky, New Engl. J. Med., 325:928-937 (1991) and Podolsky, New Engl. J. Med., 325:1008-1016 (1991) GORDON F. H. ET AL., Gastroenterology, vol. 116, no. 4, April 1999, page A726 (abstract G3151) discloses the treatment of active ulcerative colitis with the recombinant humanised antibody to alpha4 integrin Antegren ™ used at 3mg/ kg body weight. GORDON F. H. ET AL., Gastroenterology, vol. 116, no. 4, April 1999, page A726 (abstract G3152) discloses the treatment of active Crohn's disease with the recombinant humanised antibody to alpha4 integrin Antegren ™ at 3mg/kg body weight. Antegren ™ is a recombinant humanized antibody to alpha4 integrin with activity against $\alpha4\beta1$ and $\alpha4\beta7$ integrins. However, such therapeutic agents have not been effective in maintaining remission of EBD.

Antibodies against human $\alpha4\beta7$ integrin, such as murine monoclonal antibody Act-1 (mAb Act-1), interfere with $\alpha4\beta7$ integrin binding to mucosal addressin cell adhesion molecule-1 (MAdCAM-1) present on high endothelial venules in mucosal lymph nodes. Act-1 was originally isolated by Lazarovits, A.I., et al., J. Immunol. 133:1857-1862 (1984), from mice immunized with human tetanus toxoid-specific T lymphocytes and was reported to be a mouse IgG1/$\kappa$ antibody. More recent analysis of the antibody by Schweighoffer, T., et al., J. Immunol. 151:717-729 (1993) demonstrated that it can bind to a subset of human memory CD4+ T lymphocytes which selectively express the $\alpha4\beta7$ integrin. However, a serious problem with using murine antibodies for therapeutic applications in humans is that they are highly immunogenic in humans and quickly induce a human anti-murine antibody response (HAMA), which reduces the efficacy of the mouse antibody in patients and can prevent continued administration. The HAMA response results in rapid clearance of the mouse antibody, severely limiting any therapeutic benefit.

Thus, a need exists for improved therapeutic approaches to inflammatory bowel diseases and other inflammatory disorders of mucosal tissues.

SUMMARY OF THE INVENTION

The invention relates to use of an antibody, namely a humanized antibody. In one aspect the invention is a use in treating a human having a disease associated with leukocyte infiltration of mucosal tissues comprising administering to the human an effective amount of an immunoglobulin having binding specificity for $\alpha4\beta7$ integrin, as further defined in the claims. In preferred embodiments no more than about 8 mg immunoglobulin per kg body weight is administered in a period of about one month. In particular embodiments, the immunoglobulin includes the complementarity determining regions (CDRs) having the amino acid sequence of a CDR of murine Act-1 mAb. LDP-02 is the prefered antibody for administration. The immunoglobulin is administered in multiple doses and the interval between doses is at least 1 day or longer as defined in the claims. In particular embodiments, the interval between doses can be at least about 7, 14 or 21 days or about one month. In one embodiment, the amount of immunoglobulin administered per dose can be an amount which is sufficient to achieve about 50% or greater saturation of $\alpha4\beta7$ binding sites on circulating lymphocytes and/or about 50% or greater inhibition of $\alpha4\beta7$ integrin expression on the surface of circulating lymphocytes for a period of at least about 10 days following administration of the dose. In another embodiment, the amount of immunoglobulin administered per dose can be an amount which is sufficient to achieve and maintain a serum concentration of said immunoglobulin of at least about 1 $\mu$g/mL for a period of about 10 days following administration of the dose.

The immunoglobulin can be administered alone or together with one or more other agents to treat a disease associated with leukocyte infiltration of mucosal tissues. For example, the immunoglobulin can be administered with steroids, immunosuppressive agents, non-steroidal anti-inflammatory agents or immunomodulators. The immunoglobulin is administered to treat a human having an inflammatory bowel disease, such as Crohn's disease or ulcerative colitis.

BRIEF DESCRIPTION OF THE DRAWINGS

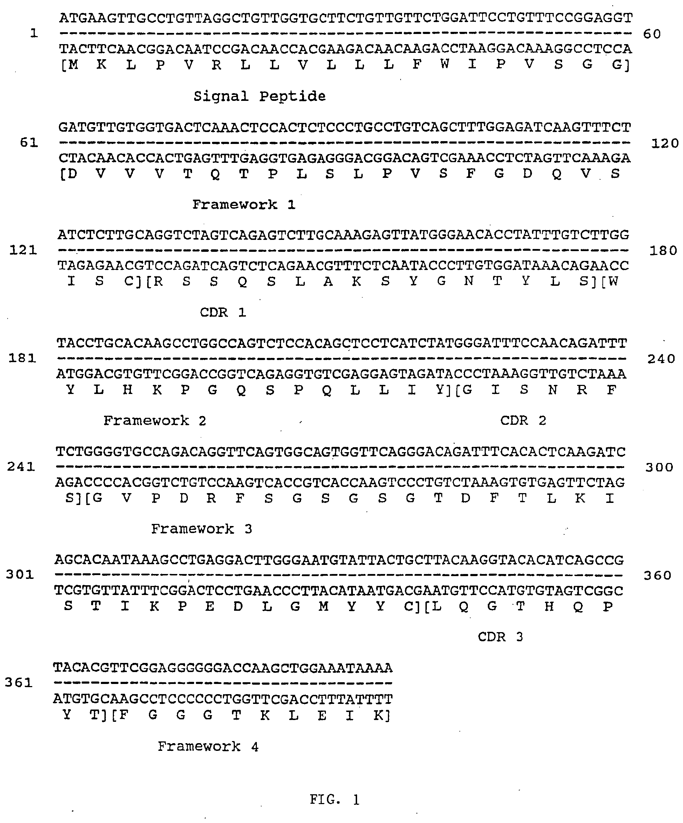

FIG. 1 is an illustration of the nucleotide sequence (SEQ ID NO:1) and deduced amino acid sequence (SEQ ID NO:2) of the mouse (*Mus musculus*) Act-1 light chain variable region joined to the mouse Act-1 light chain signal peptide sequence.

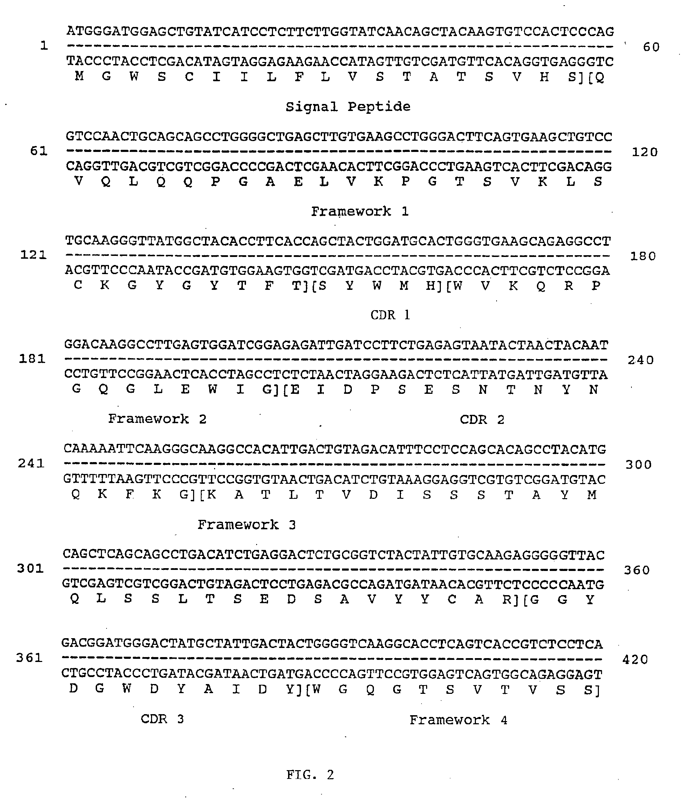

FIG. 2 is an illustration of the nucleotide sequence (SEQ ID NO:3) and amino acid sequence (SEQ ID NO:4) of the mouse Act-1 antibody heavy chain variable region. The nucleotide sequence of the variable region is joined to a nucleotide sequence which encodes a deduced mouse Act-1 heavy chain signal peptide sequence, to yield a composite sequence. (The identity of the primer which amplified the heavy chain region was deduced from the degenerate sequence, and an amino acid sequence for the signal peptide was derived from the primer, downstream sequence and sequences of other signal peptides. The signal peptide shown may not be identical to that of the Act-1 hybridoma.)

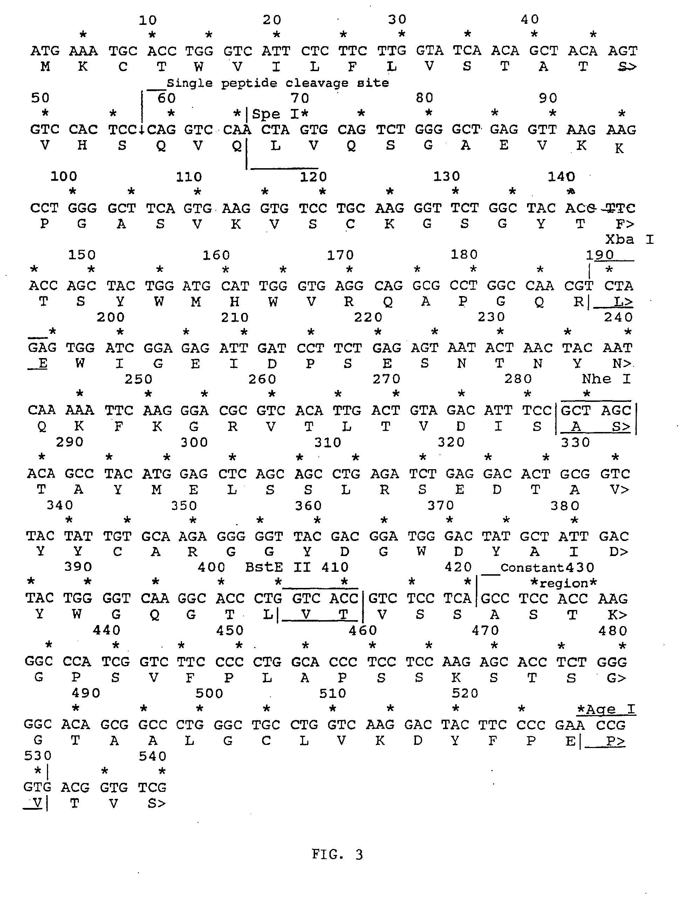

FIG. 3 is an illustration of the nucleotide sequence (SEQ ID NO:5) and amino acid sequence (SEQ ID NO:6) of a portion of the heavy chain of a humanized Act-1 antibody (LDP-02) with a heavy chain signal peptide.

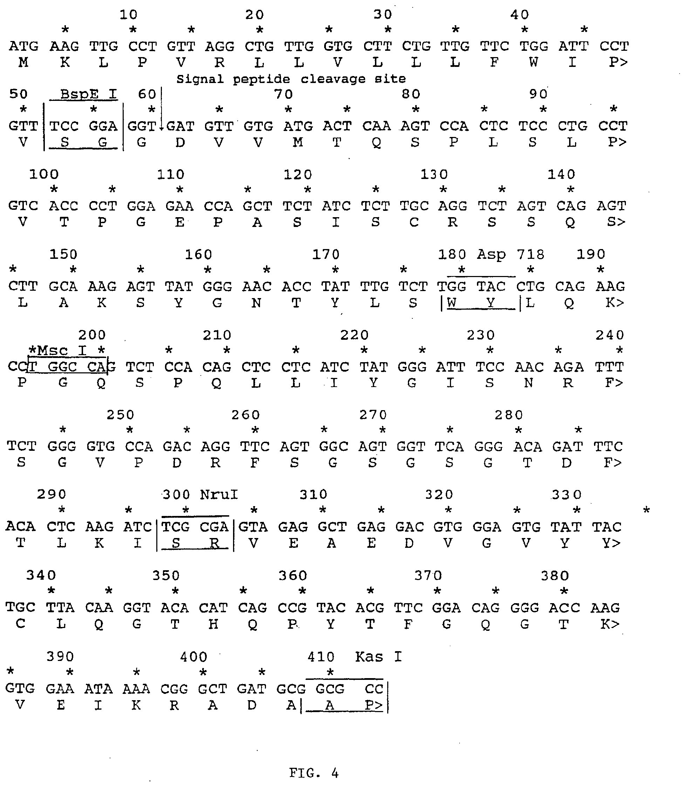

FIG. 4 is an illustration of the nucleotide sequence (SEQ ID NO:7) and amino acid sequence (SEQ ID NO:8) of a portion of the light chain of a humanized Act-1 antibody (LDP-02) with a light chain signal peptide.

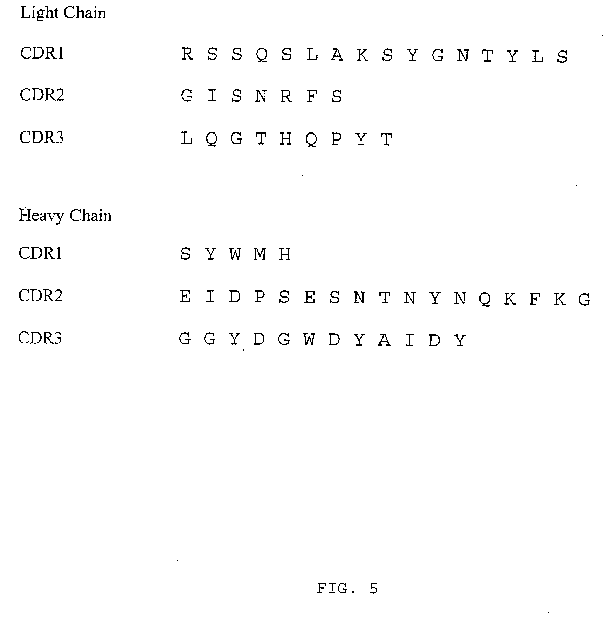

FIG. 5 is an illustration of the amino acid sequence of the light chain complementarity determining regions (CDR1, SEQ ID NO: 9; CDR2, SEQ ID NO:10; CDR3, SEQ ID NO:11) and heavy chain complementarity determining regions (CDR1, SEQ ID NO: 12; CDR2, SEQ ID NO:13; CDR3, SEQ ID NO:14) of murine antibody Act-1 and LDP-02.

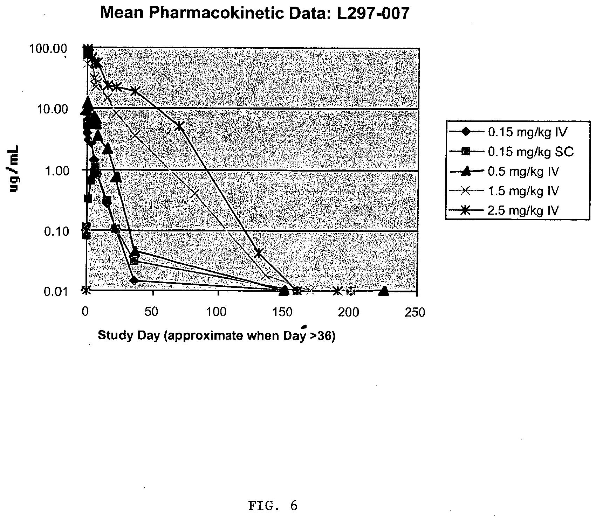

FIG. 6 is a graph showing mean serum LDP-02 levels ($\mu$g/mL) in healthy men over time following a single administration of LDP-02. Mean serum LDP-02 levels became negligible by day 36 following administration of 0.15 mg/kg by intravenous (IV)(-♦-) or subcutaneous (SC)(-■-) injection and following administration of 0.5 mg/kg by intravenous injection (-▲-). However serum LDP-02 was still measurable beyond day 36 following administration of 1.5 mg/kg (-x-) or 2.5 mg/kg (- *-) by intravenous injection.

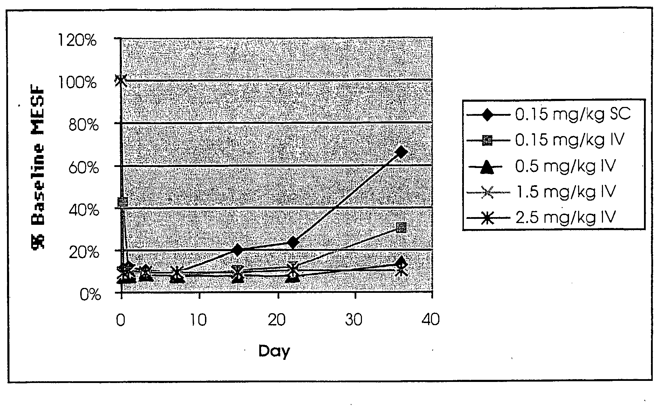

FIG. 7 is a graph showing persistent loss of $\alpha4\beta7$ signal (detected with Act-1 mAb) following administration of LDP-02. About 90% of $\alpha4\beta7$ signal was rapidly lost (MESF $\approx$ 10%) after administration of LDP-02 and persisted following administration of all LDP-02 doses. Between about day 7 and day 22, $\alpha4\beta7$ signal started to return to baseline for the 0.15 mg/kg IV dose group (-■-) and for the 0.15 mg/kg SC dose group (-♦-). Between day 22 and day 36, $\alpha4\beta7$ signal started to return to baseline for the 0.5 mg/kg IV (-▲-) dose group. At the higher doses of LDP-02 studied (1.5 mg/kg (-x-) and 2.5 mg/kg (- *-)), loss of $\alpha4\beta7$ signal persisted for longer than 36 days following single IV doses. For the 2.5 mg/kg dose group (-*-), loss of $\alpha4\beta7$ signal persisted up to about Day 70 (data provided herein in Appendix to Study L297-007). MESF: mean equivalent soluble fluorescence.

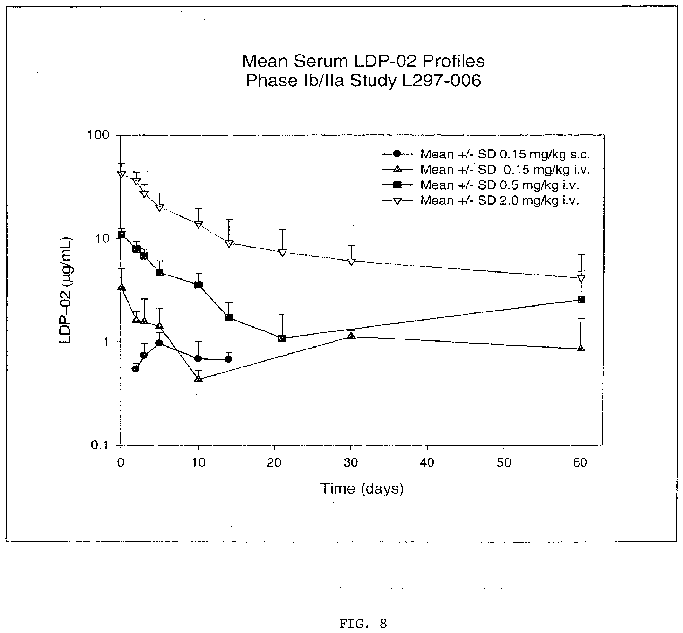

FIG. 8 is a graph showing mean serum LDP-02 levels ($\mu$g/mL) in patients with ulcerative colitis over time following a single administration of LDP-02. Mean serum LDP-02 levels rose rapidly following administration of LDP-02. The concentration of serum LDP-02 fell to below 1.0 $\mu$g/mL in patients administered LDP-02 at 0.15 mg/kg by intravenous (-▲-) or subcutaneous (-●-) injection by 10 days following dosing. However, serum LDP-02 concentrations remained above 1.0 $\mu$g/mL for about 20 days following administration of 0.5 mg/kg by intravenous injection (-■-). The serum concentration of LDP-02 remained above 1 $\mu$g/mL for about 60 days following administration of 2.0 mg/kg by intravenous injection (-▼-).

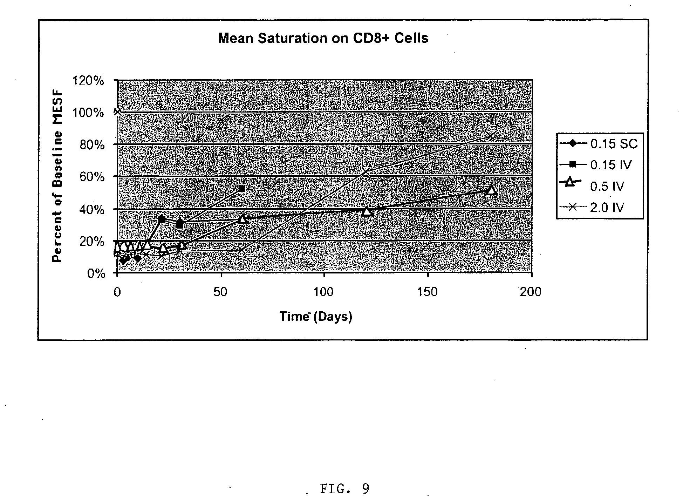

FIG. 9 is a graph showing persistent loss of $\alpha4\beta7$ signal (detected with Act-1 mAb) following administration of LDP-02. About 90% of $\alpha4\beta7$ signal was rapidly lost (MESF $\approx$ 10%) after administration of LDP-02 and the duration of signal loss was dependent upon dose. Starting at about Day 10, $\alpha4\beta7$ signal started to return to baseline for the group administered 0.15 mg/kg of LDP-02 by IV (-■-) or SC (-♦-) injection. However, $\alpha4\beta7$ signal started to return to baseline between day 30 and day 60 for the group administered 0.5 mg/kg (-Δ-) intravenously, and after day 60 for the group administered 2.0 mg/kg (-x-) intravenously (data provided herein in Appendix to Study L297-006). MESF: mean equivalent soluble fluorescence.

DETAILED DESCRIPTION OF THE INVENTION

The present invention relates to a method of administering an antibody (immunoglobulin) to a subject. In one aspect, the antibody to be administered is a human or humanized antibody having binding specificity for $\alpha4\beta7$ integrin (e.g., mammalian $\alpha4\beta7$ (e.g., human (*Homo sapiens*) $\alpha4\beta7$). Preferably, the human or humanized immunoglobulins can bind $\alpha4\beta7$ integrin with an affinity of at least about $10^7M^{-1}$, preferably at least about $10^8M^{-1}$, and more preferably at least about $10^9M^{-1}$. In one embodiment, the humanized immunoglobulin includes an antigen binding region of nonhuman origin which binds $\alpha4\beta7$ integrin and a constant region derived from a human constant region. In another embodiment, the humanized immunoglobulin which binds $\alpha4\beta7$ integrin comprises a complementarity determining region of nonhuman origin and a variable framework region of human origin, and if desired, a constant region of human origin. For example, the humanized immunoglobulin can comprise a heavy chain and a light chain, wherein the light chain comprises a complementarity determining region derived from an antibody of nonhuman origin which binds $\alpha4\beta7$ integrin and a framework region derived from a light chain of human origin, and the heavy chain comprises a complementarity determining region derived from an antibody of nonhuman origin which binds $\alpha4\beta7$ integrin and a framework region derived from a heavy chain of human origin.

Naturally occurring immunoglobulins have a common core structure in which two identical light chains (about 24 kD) and two identical heavy chains (about 55 or 70 kD) form a tetramer. The amino-terminal portion of each chain is known as the variable (V) region and can be distinguished from the more conserved constant (C) regions of the remainder of each chain. Within the variable region of the light chain is a C-terminal portion known as the J region. Within the variable region of the heavy chain, there is a D region in addition to the J region. Most of the amino acid sequence variation in immunoglobulins is confined to three separate locations in the V regions known as hypervariable regions or complementarity determining regions (CDRs) which are directly involved in antigen binding. Proceeding from the amino-terminus, these regions are designated CDR1, CDR2 and CDR3, respectively. The CDRs are held in place by more conserved framework regions (FRs). Proceeding from the amino-terminus, these regions are designated FR1, FR2, FR3, and FR4, respectively. The locations of CDR and FR regions and a numbering system have been defined by Kabat *et al.* (Kabat, E.A. et al., Sequences of Proteins of Immunological Interest, Fifth Edition, U.S. Department of Health and Human Services, U.S. Government Printing Office (1991)).

Human immunoglobulins can be divided into classes and subclasses, depending on the isotype of the heavy chain. The classes include IgG, IgM, IgA, IgD and IgE, in which the heavy chains are of the gamma ($\gamma$), mu ($\mu$), alpha ($\alpha$), delta ($\delta$) or epsilon ($\varepsilon$) type, respectively. Subclasses include IgG1, IgG2, IgG3, IgG4, IgA1 and IgA2, in which the heavy chains are of the $\gamma1$, $\gamma2$, $\gamma3$, $\gamma4$, $\alpha1$ and $\alpha2$ type, respectively. Human immunoglobulin molecules of a selected class or subclass may contain either a kappa ($\kappa$) or lambda ($\lambda$) light chain. See e.g., Cellular and Molecular Immunology, Wonsiewicz, M.J., Ed., Chapter 45, pp. 41-50, W. B. Saunders Co, Philadelphia, PA (1991); Nisonoff, A., Introduction to Molecular Immunology, 2nd Ed., Chapter 4, pp. 45-65, Sinauer Associates, Inc., Sunderland, MA (1984).

The term "immunoglobulin" as used herein includes whole antibodies and biologically functional fragments thereof. Such biologically functional fragments retain at least one antigen binding function of a corresponding full-length antibody (e.g., specificity for $\alpha4\beta7$ of Act-1 antibody), and preferably, retain the ability to inhibit the interaction of $\alpha4\beta7$ with one or more of its ligands (e.g., MAdCAM-1, fibronectin). In a particularly preferred embodiment, biologically functional fragments can inhibit binding of $\alpha4\beta7$ to the mucosal addressin (MAdCAM-1). Examples of biologically functional antibody fragments which can be administered as described herein include fragments capable of binding to an $\alpha4\beta7$ integrin, such as single chain antibodies, Fv, Fab, Fab' and F(ab')$_2$ fragments. Such fragments can be produced by enzymatic cleavage or by recombinant techniques. For example, papain or pepsin cleavage can generate Fab or F(ab')$_2$ fragments, respectively. Other proteases with the requisite substrate specificity can also be used to generate Fab, F(ab')$_2$ or other antigen-binding fragments. Antibodies can also be produced in a variety of truncated forms using antibody genes in which one or more stop codons have been introduced upstream of the natural stop site. For example, a chimeric gene encoding a F(ab')$_2$ heavy chain portion can be designed to include DNA sequences encoding the CH$_1$ domain and hinge region of the heavy chain.

The term "humanized immunoglobulin" as used herein refers to an immunoglobulin (antibody) comprising portions of immunoglobulins of different origin, wherein at least one portion is of human origin. For example, the humanized antibody can comprise portions derived from an immunoglobulin of nonhuman origin with the requisite specificity, such as a mouse, and from immunoglobulin sequences of human origin (e.g., chimeric immunoglobulin), joined together chemically by conventional techniques (e.g., synthetic) or prepared as a contiguous polypeptide using recombinant DNA technology (e.g., DNA encoding the protein portions of the chimeric antibody can be expressed to produce a contiguous polypeptide chain). Another example of a humanized immunoglobulin is an immunoglobulin containing one or more immunoglobulin chains comprising a CDR derived from an antibody of nonhuman origin and a framework region derived from a light and/or heavy chain of human origin (e.g., CDR-grafted antibodies with or without framework changes). Chimeric or CDR-grafted single chain antibodies are also encompassed by the term humanized immunoglobulin. See, e.g., Cabilly et al., U.S. Patent No. 4,816,567; Cabilly *et al.,* European Patent No. 0,125,023 B1; Boss et al., U.S. Patent No. 4,816,397; Boss *et al.,* European Patent No. 0,120,694 B1; Neuberger, M.S. et al., WO 86/01533; Neuberger, M.S. *et al.,* European Patent No. 0,194,276 B1; Winter, U.S. Patent No. 5,225,539; Winter, European Patent No. 0,239,400 B1; Queen *et al.*, European Patent No. 0 451 216 B1; Padlan, E.A. *et al.,* European Patent Application No. 0,519,596 A1. See also, Ladner et al., U.S. Patent No. 4,946,778; Huston, U.S. Patent No. 5,476,786; and Bird, R.E. et al., Science, 242: 423-426 (1988)), regarding single chain antibodies. In particular embodiments, the humanized immunoglobulin can include an immunoglobulin chain (e.g., heavy chain) having a variable region of non-human origin (e.g., murine origin) and at least a portion of a human constant region (e.g, C$\gamma$1), and an immunoglobulin chain (e.g., light chain) where at least one CDR is of non-human origin (e.g., murine origin) and the framework regions (FR1, FR2, FR3, FR4) and, optionally, the constant region (e.g., C$\kappa$, C$\lambda$) are of human origin.

The antigen binding region of the humanized immunoglobulin (the nonhuman portion) can be derived from an immunoglobulin of nonhuman origin (referred to as a donor immunoglobulin) having binding specificity for α4β7 integrin. For example, a suitable antigen binding region can be derived from the murine Act-1 monoclonal antibody (Lazarovits, A.I. et al., J. Immunol., 133(4): 1857-1862 (1984)). Other sources include α4β7 integrin-specific antibodies obtained from nonhuman sources, such as rodent (e.g., mouse, rat), rabbit, pig, goat or non-human primate (e.g., monkey). Other polyclonal or monoclonal antibodies, such as antibodies which bind to the same or similar epitope as the Act-1 antibody, or LDP-02, can be made (e.g., Kohler et al., Nature, 256:495-497 (1975); Harlow et al., 1988, Antibodies: A Laboratory Manual, (Cold Spring Harbor, NY); and Current Protocols in Molecular Biology, Vol. 2 (Supplement 27, Summer '94), Ausubel et al., Eds. (John Wiley & Sons: New York, NY), Chapter 11 (1991)).

For example, antibodies can be raised against an appropriate immunogen in a suitable mammal (e.g., a mouse, rat, rabbit, sheep). Preparation of immunizing antigen, and polyclonal and monoclonal antibody production can be performed using any suitable technique. A variety of methods have been described (see e.g., Kohler et al., Nature, 256: 495-497 (1975) and Eur. J. Immunol. 6: 511-519 (1976); Milstein et al., Nature 266: 550-552 (1977); Koprowski et al., U.S. Patent No. 4,172,124; Harlow, E. and D. Lane, 1988, Antibodies: A Laboratory Manual, (Cold Spring Harbor Laboratory: Cold Spring Harbor, NY); Current Protocols In Molecular Biology, Vol. 2 (Supplement 27, Summer '94), Ausubel, F.M. et al., Eds., (John Wiley & Sons: New York, NY), Chapter 11, (1991)). For example, suitable immunizing agents include cells bearing α4β7, membrane fractions containing α4β7, immunogenic fragments of suitable immunogens include α4β7, a β7 peptide conjugated to a suitable carrier and the like. Antibody-producing cells (e.g., a lymphocyte) can be isolated from, for example, the lymph nodes or spleen of an immunized animal. The cells can then be fused to a suitable immortalized cell (e.g., a myeloma cell line (e.g., SP2/0, P3x63Ag8.653)), thereby forming a hybridoma. Fused cells can be isolated employing selective culturing techniques. Cells which produce antibodies with the desired specificity can be selected using a suitable assay (e.g., ELISA). Other suitable methods of producing or isolating antibodies (human antibodies, non-human antibodies) of the requisite specificity can be used, including, for example, methods which select recombinant antibody from a library (e.g., a phage display library). Transgenic animals capable of producing a repertoire of human antibodies (e.g., XenoMouse™ (Abgenix, Fremont, CA) can be produced using suitable methods (see e.g., WO 98/24893 (Abgenix), published June 11, 1998; Kucherlapate, R. and Jakobovits, A., U.S. Patent No. 5,939,598; Jakobovits et al., Proc. Natl. Acad. Sci. USA, 90: 2551-2555 (1993); Jakobovits et al., Nature, 362: 255-258 (1993)). Additional methods for production of transgenic animals capable of producing a repertoire of human antibodies have been described (e.g., Lonberg et al., U.S. Patent No. 5,545,806; Surani et al., U.S. Patent No. 5,545,807; Lonberg et al., WO97/13852).

The antigen binding region of the humanized immunoglobulin comprises CDRs of nonhuman origin. All six CDRs of the light and heavy chains are substantially the same as the CDRs of the corresponding donor chains. The one or more CDRs of nonhuman origin have the amino acid sequences of the CDRs of murine Act-1 Ab (SEQ ID NOS: 9-14).

The portion of the humanized immunoglobulin or immunoglobulin chain which is of human origin (the human portion) can be derived from any suitable human immunoglobulin or immunoglobulin chain. For example, a human constant region or portion thereof, if present, can be derived from the κ or λ light chains, and/or the γ (e.g., γ1, γ2, γ3, γ4), μ, α (e.g., α1, α2), δ or ε heavy chains of human antibodies, including allelic variants. A particular constant region (e.g., IgG1), variant or portions thereof can be selected in order to tailor effector function. For example, a mutated constant region (variant) can be incorporated into a fusion protein to minimize binding to Fc receptors and/or ability to fix complement (see e.g., Winter et al., GB 2,209,757 B; Morrison et al., WO 89/07142; Morgan et al., WO 94/29351, December 22, 1994). LDP-02 contains a heavy chain constant region (human γ1 heavy chain constant region) that was modified to reduce binding to human Fcγ receptors. The LDP-02 Fc modifications are at positions 235 and 237 (i.e., $Leu^{235} \rightarrow Ala^{235}$ and $Gly^{237} \rightarrow Ala^{237}$).

If present, framework regions of human origin (e.g., of the light chain variable region) are preferably derived from a human antibody variable region having sequence similarity to the analogous region (e.g., light chain variable region) of the antigen binding region donor. Other sources of framework regions for portions of human origin of a humanized immunoglobulin include human variable consensus sequences (see e.g., Kettleborough, C.A. et al., Protein Engineering 4:773-783 (1991); Carter et al., WO 94/04679, published March 3, 1994)). For example, the sequence of the antibody or variable region used to obtain the nonhuman portion can be compared to human sequences as described in Kabat, E.A., et al., Sequences of Proteins of Immunological Interest, Fifth Edition, U.S. Department of Health and Human Services, U.S. Government Printing Office (1991). In a particularly preferred embodiment, the framework regions of a humanized immunoglobulin chain are derived from a human variable region having at least about 65% overall sequence identity, and preferably at least about 70% overall sequence identity, with the variable region of the nonhuman donor antibody (e.g., mouse Act-1 antibody). A human portion can also be derived from a human antibody having at least about 65% sequence identity, and preferably at least about 70% sequence identity, within the particular portion (e.g., FR) being used, when compared to the equivalent portion (e.g., FR) of the nonhuman donor. Amino acid sequence identity can be determined using a suitable sequence alignment algorithm, such as the Lasergene system (DNASTAR, Inc., Madison, WI), using the default parameters.

In one embodiment, the humanized immunoglobulin comprises at least one of the framework regions (FR) derived from one or more chains of an antibody of human origin. Thus, the FR can include a FR1 and/or FR2 and/or FR3 and/or FR4 derived from one or more antibodies of human origin. Preferably, the human portion of a selected humanized chain includes FR1, FR2, FR3 and FR4 derived from a variable region of human origin (e.g., from a human immunoglobulin chain, from a human consensus sequence).

The immunoglobulin portions of nonhuman and human origin for use in preparing humanized antibodies can have sequences identical to immunoglobulins or immunoglobulin portions from which they are derived or to variants thereof. Such variants include mutants differing by the addition, deletion, or substitution of one or more residues. As indicated above, the CDRs which are of nonhuman origin are substantially the same as in the nonhuman donor, and preferably are identical to the CDRs of the nonhuman donor. Changes in the framework region, such as those which substitute a residue of the framework region of human origin with a residue from the corresponding position of the donor, can be made. One or more mutations in the framework region can be made, including deletions, insertions and substitutions of one or more amino acids. For a selected humanized antibody or chain, suitable framework mutations can be designed. Preferably, the humanized immunoglobulins can bind $\alpha 4\beta 7$ integrin with an affinity similar to or better than that of the nonhuman donor. Variants can be produced by a variety of suitable methods, including mutagenesis of nonhuman donor or acceptor human chains.

The selectively bind a determinant of the $\alpha 4\beta 7$ complex, but not bind determinants (epitopes) on the $\alpha 4$ chain or the $\beta 7$ chain. In one embodiment, the human or humanized immunoglobulin can have binding specificity for a combinatorial epitope on the $\alpha 4\beta 7$ heterodimer. Such an immunoglobulin can bind $\alpha 4\beta 7$ and not bind $\alpha 4\beta 1$, for example. Antibodies which have binding specificity for the $\alpha 4\beta 7$ complex include, murine Act-1 antibody and a humanized Act-1 referred to as LDP-02 (see, WO 98/06248 by LeukoSite, Inc., published February 19, 1998 and U.S. Application No. 08/700,737, filed August 15, 1996). In a preferred embodiment, the humanized immunoglobulin has at least one function characteristic of murine Act-1 antibody, such as binding function (e.g., having specificity for $\alpha 4\beta 7$ integrin, having the same or similar epitopic specificity), and/or inhibitory function (e.g., the ability to inhibit $\alpha 4\beta 7$-dependent adhesion *in vitro* and/or *in vivo,* such as the ability to inhibit $\alpha 4\beta 7$ integrin binding to MAdCAM-1 *in vitro* and/or *in vivo,* or the ability to inhibit the binding of a cell bearing $\alpha 4\beta 7$ integrin to a ligand thereof (e.g., a cell bearing MAdCAM-1)). Thus, preferred humanized immunoglobulins can have the binding specificity of the murine Act-1 antibody, the epitopic specificity of murine Act-1 antibody (e.g., can compete with murine Act-1, a chimeric Act-1 antibody, or humanized Act-1 (e.g., LDP-02) for binding to $\alpha 4\beta 7$ (e.g., on a cell bearing $\alpha 4\beta 7$ integrin)), and/or inhibitory function. A particularly preferred humanized Ab for administration in accordance with the method is LDP-02.

The binding function of a human or humanized immunoglobulin having binding specificity for $\alpha 4\beta 7$ integrin can be detected by standard immunological methods, for example using assays which monitor formation of a complex between humanized immunoglobulin and $\alpha 4\beta 7$ integrin (e.g., a membrane fraction comprising $\alpha 4\beta 7$ integrin, on a cell bearing $\alpha 4\beta 7$ integrin, such as a human lymphocyte (e.g., a lymphocyte of the CD4$^+\alpha 4^{hi}\beta 1^{lo}$ subset), human lymphocyte cell line or recombinant host cell comprising nucleic acid encoding $\alpha 4$ and/or $\beta 7$ which expresses $\alpha 4\beta 7$ integrin). Binding and/or adhesion assays or other suitable methods can also be used in procedures for the identification and/or isolation of immunoglobulins (e.g., human and/or humanized immunoglobulins) (e.g., from a library) with the requisite specificity (e.g., an assay which monitors adhesion between a cell bearing an $\alpha 4\beta 7$ integrin and a ligand thereof (e.g., a second cell expressing MAdCAM, an immobilized MAdCAM fusion protein (e.g., MAdCAM-Ig chimera)), or other suitable methods.

The immunoglobulin portions of nonhuman and human origin for use in preparing humanized immunoglobulins include light chains, heavy chains and portions of light and heavy chains. These immunoglobulin portions can be obtained or derived from immunoglobulins (e.g., by *de novo* synthesis of a portion), or nucleic acids encoding an immunoglobulin or chain thereof having the desired property (e.g., binds $\alpha 4\beta 7$ integrin, sequence similarity) can be produced and expressed. Humanized immunoglobulins comprising the desired portions (e.g., antigen binding region, CDR, FR, constant region) of human and nonhuman origin can be produced using synthetic and/or recombinant nucleic acids to prepare genes (e.g., cDNA) encoding the desired humanized chain. To prepare a portion of a chain, one or more stop codons can be introduced at the desired position. For example, nucleic acid (e.g., DNA) sequences coding for newly designed humanized variable regions can be constructed using PCR mutagenesis methods to alter existing DNA sequences (see e.g., Kamman, M., et al., Nucl. Acids Res. 17:5404 (1989)). PCR primers coding for the new CDRs can be hybridized to a DNA template of a previously humanized variable region which is based on the same, or a very similar, human variable region (Sato, K., et al., Cancer Research 53:851-856 (1993)). If a similar DNA sequence is not available for use as a template, a nucleic acid comprising a sequence encoding a variable region sequence can be constructed from synthetic oligonucleotides (see e.g., Kolbinger, F., Protein Engineering 8:971-980 (1993)). A sequence encoding a signal peptide can also be incorporated into the nucleic acid (e.g., on synthesis, upon insertion into a vector). If the natural signal peptide sequence is unavailable, a signal peptide sequence from another antibody can be used (see, e.g., Kettleborough, C.A., Protein Engineering 4:773-783 (1991)). Using these methods, methods described herein or other suitable methods, variants can be readily produced. In one embodiment, cloned variable regions (e.g., of LDP-02) can be mutagenized, and sequences encoding variants with the desired specificity can be selected (e.g., from a phage library; see e.g., Krebber et al., U.S. 5,514,548; Hoogenboom et al., WO 93/06213, published April 1, 1993)).

Human and/or humanized immunoglobulins can be administered (e.g., to a human) for therapeutic purposes in accordance with the use of the invention. For example, an effective amount of a humanized immunoglobulins having binding specificity for $\alpha4\beta7$ integrin can be administered to a human to treat a disease associated with leukocyte infiltration of mucosal tissues (i.e. inflammatory bowel disease, such as Crohn's disease or ulcerative colitis). Treatment includes therapeutic or prophylactic treatment (e.g., maintenance therapy). According to the invention, the disease can be prevented or delayed (e.g., delayed onset, prolonged remission or quiescence) or the severity of disease can be reduced in whole or in part.

In one embodiment, no more than about 8 mg of immunoglobulin per kg body weight is administered during a period of about 1 month. In additional embodiments, no more than about 7 or about 6 or about 5 or about 4 or about 3 or about 2 or about 1 mg of immunoglobulin per kg body weight is administered during a period of about 1 month. As used herein, the term "month" refers to a calendar month and encompasses periods of 28, 29, 30 and 31 days. When an antigen-binding fragment of a humanized immunoglobulin is to be administered, the amount which is administered during the period of about one month can be adjusted in accordance with the size of the fragment. For example, if the antigen-binding fragment is about half the size of the intact antibody by weight (e.g., measured in kDa), the amount administered during a period of about 1 month can be about 4 mg per kg body weight or less. The amount of immunoglobulin or antigen-binding fragment administered can be expressed as mg/kg body weight or using any other suitable units. For example, the amount of immunoglobulin or antigen-binding fragment administered can be expressed as moles of antigen binding sites per kg body weight. The number of moles of antigen-binding sites is dependent upon the size, quantity and valency of the immunoglobulin or fragment and is readily determined. For example, IgG and $F(ab')_2$ fragments thereof are divalent and a dose which comprises 1 nanomole of IgG or $F(ab')_2$ fragment comprises 2 nanomoles of antigen-binding sites. The size of an antibody or antigen-binding fragment can be determined using any suitable method (e.g., gel filtration).

The humanized antibody or antigen-binding fragment can be administered in an initial dose followed by one or more subsequent doses. When multiple doses are desired, the interval between doses and the amount of immunoglobulin or antigen-binding fragment can be adjusted to achieve the desired therapeutic and/or diagnostic effect and is as defined in the claims. For example, each of the doses to be administered can independently comprise up to about 8 mg immunoglobulin or fragment per kg body weight. When a dose comprises about 8 mg immunoglobulin or fragment per kg body weight the minimum interval before a subsequent dose is administered is a period of about 1 month. Preferably, each dose independently comprises about 0.1 to about 8 mg or about 0.1 to about 5 mg immunoglobulin or fragment per kg body weight. More preferably, each dose independently comprises about 0.1 to about 2.5 mg immunoglobulin or fragment per kg body weight. Most preferably, each dose independently comprises about 0.15, about 0.5, about 1.0, about 1.5 or about 2.0 mg immunoglobulin or fragment per kg body weight.

The interval between any two doses (e.g., initial dose and first subsequent dose, first subsequent dose and second subsequent dose) can independently vary from at least one day to about 120 days or more. For example, the initial dose can be administered and a first subsequent dose can be administered about 1 day later. Thereafter, second and third subsequent doses can be administered at intervals of about 1 month. Generally the minimum interval between doses is a period of at least about 1 day or at least about 7 days. In particular embodiments, the minimum interval between doses is a period of at least about 14 days, or at least about 21 days or at least about 1 month (e.g., 28, 29, 30, 31 days). In additional embodiments, the interval between doses can be at least about 40, about 50, about 60, about 70, about 80, about 90, about 100, about 110 or about 120 days.

The amount of humanized immunoglobulin or antigen-binding fragments thereof administered in each dose can be an amount which is sufficient to produce a desired pharmacokinetic or pharmacodynamic effect as defined in the claims. A variety of pharmacokinetic and pharmacodynamic parameters of human and/or humanized immunoglobulins or antigen-binding fragments thereof can be measured using suitable methods. For instance, pharmacodynamic parameters of antibodies and antigen-binding fragments (e.g., antigen saturation, antibody-induced inhibition of expression of antigen) can be measured using a suitable immunoassay. For example, as described herein, $\alpha4\beta7$ signal (i.e., binding of labeled antibody to $\alpha4\beta7$) following administration of LDP-02 was measured by flow cytometry. The results of the assay revealed that administration of LDP-02 can result in saturation of $\alpha4\beta7$ and/or inhibition of expression of $\alpha4\beta7$ on the surface of circulating lymphocytes.

Accordingly, each dose to be administered can comprise an amount of immunoglobulin or fragment which is sufficient to achieve a) about 50% or greater saturation of $\alpha4\beta7$ integrin binding sites on circulating lymphocytes (e.g., CD8[+] cells) and/or b) about 50% or greater inhibition of $\alpha4\beta7$ integrin expression on the cell surface of circulating lymphocytes for a period of at least about 10 days following administration of the dose. In other embodiments, each dose can comprise an amount of immunoglobulin or fragment which is sufficient to achieve and maintain a) about 60% or greater, about 70% or greater, about 80% or greater or about 85% or greater saturation of $\alpha4\beta7$ integrin binding sites

on circulating lymphocytes and/or b) about 60% or greater, about 70% or greater, about 80% or greater or about 85% or greater inhibition of $\alpha 4\beta 7$ integrin expression on the cell surface of circulating lymphocytes for a period of at least about 10 days following administration of the dose.

In other particular embodiments, each dose can comprise an amount of immunoglobulin or fragment which is sufficient to achieve a desired degree of saturation of $\alpha 4\beta 7$ integrin binding sites on circulating lymphocytes (e.g., CD8+ cells) and/or inhibit expression of $\alpha 4\beta 7$ integrin on the cell surface of circulating lymphocytes to the desired degree for a period of at least about 14 days, at least about 20 days, at least about 25 days or at least about one month following administration of the dose. In additional embodiments, each dose can comprise an amount of immunoglobulin or fragment which is sufficient to achieve a desired degree of saturation of $\alpha 4\beta 7$ integrin binding sites on circulating lymphocytes (e.g., CD8+ cells) and/or inhibit expression of $\alpha 4\beta 7$ integrin on the cell surface of circulating lymphocytes to the desired degree for a period of at least about 40, about 50, about 60, about 70, about 80, about 90, about 100, about 110 or about 120 days.

Suitable assays for determining the dose of antibody required to achieve a desired serum concentration or to saturate and/or inhibit expression of a target antigen can be readily designed. For example, a flow cytometry based assay can be used to measure $\alpha 4\beta 7$ expression on the surface of cells isolated from a subject following administration of an immunoglobulin (e.g., human, humanized) which binds to $\alpha 4\beta 7$. In one embodiment, a murine antibody which binds human $\alpha 4\beta 7$ can be used. Preferably the murine antibody can bind to an epitope on $\alpha 4\beta 7$ which is distinct from the epitope bound by the human or humanized immunoglobulin and the binding of the murine antibody to $\alpha 4\beta 7$ is not inhibited (e.g., blocked) by the prior binding of the humanized immunoglobulin. Murine antibodies or other antibodies with these properties can be prepared and selected using the methods described herein or other suitable methods. The level of $\alpha 4\beta 7$ expression on circulating lymphocytes (e.g., CD8+ cells) isolated from a human can be measured or determined using each of the antibodies (i.e., immunoglobulin to be administered, murine antibody) by flow cytometry or other suitable methods. Then, the humanized antibody to be administered can be administered to the human, peripheral blood can be drawn at predetermined times following the administration, and lymphocytes can be isolated (e.g., by density gradient centrifugation) for analysis. The peripheral blood lymphocytes (e.g., CD8+ cells) can be stained with each of the antibodies and the amount of $\alpha 4\beta 7$ detected by each antibody can be measured or detected by flow cytometry or other suitable methods. A decrease in the amount of $\alpha 4\beta 7$ integrin measured or determined using the human or humanized immunoglobulin is indicative of a) persistent integrin occupancy by the administered immunoglobulin (e.g., antigen saturation) and/or b) inhibition of $\alpha 4\beta 7$ expression on the surface of the lymphocytes (e.g., down modulation of $\alpha 4\beta 7$, shedding of $\alpha 4\beta 7$). A decrease in the amount of $\alpha 4\beta 7$ integrin measured or detected using the humanized immunoglobulin together with no change in the amount of $\alpha 4\beta 7$ integrin measured or determined using the murine antibody is indicative of persistent occupancy of $\alpha 4\beta 7$ (e.g., saturation) by the humanized immunoglobulin administered. A decrease in the amount of $\alpha 4\beta 7$ integrin measured or detected using the humanized immunoglobulin together with a decrease in the amount of $\alpha 4\beta 7$ integrin measured or detected using the murine antibody is indicative of inhibition of $\alpha 4\beta 7$ expression on the surface of circulating lymphocytes.

Pharmacokinetic parameters, such as the serum concentration of antibody over time following administration of said antibody can be measured using an immunoassay such as an ELISA or cell-based assay. For example, as described herein, the serum concentration of a humanized anti-$\alpha 4\beta 7$ immunoglobulin (LDP-02) at predetermined time points following a single administration of antibody (LDP-02) was measured using a cell-based assay. The results of the assay revealed that the serum concentration of LDP-02 can remain elevated (e.g., at or above 1 $\mu$g/mL) for a period of about 10 days or more following administration of the humanized antibody. The prolonged presence of LDP-02 in the serum can be indicative of superior efficacy as a result of persistent inhibition of $\alpha 4\beta 7$ function, for example persistent inhibition of $\alpha 4\beta 7$ mediated adhesion of leukocytes to MAdCAM.

Accordingly, each dose to be administered can comprise an amount of immunoglobulin or fragment which is sufficient to achieve and maintain a serum concentration of at least about 1 $\mu$g/mL for a period of at least about 10 days following administration of the dose. In particular embodiments, each dose can comprise an amount of immunoglobulin or fragment which is sufficient to achieve and maintain a serum concentration of at least about 1 $\mu$g/mL for a period of at least about 14 days, at least about 20 days, at least about 25 days or at least about one month following administration of the dose. In additional embodiments, each dose can comprise amount of immunoglobulin or fragment which is sufficient to achieve and maintain a serum concentration of at least about 1 $\mu$g/mL for a period of at least about 40, about 50, about 60, about 70, about 80, about 90, about 100, about 110 or about 120 days.

As discussed herein, antigen-binding fragments of a humanized immunoglobulin can be substantially smaller and, therefore, bind more antigen ($\alpha 4\beta 7$) per unit of protein ($\mu$g) than intact or native immunoglobulin. Accordingly, the serum concentration of an antigen-binding fragment of a humanized immunoglobulin which can be indicative of superior efficacy can be lower than 1 $\mu$g/mL. Thus, when administration of an antigen-binding fragment of a humanized immunoglobulin is desired, the dose can comprise an amount of antigen-binding fragment which is sufficient to achieve a serum concentration which is proportionate to 1 $\mu$g/mL for an intact immunoglobulin. For example, if the antigen-binding fragment is about half the size of the intact antibody by weight (e.g., measured in kDa), the dose can comprise an amount sufficient to achieve and maintain a serum concentration of about 0.5 μg/mL for a period of at least about 10 days. The desired serum concentration of immunoglobulin or antigen-binding fragment can be expressed as μg/mL or using any other suitable units. For example, the amount of immunoglobulin or antigen-binding fragment administered can be expressed as moles of antigen binding sites per volume of serum (e.g., M).

Humanized immunoglobulins can be administered in accordance with the present invention to modulate α4β7 integrin function in therapeutic (including prophylactic) applications. In reference aspects, human and humanized immunoglobulins can be used to detect and/or measure the level of an α4β7 integrin in a subject. For example, a humanized immunoglobulin having binding specificity for α4β7 integrin can be administered to a human and antibody-α4β7 integrin complexes which are formed can be detected using suitable methods. For example, the humanized antibody can be labeled with, for example, radionuclides ($^{125}$I, $^{111}$In, technetium-99m), an epitope label (tag), an affinity label (e.g., biotin, avidin), a spin label, an enzyme, a fluorescent group or a chemiluminescent group and suitable detection methods can be used. In an application of the method, humanized immunoglobulins can be used to analyze normal versus inflamed tissues (e.g., from a human) for α4β7 integrin reactivity and/or expression (e.g. radiologically) or to detect associations between IBD or other conditions and increased expression of α4β7 (e.g., in affected tissues). The immunoglobulins described herein can be administered in accordance with the specification for assessment of the presence of α4β7 integrin in normal versus inflamed tissues, through which the presence of disease, disease progress and/or the efficacy of anti-α4β7 integrin therapy in inflammatory disease can be assessed.

Humanized immunoglobulins (including antigen-binding fragments) can be administered to an individual to modulate (e.g., inhibit (reduce or prevent)) binding function and/or leukocyte (e.g., lymphocyte, monocyte) infiltration function of α4β7 integrin. For example, human and humanized immunoglobulins which inhibit the binding of α4β7 integrin to a ligand (i.e., one or more ligands) can be administered according to the inventive use for the treatment of diseases associated with leukocyte (e.g., lymphocyte, monocyte) infiltration of tissues (including recruitment and/or accumulation of leukocytes in tissues), particularly of tissues which express the molecule MAdCAM. An effective amount of a humanized immunoglobulin or antigen-binding fragment thereof (i.e., one or more immunoglobulins or fragments) is administered to an individual (e.g., a mammal, such as a human or other primate) in order to treat such a disease. For example, inflammatory diseases, including diseases which are associated with leukocyte infiltration of the gastrointestinal tract (including gut-associated endothelium), other mucosal tissues, or tissues expressing the molecule MAdCAM-1 (e.g., gut-associated tissues, such as venules of the lamina propria of the small and large intestine; and mammary gland (e.g., lactating mammary gland)), can be treated according to the present method. Similarly, an individual having a disease associated with leukocyte infiltration of tissues as a result of binding of leukocytes to cells (e.g., endothelial cells) expressing MAdCAM-1 can be treated according to the present invention.

Diseases which can be treated accordingly are inflammatory bowel diseases (IBD), such as ulcerative colitis, Crohn's disease, ileitis, Celiac disease, nontropical Sprue, enteropathy associated with seronegative arthropathies, microscopic or collagenous colitis, eosinophilic gastroenteritis, or pouchitis resulting after proctocolectomy, and ileoanal anastomosis.

Pancreatitis and insulin-dependent diabetes mellitus are other diseases which can be treated in reference aspects. It has been reported that MAdCAM-1 is expressed by some vessels in the exocrine pancreas from NOD (nonobese diabetic) mice, as well as from BALB/c and SJL mice. Expression of MAdCAM-1 was reportedly induced on endothelium in inflamed islets of the pancreas of the NOD mouse, and MAdCAM-1 was the predominant addressin expressed by NOD islet endothelium at early stages of insulitis (Hanninen, A., et *al*., J. Clin. Invest., 92: 2509-2515 (1993)). Further, accumulation of lymphocytes expressing α4β7 within islets was observed, and MAdCAM-1 was implicated in the binding of lymphoma cells via α4β7 to vessels from inflamed islets (Hanninen, A., et al., J. Clin. Invest., 92: 2509-2515 (1993)).

Examples of inflammatory diseases associated with mucosal tissues which can be treated according to the present specification include mastitis (mammary gland), cholecystitis, cholangitis or pericholangitis (bile duct and surrounding tissue of the liver), chronic bronchitis, chronic sinusitis, asthma, and graft versus host disease (e.g., in the gastrointestinal tract). As seen in Crohn's disease, inflammation often extends beyond the mucosal surface, accordingly chronic inflammatory diseases of the lung which result in interstitial fibrosis, such as hypersensitivity pneumonitis, collagen diseases, sarcoidosis, and other idiopathic conditions can be amenable to treatment.

Treatment can be curative, induce remission or quiescence or prevent relapse or recurrence of active disease. According to the method, treatment can be episodic or chronic (e.g., chronic treatment of active disease, to maintain quiescent disease, to induce quiescence and maintain quiescence), for example.

The humanized immunoglobulin having binding specificity for α4β7 integrin is administered to a human having inflammatory bowel disease, such as ulcerative colitis or Crohn's disease. The immunoglobulin can be administered to treat active disease and/or to maintain quiescence (i.e., inhibit relapse or recurrence). In a particular embodiment, the humanized immunoglobulin can be administered to maintain quiescence of inflammatory bowel disease which has been induced by treatment with one or more other agents (e.g., steroids (prednisone, prednisolone, adrenocorticotrophic hormone (ACTH)), cyclosporin A, FK506, antibody having binding specificity for TNFα (infliximab, CDP571), azathioprene, 6-mercaptopurine, 5-aminosalicylic acid (5-ASA) or compounds containing 5-ASA (e.g., sulfsalazine, olsalazine, balsalazide), antibiotics (e.g., metronidazole), interleukins (IL-10, IL-11), nicotine, heparin, thalidomide, lidocane) or surgery (e.g., intestinal resection).

The humanized immunoglobulin or antigen-binding fragment thereof is administered in an effective amount. For therapy, an effective amount is an amount sufficient to achieve the desired therapeutic (including prophylactic) effect as defined in the claims (such as, an amount sufficient to reduce or prevent $\alpha 4\beta 7$ integrin-mediated binding to a ligand thereof and/or signalling, thereby inhibiting leukocyte adhesion and infiltration and/or associated cellular responses; an amount sufficient to induce remission or prevent relapse or recurrence of disease). The humanized immunoglobulin or antigen-binding fragment thereof can be administered in a single dose or in an initial dose followed by one or more subsequent doses as described herein. The amount of immunoglobulin or antigen-binding fragment administered in a particular dose as well as the interval between doses can depend on the characteristics of the individual, such as general health, age, sex, body weight and tolerance to drugs as well as the type and severity of disease. The skilled artisan will be able to determine appropriate dosages depending on these and other factors.

According to the present use, the humanized immunoglobulin of the claims can be administered to an individual (e.g., a human) alone or in conjunction with another agent (i.e., one or more additional agents). A humanized immunoglobulin can be administered before, along with or subsequent to administration of the additional agent. In one embodiment, more than one human or humanized immunoglobulin which inhibits the binding of $\alpha 4\beta 7$ integrin to its ligands is administered. In another embodiment, an antibody (e.g, human antibody, humanized antibody), such as an anti-MAdCAM-1, anti-VCAM-1, or anti-ICAM-1 antibody, which inhibits the binding of leukocytes to an endothelial ligand is administered in addition to a human or humanized immunoglobulin which binds $\alpha 4\beta 7$ integrin. In yet another embodiment, an additional pharmacologically active ingredient (e.g., an antiinflammatory compound, such as 5-aminosalicylic acid (5-ASA) or compounds containing 5-ASA (e.g., sulfsalazine, olsalazine, balsalazide), another non-steroidal antiinflammatory compound, or a steroidal antiinflammatory compound (e.g., prednisone, prednisolone, adrenocorticotrophic hormone (ACTH)), immunosuppressive agents (azathioprene, 6-mercaptopurine, cyclosporin A, FK506), immunomodulators (e.g., antibody having binding specificity for TNF$\alpha$ (infliximab, CDP571), thalidomide, interleukins (e.g., recombinant human IL-10, recombinant human IL-11)), antibiotics (e.g., metronidazole), nicotine, heparin, lidocaine) can be administered in conjunction with a humanized immunoglobulin of the present invention.

A variety of routes of administration are possible, including, but not necessarily limited to, parenteral (e.g., intravenous, intraarterial, intramuscular, intrathecal, subcutaneous injection), oral (e.g., dietary), topical, inhalation (e.g., intrabronchial, intranasal or oral inhalation, intranasal drops), or rectal, depending on the disease or condition to be treated. Parenteral administration, particularly intravenous injection and subcutaneous injection, is preferred.

The humanized immunoglobulin or antigen-binding fragment thereof can be administered to the individual as part of a pharmaceutical or physiological composition for the treatment of a disease associated with leukocyte infiltration of mucosal tissues (e.g., inflammatory bowel disease (e.g., ulcerative colitis, Crohn's disease). Such a composition comprises an immunoglobulin or antigen-binding fragment having binding specificity for $\alpha 4\beta 7$ integrin as described herein, and a pharmaceutically or physiologically acceptable carrier. Pharmaceutical or physiological compositions for co-therapy can comprise an immunoglobulin or antigen-binding fragment having binding specificity for $\alpha 4\beta 7$ integrin and one or more additional therapeutic agents. An immunoglobulin or antigen-binding fragment having binding specificity for $\alpha 4\beta 7$ integrin function and an additional therapeutic agent can be components of separate compositions which can be mixed together prior to administration or administered separately. Formulation will vary according to the route of administration selected (e.g., solution, emulsion, capsule). Suitable carriers can contain inert ingredients which do not interact with the immunoglobulin or antigen-binding fragment and/or additional therapeutic agent. Standard pharmaceutical formulation techniques can be employed, such as those described in Remington's Pharmaceutical Sciences, Mack Publishing Company, Easton, PA. Suitable carriers for parenteral administration include, for example, sterile water, physiological saline, bacteriostatic saline (saline containing about 0.9% benzyl alcohol), phosphate-buffered saline, Hank's solution, Ringer's-lactate and the like. Methods for encapsulating compositions (such as in a coating of hard gelatin or cyclodextran) are known in the art (Baker, et al., "Controlled Release of Biological Active Agents", John Wiley and Sons, 1986). For inhalation, the agent can be solubilized and loaded into a suitable dispenser for administration (e.g., an atomizer, nebulizer or pressurized aerosol dispenser).

The present invention will now be illustrated by the following Examples, which are not intended to be limiting in any way.

EXAMPLES

Introduction

LDP-02 is a humanized IgG1 monoclonal antibody that binds $\alpha 4\beta 7$ integrin, a cell surface glycoprotein present on the surface of most T and B lymphocytes. $\alpha 4\beta 7$ mediates lymphocyte trafficking to gastrointestinal mucosa and gut-associated lymphoid tissue through adhesion interaction with the homing receptor MAdCAM-1. By blocking α4β7-MAd-CAM-1 interactions, LDP-02 can inhibit the recruitment of leukocytes from the vasculature to the gastrointestinal mucosa, thus having a beneficial effect on the inflammatory activity in patients afflicted with inflammatory bowel disease (IBD) such as ulcerative colitis and Crohn's Disease.

This section presents information from the two LDP-02 clinical trials that have been completed. These trials include one completed Phase I study conducted in healthy subjects (Study L297-007) and one completed Phase Ib/IIa trials in patients with ulcerative colitis (UC)(Study L297-006). Table 1 describes each of the studies.

Table 1

| Study No. # Sites Country | Study Status | Study Design/ Population | Dosing Regimen, Dose, Route | Number of Subjects Enrolled |
|---|---|---|---|---|
| L297-007<br><br>1<br><br>UK | Completed | Phase I, randomized, double-blind, placebo-controlled, ascending single dose study.<br><br><br>Healthy Male Subjects<br>18-50 years of age | Day 1 (single dose)<br>0.15 mg/kg IV<br>0.15 mg/kg SC<br>0.5 mg/kg IV<br>1.5 mg/kg IV<br>2.5 mg/kg IV | Total= 19<br><br>LDP-02= 14<br>Placebo= 5 |
| L297-006<br><br>5<br><br>Canada | Completed | Phase Ib/IIa, randomized, double-blind, placebo-controlled, single rising dose, multicenter study.<br><br><br>Patients with moderately severe ulcerative colitis. Prior steroid use was limited (≤20mg/day). Use of 5-ASAs was allowed. | Day 1 (single dose)<br>0.15 mg/kg SC<br>0.15 mg/kg IV<br>0.5 mg/kg IV<br>2.0 mg/kg IV<br>placebo IV | Total= 29<br><br>LDP-02= 21<br>Placebo= 8 |

Example 1: Study L297-007

Study L297-007 entitled, "A Placebo-Controlled, Double-Blind, Rising Dose Study Investigating the Tolerability, Pharmacodynamics and Pharmacokinetics of LDP-02 Given by the Subcutaneous and Intravenous Routes in Healthy Male Volunteers" has been completed and final results are presented in this section.

Study Design

Study L297-007 was a randomized, double-blind, placebo-controlled, ascending single-dose study in healthy male volunteers. Healthy male volunteers 18 to 50 years of age meeting all inclusion/exclusion criteria were enrolled in the study sequentially by study group and, within each study group, were randomly assigned to receive LDP-02 or placebo (i.e., isotonic sodium citrate buffer). To minimize risk to subjects, safety and tolerability were reviewed at each dose level prior to escalating to the next dose level. The treatment groups and numbers of subjects planned for the study are shown in Table 2.

Table 2 Study L297-007: Study Groups

| Group | Route of Administration * | LDP-02<br># subjects Dose | | Placebo # subjects |
|---|---|---|---|---|
| 1 | IV<br>SC | 3<br>3 | 0.15 mg/kg<br>0.15 mg/kg | 1<br>1 |
| 2 | IV | 3 | 0.5 mg/kg | 1 |
| 3 | IV | 3 | 1.5 mg/kg | 1 |

(continued)

| Group | Route of Administration * | LDP-02 # subjects Dose | | Placebo # subjects |
|---|---|---|---|---|
| 4 | IV | 3 | 2.5 mg/kg | 1 |
| *SC= subcutaneous administration; IV = intravenous administration | | | | |

On study Day 1, LDP-02 or placebo was administered either SC into the thigh (Group 1 SC dosing only) or via a 30 minute constant rate IV infusion (Groups 1-4). Safety assessments included recording of adverse events, physical examinations, vital signs, clinical laboratories (i.e., hematology, blood chemistries, and urinalysis), plasma cytokine levels, and 12-lead electrocardiograms (ECGs). In addition, since this was the first clinical trial of LDP-02, continuous cardiac monitoring was carried out pre-dose through 4 hours post-dose. Blood samples were obtained to assess anti-antibody response to LDP-02, cytokine levels, serum LDP-02 concentration (pharmacokinetics), and saturation and binding site occupation of $\alpha4\beta7$ receptors and lymphocyte subsets (pharmacodynamics). Study assessments were conducted at specified times through 36 days post-treatment. Following the results of the Day 36 pharmacokinetic and pharmacodynamic (immunological) analyses, the protocol was amended to allow additional blood draws for subjects who received LDP-02. These blood draws were used to follow LDP-02 serum levels until they became non-quantifiable (i.e., below the limit of quantification [BLQ]) and to ensure that $\alpha4\beta7$ saturation and memory cell populations had returned to baseline (pre-dose) levels. This amendment was particularly important in the higher dose groups where the characteristics of terminal phase kinetics were not well established by Day 36.

Study Results

Pharmacokinetics

The assay of LDP-02 in serum was performed using a validated cell-based assay. Standards and samples were incubated with a target cell line (HUT-78) which expresses the $\alpha4\beta7$ antigen. After washing, a fluorescently labeled polyclonal antihuman IgG1 was added. Fluorescence intensity was measured by flow cytometry and compared with the fluorescence intensity of LDP-02 standards. The effective serum concentration of LDP-02 was then defined by comparison of the sample with a standard curve generated with known concentrations of LDP-02.

Blood samples for determination of LDP-02 serum concentration were collected pre-dose, 1, 1.5, 3, 8, 12 and 24 hours after dosing, and on Days 3, 5, 7, 8, 15, 22, and 36. When it became known that LDP-02 was still detectable at Day 36, blood draws for subjects who received LDP-02 continued until levels had fallen to below the limits of quantitation of the assay. Thirteen of the 14 subjects who received LDP-02 returned for follow-up blood draws up to a maximum of 226 days post-dose.

LDP-02 concentrations over time by individual patient and mean pharmacokinetic parameters by LDP-02 dose group are presented in the Appendix to Study L297-007. Mean LDP-02 serum concentrations over time are plotted out to the last blood draw for all treatment groups in FIG. 6.

Table 3 Study L297-007: Mean Pharmacokinetic Parameters of LDP-02 in Healthy Subjects[1]

| Pharmacokinetic Parameter | Dose and Route of Administration of LDP-02 (number of subjects) | | | | |
|---|---|---|---|---|---|
| | 0.15 mg/kg SC (n=3) | 0.15mg/kg IV (n=3) | 0.5 mg/kg IV (n=3) | 1.5 mg/kg IV (n=3) | 2.5 mg/kg IV (n=2) |
| $C_{max}$ ($\mu$g/mL) | 1.112 (0.519) | 7.648 (3.201) | 15.760 (7.476) | 118.813 (14.544) | 101.749 (5.117) |
| $t_{max}$ (days) (median & range) | 6.01 (4.01 - 6.01) | 0.13 (0.04 - 0.33) | 0.5 (0.06 - 0.5) | 0.13 (0.06-0.33) | 0.05 (0.04-0.06) |
| $T_{1/2z}$ (days) | 4.33 (2.23) | 4.39 (1.51) | 4.02 (0.71) | 14.9 (10.3) | 17.1 (8.91) |
| $AUC_t$ ($\mu$g·day/mL) | 10.4 (4.40) | 19.5 (5.00) | 83.6 (18.3) | 660 (229) | 1651 (229) |
| $\lambda_z$ (1/day) | 0.1852 (0.0735) | 0.1731 (0.0673) | 0.1763 (0.0344) | 0.0994 (0.1145) | 0.0469 (0.0244) |
| AUC ($\mu$g·day/mL) | 11.4 (5.80) | 20.3 (5.88) | 85.1 (18.2) | 755 (308) | 1747 (95.8) |

(continued)

| Pharmacokinetic Parameter | Dose and Route of Administration of LDP-02 (number of subjects) | | | | |
|---|---|---|---|---|---|
| | 0.15 mg/kg SC (n=3) | 0.15mg/kg IV (n=3) | 0.5 mg/kg IV (n=3) | 1.5 mg/kg IV (n=3) | 2.5 mg/kg IV (n=2) |
| AUC Extrapolated % | 5.9 (7.3) | 3.4 (3.2) | 1.8 (1.4) | 9.5 (16.1) | 5.7 (8.0) |
| CL* (mLday/kg) | 15.3 (6.26) | 7.75 (1.93) | 6.06 (1.32) | 2.31 (1.19) | 1.43 (0.08) |
| $V_z$* (mL/kg) | 82.5 (6.88) | 46.6 (10.1) | 34.3 (2.84) | 54.0 (51.4) | 35.9 (20.3)_ |
| [1]All values are mean +/- SD unless otherwise indicated. The SD appears in parenthesis. *Clearance and volume terms for the SC dose group are the apparent clearance (CL/F) and apparent volume ($V_z$/F). | | | | | |

Values were obtained for the mean single dose IV pharmacokinetic parameters for the 4 dose groups ($C_{max}$, $t_{1/2z}$ and AUC). Follow-up samples (i.e., those taken beyond Day 36), where the focus was on safety, allowed some further characterization of the concentration-time profiles. The difference in the $t_{1/2z}$ values between the 2 lower dose groups (0.15 and 0.5 mg/kg) and the higher dose groups (1.5 and 2.5 mg/kg) of around 10 days could be explained in that the "true" terminal phase for the higher dose groups had not been characterized. The non-compartmental pharmacokinetics of the lower doses of LDP-02 (0.15 and 0.5 mg/kg) were well characterized and non-linear pharmacokinetics became evident as the dose was increased up to 2.5 mg/kg.

Assessment of the Pharmacodynamic Effect of LDP-02

Fluorescece-activated cell sorting (FACS) analysis was used to measure the presence of $\alpha 4\beta 7$ sites on peripheral blood lymphocytes pre- and post-LDP-02 administration. To detect $\alpha 4\beta 7$ that were recognized by antibody, biotin labeled ACT-1, the murine homologue of LDP-02, was added to samples of patient blood and detected using PE-streptavidin. The standardized mean equivalent soluble fluorescence (MESF) is proportional to the number of detectable $\alpha 4\beta 7$ sites.

Serum $\alpha 4\beta 7$ binding over time (MESF values and percentage of baseline at each post-dose time point) are presented by individual subject and by treatment group in the Appendix to Study L297-007.

As measured by FACS analysis, mean saturation of $\alpha 4\beta 7$ integrin on lymphocytes over time (i.e., to Day 36) for each treatment is presented in FIG. 7. As seen in FIG. 7, there was no detection of free $\alpha 4\beta 7$ binding sites on lymphocytes for at least two weeks following administration of all LDP-02 doses. Between about day 7 and day 22, $\alpha 4\beta 7$ signal started to return to baseline for the 0.15 mg/kg IV dose group and for the 0.15 mg/kg SC dose group. Between day 22 and day 36, $\alpha 4\beta 7$ signal started to return to baseline for the 0.5 mg/kg IV dose group. At the higher doses of LDP-02 studied (1.5, and 2.5 mg/kg) loss of $\alpha 4\beta 7$ signal persisted for longer than 36 days following single IV doses. For the 2.5 mg/kg dose group, $\alpha 4\beta 7$ binding saturation continued up to Day 70 (see, data in Appendix to Study L297-007).

Follow-up blood sampling up to about Study Day 200 was done to confirm that free $\alpha 4\beta 7$ binding sites on lymphocytes has returned to baseline (pre-dose) levels. The initial reappearance of free $\alpha 4\beta 7$ sites appeared to occur when LDP-02 blood concentrations became non-detectable.

Conclusions

The administration of LDP-02 at IV doses of 0.15, 0.50, 1.50, and 2.5 mg/kg and a SC dose of 0.15 mg/kg to healthy male subjects was well-tolerated.

Following administration of all LDP-02 doses there was no detection of free $\alpha 4\beta 7$ binding sites on lymphocytes for approximately two weeks post-dose. Saturation of $\alpha 4\beta 7$ binding sites continued for up to approximately 2 weeks post-dosing for the 0.15 mg/kg IV group and for up to approximately 3 weeks post-dosing for the 0.15 mg/kg SC and 0.5 mg/kg IV groups. Duration of effect persisted for a month or longer with the 1.5 mg/kg IV dose and continued to approximately Day 70 with 2.5 mg/kg LDP-02 IV. Follow-up samples obtained after Day 36 demonstrated that expression of free $\alpha 4\beta 7$ binding sites had returned to baseline (pre-dose levels). No anti-idiotype antibodies were raised to LDP-02 indicating that it did not initiate a humoral immunogenic response. The non-compartmental pharmacokinetics of the lower doses of LDP-02 (0.15 and 0.5 mg/kg) became evident as the dose was increased up to 2.5 mg/kg.

APPENDIX TO STUDY L297-007

LDP-02 Serum Concentration Over Time by Subject by Treatment Group. Data from individual patients are presented in Tables 4-9.

Table 4 0.15 mg/kg LDP-02 IV

| Subject #2 | | | Subject # 3 | | | Subject # 4 | | | Mean |
|---|---|---|---|---|---|---|---|---|---|
| Time (hr) | Time (day) | μg/mL | Time (hr) | Time (day) | μg/mL | Time (hr) | Time (day) | μg/mL | μg/mL (n=3) |
| Pre-Dose | Pre-Dose | 0.01 | Pre-Dose | Pre-Dose | 0.01 | Pre-Dose | Pre-Dose | 0.01 | 0.01 |
| 1.0 | 0.042 | 5.24 | 1.0 | 0.042 | 7.98 | 1.0 | 0.042 | 2.48 | 5.24 |
| 1.5 | 0.063 | 5.33 | 1.5 | 0.063 | 6.21 | 1.5 | 0.063 | 3.42 | 4.99 |
| 3.0 | 0.125 | 5.47 | 3.0 | 0.125 | 4.66 | 3.0 | 0.125 | 4.29 | 4.81 |
| 8.0 | 0.333 | 10.67 | 8.0 | 0.333 | 5.10 | 8.0 | 0.333 | 3.26 | 6.34 |
| 12.0 | 0.500 | 4.49 | 12.0 | 0.500 | 4.50 | 12.0 | 0.500 | 2.42 | 3.80 |
| 24.0 | 1.000 | 3.23 | 24.0 | 1.000 | 3.63 | 24.0 | 1.000 | 2.24 | 3.03 |
| 72.0 | 3.000 | 1.84 | 72.0 | 3.000 | 2.94 | 72.0 | 3.000 | 3.05 | 2.61 |
| 120.0 | 5.000 | 1.21 | 120.0 | 5.000 | 1.84 | 120.0 | 5.000 | 1.16 | 1.40 |
| 168.0 | 7.000 | 0.94 | 168.0 | 7.000 | 1.29 | 168.0 | 7.000 | 0.74 | 0.99 |
| 192.0 | 8.000 | 0.62 | 192.0 | 8.000 | 1.13 | 192.0 | 8.000 | 0.70 | 0.82 |
| 360.0 | 15.000 | 0.04 | 360.0 | 15.000 | 0.53 | 360.0 | 15.000 | 0.26 | 0.28 |
| 528.0 | 22.000 | 0.02 | 528.0 | 22.000 | 0.21 | 528.0 | 22.000 | 0.09 | 0.10 |
| 864.0 | 36.000 | 0.02 | 864.0 | 36.000 | 0.01 | 864.0 | 36.000 | 0.01 | 0.01 |
| | | | 3912.0 | 163.000 | 0.01 | 3912.0 | 163.000 | 0.01 | 0.01 |
| | | | 4920.0 | 205.000 | 0.01 | 4752.0 | 198.000 | 0.01 | 0.01 |

Table 5 0.15 mg/kg LDP-02 SC

| Subject # 5 | | | Subject # 6 | | | Subject # 8 | | | Mean |
|---|---|---|---|---|---|---|---|---|---|
| Time (hr) | Time (day) | μg/mL | Time (hr) | Time (day) | μg/mL | Time (hr) | Time (day) | μg/mL | μg/mL (n=3) |
| Pre-Dose | Pre-Dose | 0.01 | Pre-Dose | Pre-Dose | 0.01 | Pre-Dose | Pre-Dose | 0.01 | 0.01 |
| 1.0 | 0.042 | 0.01 | 1.0 | 0.042 | 0.01 | 1.0 | 0.042 | 0.01 | 0.01 |
| 1.5 | 0.063 | 0.01 | 1.5 | 0.063 | 0.01 | 1.5 | 0.063 | 0.01 | 0.01 |
| 3.0 | 0.125 | 0.01 | 3.0 | 0.125 | 0.01 | 3.0 | 0.125 | 0.01 | 0.01 |
| 8.0 | 0.333 | 0.06 | 8.0 | 0.333 | 0.09 | 8.0 | 0.333 | 0.09 | 0.08 |
| 12.0 | 0.500 | 0.11 | 12.0 | 0.500 | 0.12 | 12.0 | 0.500 | 0.10 | 0.11 |
| 24.0 | 1.000 | 0.12 | 24.0 | 1.000 | 0.30 | 24.0 | 1.000 | 0.55 | 0.32 |
| 72.0 | 3.000 | 0.23 | 72.0 | 3.000 | 0.81 | 72.0 | 3.000 | 0.91 | 0.65 |
| 120.0 | 5.000 | 0.54 | 120.0 | 5.000 | 0.93 | 120.0 | 5.000 | 1.13 | 0.86 |
| 168.0 | 7.000 | 0.71 | 168.0 | 7.000 | 0.88 | 168.0 | 7.000 | 1.70 | 1.10 |
| 192.0 | 8.000 | 0.62 | 192.0 | 8.000 | 0.81 | 192.0 | 8.000 | 1.05 | 0.83 |
| 360.0 | 15.000 | 0.28 | 360.0 | 15.000 | 0.08 | 360.0 | 15.000 | 0.53 | 0.30 |
| 528.0 | 22.000 | 0.02 | 528.0 | 22.000 | 0.03 | 528.0 | 22.000 | 0.26 | 0.11 |
| 864.0 | 36.000 | 0.04 | 864.0 | 36.000 | 0.04 | 864:0 | 36.000 | 0.01 | 0.03 |
| 3912.0 | 163.000 | 0.01 | 3912.0 | 163.000 | 0.01 | 3912.0 | 163.000 | 0.01 | 0.01 |
| 5088.0 | 212.000 | 0.01 | 5088.0 | 212.000 | 0.01 | 5088.0 | 212.000 | 0.01 | 0.01 |

Table 6 0.5 mg/kg LDP-02 IV

| Subject # 9 | | | Subject # 10 | | | Subject # 12 | | | Mean µg/mL (n=3) |
|---|---|---|---|---|---|---|---|---|---|
| Time (hr) | Time (day) | µg/mL | Time (hr) | Time (day) | µg/mL | Time (hr) | Time (day) | µg/mL | |
| Pre-Dose | Pre-Dose | 0.01 | Pre-Dose | Pre-Dose | 0.01 | Pre-Dose | Pre-Dose | 0.01 | 0.01 |
| 1.0 | 0.042 | 9.06 | 1.0 | 0.042 | 10.74 | 1.0 | 0.042 | 10.93 | 10.24 |
| 1.5 | 0.063 | 24.39 | 1.5 | 0.063 | 6.62 | 1.5 | 0.063 | 8.17 | 13.06 |
| 3.0 | 0.125 | 16.37 | 3.0 | 0.125 | 10.14 | 3.0 | 0.125 | 9.94 | 12.15 |
| 8.0 | 0.333 | 15.04 | 8.0 | 0.333 | 9.30 | 8.0 | 0.333 | 9.35 | 11.23 |
| 12.0 | 0.500 | 10.64 | 12.0 | 0.500 | 11.70 | 12.0 | 0.500 | 11.19 | 11.18 |
| 24.0 | 1.000 | 9.17 | 24.0 | 1.000 | 9.00 | 24.0 | 1.000 | 8.52 | 8.90 |
| 72.0 | 3.000 | 5.34 | 72.0 | 3.000 | 7.55 | 72.0 | 3.000 | 7.60 | 6.83 |
| 120.0 | 5.000 | 10.25 | 120.0 | 5.000 | 2.43 | 120.0 | 5.000 | 8.58 | 7.09 |
| 168.0 | 7.000 | 5.74 | 168.0 | 7.000 | 6.59 | 168.0 | 7.000 | 4.93 | 5.75 |
| 192.0 | 8.000 | 3.79 | 192.0 | 8.000 | 2.48 | 192.0 | 8.000 | 4.32 | 3.53 |
| 360.0 | 15.000 | 1.70 | 360.0 | 15.000 | 2.21 | 360.0 | 15.000 | 2.49 | 2.13 |
| 528.0 | 22.000 | 0.41 | 528.0 | 22.000 | 0.12 | 528.0 | 22.000 | 1.65 | 0.73 |
| 864.0 | 36.000 | 0.01 | 864.0 | 36.000 | 0.01 | 864.0 | 36.000 | 0.11 | 0.04 |
| 3576.0 | 149.00 | 0.01 | 3912.0 | 163.000 | 0.01 | 3576.0 | 149.000 | 0.01 | 0.01 |
| | | | 5424.0 | 226.000 | 0.01 | | | | 0.01 |

Table 7 1.5 mg/kg LDP-02 IV

| Subject # 13 | | | Subject # 15 | | | Subject # 16 | | | Mean µg/mL (n=3) |
|---|---|---|---|---|---|---|---|---|---|
| Time (hr) | Time (day) | µg/mL | Time (hr) | Time (day) | µg/mL | Time (hr) | Time (day) | µg/mL | |
| Pre-Dose | Pre-Dose | 0.01 | Pre-Dose | Pre-Dose | 0.01 | Pre-Dose | Pre-Dose | 0.01 | 0.01 |
| 1.0 | 0.042 | 87.62 | 1.0 | 0.042 | 58.06 | 1.0 | 0.042 | 103.10 | 82.93 |
| 1.5 | 0.063 | 63.67 | 1.5 | 0.063 | 134.97 | 1.5 | 0.063 | 86.05 | 94.90 |
| 3.0 | 0.125 | 92.78 | 3.0 | 0.125 | 63.78 | 3.0 | 0.125 | 106.78 | 87.78 |
| 8.0 | 0.333 | 114.69 | 8.0 | 0.333 | 64.12 | 8.0 | 0.333 | 84.42 | 87.74 |
| 12.0 | 0.500 | 73.02 | 12.0 | 0.500 | 43.76 | 12.0 | 0.500 | 44.09 | 53.62 |
| 24.0 | 1.000 | 99.61 | 24.0 | 1.000 | 77.77 | 24.0 | 1.000 | 71.80 | 83.06 |
| 72.0 | 3.000 | 102.88 | 72.0 | 3.000 | 38.82 | 72.0 | 3.000 | 67.61 | 69.77 |
| 120.0 | 5.000 | 42.46 | 120.0 | 5.000 | 25.26 | 120.0 | 5.000 | 23.95 | 30.56 |
| 168.0 | 7.000 | 26.10 | 168.0 | 7.000 | 18.42 | 168.0 | 7.000 | 23.85 | 22.79 |
| 192.0 | 8.000 | 46.47 | 192.0 | 8.000 | 11.90 | 192.0 | 8.000 | 19.85 | 26.07 |
| 360.0 | 15.000 | 19.83 | 360.0 | 15.000 | 5.80 | 360.0 | 15.000 | 19.54 | 15.06 |
| 528.0 | 22.000 | 10.93 | 528.0 | 22.000 | 0.11 | 528.0 | 22.000 | 13.89 | 8.31 |
| 864.0 | 36.000 | 0.19 | 864.0 | 36.000 | 0.69 | 864.0 | 36.000 | 9.49 | 3.46 |
| 1968.0 | 82.000 | 0.48 | 1968.0 | 163.000 | 0.30 | | | | 0.39 |
| 3264.0 | 136.000 | 0.01 | 3264.0 | 212.000 | 0.03 | | | | 0.02 |
| 4272.0 | 178.000 | 0.01 | | | | 3960.0 | 165.000 | 0.01 | 0.01 |
| | | | 4824.0 | 201.000 | 0.01 | | | | 0.01 |

Table 8 2.5 mg/kg LDP-02 IV

| Subject # 18 | | | Subject # 19 | | | Mean μg/mL (n=2) |
|---|---|---|---|---|---|---|
| Time (hr) | Time (day) | μg/mL | Time (hr) | Time (day) | μg/mL | |
| Pre-Dose | Pre-Dose | 0.01 | Pre-Dose | Pre-Dose | 0.01 | 0.01 |
| 1.0 | 0.042 | 105.37 | 1.0 | 0.042 | 84.06 | 94.72 |
| 1.5 | 0.063 | 71.27 | 1.5 | 0.063 | 98.13 | 84.70 |
| 3.0 | 0.125 | 73.49 | 3.0 | 0.125 | 81.59 | 77.54 |
| 8.0 | 0.333 | 84.00 | 8.0 | 0.333 | 80.17 | 82.09 |
| 12.0 | 0.500 | 103.81 | 12.0 | 0.500 | 85.53 | 94.67 |
| 24.0 | 1.000 | 68.79 | 24.0 | 1.000 | 85.52 | 77.15 |
| 72.0 | 3.000 | 63.30 | 72.0 | 3.000 | 69.49 | 66.40 |
| 120.0 | 5.000 | 53.33 | 120.0 | 5.000 | 59.11 | 56.22 |
| 168.0 | 7.000 | 50.72 | 168.0 | 7.000 | 54.63 | 52.67 |
| 192.0 | 8.000 | 43.47 | 192.0 | 8.000 | 67.32 | 55.40 |
| 360.0 | 15.000 | 22.82 | 360.0 | 15.000 | 23.85 | 23.34 |
| 528.0 | 22.000 | 22.45 | 528.0 | 22.000 | 21.92 | 22.19 |
| 864.0 | 36.000 | 17.42 | 864.0 | 36.000 | 20.63 | 19.03 |
| 1680.0 | 70.000 | 5.48 | 1656.0 | 69.000 | 4.63 | 5.06 |
| 3312.0 | 138.000 | 0.01 | 2976.0 | 124.000 | 0.08 | 0.04 |
| 3984.0 | 166.000 | 0.01 | 3648.0 | 152.000 | 0.01 | 0.01 |
| | | | 4536.0 | 189.000 | 0.01 | 0.01 |

Table 9 placebo group

| Time (hr) | Time (day) | Subject # 1 | Subject # 7 | Subject # 11 | Subject # 14 | Subject # 17 |
|---|---|---|---|---|---|---|
| Pre-Dose | Pre-Dose | Its | Its | Its | Its | Its |
| 1.0 | 0.042 | Its | Its | Its | Its | Its |
| 1.5 | 0.063 | Its | Its | Its | Its | Its |
| 3.0 | 0.125 | Its | Its | Its | Its | Its |
| 8.0 | 0.333 | Its | Its | Its | Its | Its |
| 12.0 | 0.500 | Its | Its | Its | Its | Its |
| 24.0 | 1.000 | Its | Its | Its | Its | Its |
| 72.0 | 3.000 | Its | Its | Its | Its | Its |
| 120.0 | 5.000 | Its | Its | Its | Its | Its |
| 168.0 | 7.000 | Its | Its | Its | Its | Its |
| 192.0 | 8.000 | Its | Its | Its | Its | Its |
| 360.0 | 15.000 | Its | Its | Its | Its | Its |
| 528.0 | 22.000 | Its | Its | Its | Its | Its |
| 864.0 | 36.000 | Its | Its | Its | Its | Its |
| Its = below the limit of detection | | | | | | |

Study L297-007: Mean Pharmacokinetic Parameters by Treatment Group Data from individual patients are presented in Tables 10-14.

Table 10 0.15 mg/kg LDP-02 IV

| Subject | $C_{max}$ ($\mu$g/ml) | $t_{max}$ (days) | $AUC_t$ ($\mu$g·day/ml) | $\lambda_z$ (1/day) | $t_{\frac{1}{2}z}$ (days) | AUC ($\mu$g·day/ml) | $AUC_{ext}$ (%) | $V_z$ (ml/kg) | CL (ml/day/kg) |
|---|---|---|---|---|---|---|---|---|---|
| 2 | 10.667 | 0.33 | 16.4 | 0.2486 | 2.79 | 16.5 | 0.3 | 36.7 | 9.11 |
| 3 | 7.984 | 0.04 | 25.3 | 0.1196 | 5.79 | 27.1 | 6.7 | 46.3 | 5.53 |
| 4 | 4.292 | 0.13 | 16.9 | 0.1510 | 4.59 | 17.5 | 3.3 | 56.9 | 8.60 |
| Mean | 7.648 | 0.13* | 19.5 | 0.1731 | 4.39 | 20.3 | 3.4 | 46.6 | 7.75 |
| SD | 3.201 | | 5.00 | 0.0673 | 1.51 | 5.88 | 3.2 | 10.1 | 1.93 |

*Median value
$C_{max}$ = maximum concentration
$t_{max}$ = time to maximum concentration
$\lambda_z$ = a measure of elimination
$t_{1/2z}$ = terminal half-live
$AUC_t$ = $AUC_{all}$ = area under the curve using all time points
AUC = $AUC_{ext}$ = area under curve extrapolated
AUC ext (%) = % of area under curve attributed to extrapolation extrapolation
$V_z$ = apparent volume of distribution
CL = Clearance

Table 11 0.15 mg/kg LDP-02 SC

| Subject | $C_{max}$ ($\mu$g/ml) | $t_{max}$ (days) | $AUC_t$ ($\mu$g·day/ml) | $\lambda_z$ (1/day) | $t_{1/2z}$ (days) | AUC ($\mu$g·day/ml) | $AUC_{ext}$ (%) | $V_z$ (ml/kg) | CL (ml/day/kg) |
|---|---|---|---|---|---|---|---|---|---|
| 5 | 0.711 | 6.01 | 7.18 | 0.2298 | 3.02 | 7.32 | 2.0 | 89.1 | 20.5 |
| 6 | 0.927 | 4.01 | 8.71 | 0.2253 | 3.08 | 8.83 | 1.4 | 75.4 | 17.0 |
| 8 | 1.699 | 6.01 | 15.4 | 0.1003 | 6.91 | 18.0 | 14.3 | 82.9 | 8.32 |
| Mean | 1.112 | 6.01* | 10.4 | 0.1852 | 4.33 | 11.4 | 5.9 | 82.5 | 15.3 |
| SD | 0.519 | | 4.40 | 0.0735 | 2.23 | 5.80 | 7.3 | 6.88 | 6.26 |
| *Median value | | | | | | | | | |

Table 12 0.5 mg/kg LDP-02 IV

| Subject | $C_{max}$ (µg/ml) | $t_{max}$ (days) | $AUC_t$ (µg·day/ml) | $\lambda_z$ (1/day) | $t_{\frac{1}{2}z}$ (days) | AUC (µg·day/ml) | $AUC_{ext}$ (%) | $V_z$ (ml/kg) | CL (ml/day/kg) |
|---|---|---|---|---|---|---|---|---|---|
| 9 | 24.388 | 0.06 | 82.2 | 0.1586 | 4.37 | 85.1 | 3.4 | 37.0 | 5.87 |
| 10 | 11.699 | 0.50 | 66.1 | 0.2159 | 3.21 | 67.0 | 1.3 | 34.6 | 7.47 |
| 12 | 11.194 | 0.50 | 102.5 | 0.1543 | 4.49 | 103 | 0.8 | 31.4 | 4.84 |
| Mean | 15.760 | 0.50* | 83.6 | 0.1763 | 4.02 | 85.1 | 1.8 | 34.3 | 6.06 |
| SD | 7.476 | | 18.3 | 0.0344 | 0.71 | 18.2 | 1.4 | 2.84 | 1.32 |
| *Median value | | | | | | | | | |

Table 13 1.5 mg/kg LDP-02 IV

| Subject | $C_{max}$ ($\mu$g/ml) | $t_{max}$ (days) | $AUC_t$ ($\mu$g·day/ml) | $\lambda_z$ (1/day) | $t_{\frac{1}{2}z}$ (days) | AUC ($\mu$g·day/ml) | $AUC_{ext}$ (%) | $V_z$ (ml/kg) | CL (ml/day/kg) |
|---|---|---|---|---|---|---|---|---|---|
| 13 | 114.686 | 0.33 | 854 | 0.2316 | 2.99 | 855 | 0.1 | 7.58 | 1.75 |
| 15 | 134.975 | 0.06 | 408 | 0.0336 | 20.6 | 409 | 0.2 | 109 | 3.67 |
| 16 | 106.779 | 0.13 | 719 | 0.0331 | 20.9 | 1000 | 28.1 | 45.3 | 1.50 |
| Mean | 118.813 | 0.13* | 660 | 0.0994 | 14.9 | 755 | 9.5 | 54.0 | 2.31 |
| SD | 14.544 | | 229 | 0.1145 | 10.3 | 308 | 16.1 | 51.4 | 1.19 |
| *Median value | | | | | | | | | |

Table 14 2.5 mg/kg LDP-02 IV

| Subject | Cmax (μg/ml) | tmax (days) | AUCt (μg·day/ml) | $\lambda_z$ (1/day) | $t_{1/2z}$ (days) | AUC (μg·day/ml) | AUCext (%) | Vz (ml/kg) | CL (ml/day/kg) |
|---|---|---|---|---|---|---|---|---|---|
| 18 | 105.367 | 0.04 | 1489 | 0.0296 | 23.4 | 1680 | 11.3 | 50.2 | 1.49 |
| 19 | 98.131 | 0.06 | 1814 | 0.0642 | 10.8 | 1815 | 0.1 | 21.5 | 1.38 |
| Mean | 101.749 | 0.05* | 1651 | 0.0469 | 17.1 | 1747 | 5.7 | 35.9 | 1.43 |
| SD | 5.117 | | 229 | 0.0244 | 8.91 | 95.8 | 8.0 | 20.3 | 0.08 |

*Median value

L297-007: Serum $\alpha4\beta7$ Binding Over Time by Subject by Treatment Group. Data from individual patients are presented in Tables 15-20. For each subject the time of blood sampling, MESF of the sample and % of baseline (pre-dose) MESF is presented.

Table 15 0.15 mg/kg LDP-02 IV

| Subject # 2 | | | Subject # 3 | | | Subject # 4 | | | Mean | |
|---|---|---|---|---|---|---|---|---|---|---|
| Pre-Dose | 5689 | 100% | Pre-Dose | 5424 | 100% | Pre-Dose | 4177 | 100% | 5097 | 100% |
| 3 hr | 605 | 11% | 3 hr | 591 | 11% | 3 hr | 588 | 14% | 595 | 12% |
| 24 hrs | 589 | 10% | 24 hrs | 600 | 11% | 24 hrs | 631 | 15% | 607 | 12% |
| Day 3 | 501 | 9% | Day 3 | 496 | 9% | Day 3 | 548 | 13% | 515 | 10% |
| Day 7 | 474 | 8% | Day 7 | 473 | 9% | Day 7 | 512 | 12% | 487 | 10% |
| Day 15 | 1819 | 32% | Day 15 | 578 | 11% | Day 15 | 599 | 14% | 999 | 20% |
| Day 22 | 2426 | 43% | Day 22 | 558 | 10% | Day 22 | 609 | 15% | 1198 | 23% |
| Day 36 | 3028 | 53% | Day 36 | 3570 | 66% | Day 36 | 3469 | 83% | 3356 | 66% |
| | | | Day 163 | 6934 | 128% | Day 163 | 6837 | 164% | 6885 | 135% |
| | | | Day 205 | 4675 | 86% | Day 205 | 6755 | 162% | 5715 | 112% |

Table 16 0.15 mg/kg LDP-02 SC

| Subject # 5 | | | Subject # 6 | | | Subject # 8 | | | Mean | |
|---|---|---|---|---|---|---|---|---|---|---|
| Pre-Dose | 6043 | 100% | Pre-Dose | 6779 | 100% | Pre-Dose | 5857 | 100% | 6226 | 100% |
| 3 hr | 1797 | 30% | 3 hr | 4727 | 70% | 3 hr | 1514 | 26% | 2679 | 43% |
| 24 hrs | 637 | 11% | 24 hrs | 588 | 9% | 24 hrs | 616 | 11% | 614 | 10% |
| Day 3 | 529 | 9% | Day 3 | 520 | 8% | Day 3 | 527 | 9% | 525 | 8% |
| Day 7 | 486 | 8% | Day 7 | 474 | 7% | Day 7 | 485 | 8% | 482 | 8% |
| Day 15 | 598 | 10% | Day 15 | 642 | 9% | Day 15 | 635 | 11% | 625 | 10% |
| Day 22 | 759 | 13% | Day 22 | 934 | 14% | Day 22 | 579 | 10% | 757 | 12% |
| Day 36 | 1455 | 24% | Day 36 | 1452 | 21% | Day 36 | 2799 | 48% | 1902 | 131% |
| Day 163 | 2743 | 45% | Day 163 | 1989 | 29% | Day 163 | 4621 | 79% | 3118 | 50% |
| Dav 212 | 4201 | 70% | Day 212 | 2601 | 38% | Day 212 | 4832 | 82% | 3878 | 62% |

Table 17 0.5 mg/kg LDP-02 IV

| Subject # 9 | | | Sub ect # 10 | | | Subject # 12 | | | Mean | |
|---|---|---|---|---|---|---|---|---|---|---|
| Pre-Dose | 5519 | 100% | Pre-Dose | 5966 | 100% | Pre-Dose | 8550 | 100% | 6678 | 100% |
| 3 hr | 533 | 10% | 3 hr | 548 | 9% | 3 hr | 539 | 6% | 540 | 8% |
| 24 hrs | 542 | 10% | 24 hrs | 554 | 9% | 24 hrs | 527 | 6% | 541 | 8% |
| Day 3 | 565 | 10% | Day 3 | 574 | 10% | Day 3 | 539 | 6% | 560 | 8% |
| Day 7 | 544 | 10% | Day 7 | 551 | 9% | Day 7 | 547 | 6% | 547 | 8% |
| Day 15 | 540 | 10% | Day 15 | 525 | 9% | Day 15 | 520 | 6% | 528 | 8% |
| Day 22 | 555 | 10% | Day 22 | 572 | 10% | Day 22 | 543 | 6% | 557 | 8% |
| Day 36 | 885 | 16% | Day 36 | 1182 | 20% | Day 36 | 643 | 8% | 903 | 14% |

(continued)

| Subject # 9 | | | Sub ect # 10 | | | Subject # 12 | | | Mean | |
|---|---|---|---|---|---|---|---|---|---|---|
| Day 149 | 4448 | 81% | Day 163 | 5256 | 88% | Day 149 | 7810 | 91% | 5838 | 87% |

Table 18 1.5 mg/kg LDP-02 IV

| Subject # 13 | | | Subject # 15 | | | Subject # 16 | | | Mean | |
|---|---|---|---|---|---|---|---|---|---|---|
| Pre-Dose | 4966 | 100% | Pre-Dose | 5544 | 100% | Pre-Dose | 5622 | 100% | 5378 | 100% |
| 3 hr | 518 | 10% | 3 hr | 539 | 10% | 3 hr | 545 | 10% | 534 | 10% |
| 24 hrs | 482 | 10% | 24 hrs | 487 | 9% | 24 hrs | 520 | 9% | 496 | 9% |
| Day 3 | 511 | 10% | Day 3 | 475 | 9% | Day 3 | 514 | 9% | 500 | 9% |
| Day 7 | 549 | 11% | Day 7 | 535 | 10% | Day 7 | 569 | 10% | 551 | 10% |
| Day 15 | 472 | 9% | Day 15 | 474 | 9% | Day 15 | 491 | 9% | 479 | 9% |
| Day 22 | 603 | 12% | Day 22 | 617 | 11% | Day 22 | 576 | 10% | 599 | 11% |
| Day 36 | 618 | 12% | Day 36 | 866 | 16% | Day 36 | 606 | 11% | 697 | 13% |
| Day 82 | 922 | 19% | Day 80 | 832 | 15% | | | | 877 | 16% |
| Day 134 | 1647 | 33% | Day 134 | 1531 | 28% | | | | 1589 | 30% |
| Dav 176 | 2322 | 47% | | | | | | | 2322 | 43% |

Table 19 2.5 mg/kg LDP-02 IV

| Subject # 18 | | | Subject # 19 | | | Mean | |
|---|---|---|---|---|---|---|---|
| Pre-Dose | 5922 | 100% | Pre-Dose | 5065 | 100% | 5494 | 100% |
| 3 hr | 527 | 9% | 3 hr | 527 | 10% | 527 | 10% |
| 24 hrs | 568 | 10% | 24 hrs | 571 | 11% | 569 | 10% |
| Day 3 | 511 | 9% | Day 3 | 521 | 10% | 516 | 9% |
| Day 7 | 503 | 9% | Day 7 | 513 | 10% | 508 | 9% |
| Day 15 | 530 | 9% | Day 15 | 544 | 11% | 537 | 10% |
| Dav 22 | 588 | 10% | Day 22 | 595 | 12% | 591 | 11% |
| Day 36 | 550 | 9% | Day 36 | 554 | 11% | 552 | 10% |
| Day 70 | 615 | 10% | Day 69 | 566 | 11% | 590 | 11% |
| Day 138 | 4572 | 77% | Day 124 | 1103 | 22% | 2837 | 52% |
| Day 166 | 5603 | 95% | Day 152 | 4094 | 81% | 4849 | 88% |

Table 20 placebo group

| | Subject # 1 | | Subject # 7 | | Subject # 11 | | Subject # 14 | | Subject # 17 | |
|---|---|---|---|---|---|---|---|---|---|---|
| Pre-Dose | 5807 | 100% | 5198 | 100% | 8747 | 100% | 7017 | 100% | 5982 | 100% |
| 3 hr | 5630 | 97% | 4305 | 83% | 8454 | 97% | 6208 | 88% | 5520 | 92% |
| 24 hrs | 6672 | 115% | 4347 | 84% | 8033 | 92% | 6699 | 95% | 5410 | 90% |
| Day 3 | 6078 | 105% | 4008 | 77% | 8701 | 99% | 6141 | 88% | 5488 | 92% |
| Day 7 | 5617 | 97% | 4047 | 78% | 8668 | 99% | 6327 | 90% | 5194 | 87% |

(continued)

|  | Subject # 1 | | Subject # 7 | | Subject # 11 | | Subject # 14 | | Subject # 17 | |
|---|---|---|---|---|---|---|---|---|---|---|
| Day 15 | 5797 | 100% | 4758 | 92% | 7516 | 86% | 4851 | 69% | 5759 | 96% |
| Day 22 | 5164 | 89% | 4318 | 83% | 6924 | 79% | 5246 | 75% | 5922 | 99% |
| Day 36 | 6200 | 107% | 4686 | 90% | 7065 | 81% | 7857 | 112% | 5349 | 89% |

Example 2. Study L297-006

The study entitled, "A Single Dose Phase Ib/IIa, Placebo Controlled, Randomized, Double-Blind Study to Determine the Safety, Tolerability, Pharmacokinetics, Pharmacodynamics, and Effectiveness of LDP-02 in Patients with Moderately Severe Ulcerative Colitis" was completed and certain final results are presented in this section.

Study Rationale

Results from the Phase I trial (Example 1. Study L297-007) in healthy volunteers showed LDP-02 at doses of 0.15 mg/kg SC and IV, 0.5 mg/kg IV, 1.5 mg/kg IV, and 2.5 mg/kg IV was safe and well-tolerated. In addition, doses of 0.15 mg/kg IV or SC and 0.5 mg/kg IV were shown to have a $t_{1/2}$ of approximately 100 to 130 hours and flow cytometry data showed that unbound $\alpha4\beta7$ begins to reappear in the 0.15 mg/kg dosage groups approximately two weeks after dosing. Based upon these data, LDP-02 dosages of 0.15 mg/kg SC, 0.15 mg IV, 0.5 mg/kg IV, and 2.0 mg/kg IV were selected for use in the initial study in patients with ulcerative colitis. This study was designed so that each dose of LDP-02 was determined to be safe and well-tolerated prior to escalation to the next dose level.

Study Design

The study was a randomized, double-blind, placebo-controlled, ascending single-dose study in patients diagnosed with moderately-severe ulcerative colitis. Patients with a documented diagnosis of ulcerative colitis with a minimum disease extent of 25 cm from the anal verge were potentially eligible for the study. Patients with severe ulcerative colitis as defined by Truelove-Witts criteria (Br Med J; 2:1042-1048 (1955)) were excluded. Ulcerative colitis patients who met all inclusion/exclusion criteria were enrolled sequentially into four study groups and, within each study group, were randomly assigned to receive LDP-02 or placebo (i.e., 0.9% sodium chloride). Treatment groups and numbers of patients enrolled are shown in Table 21.

Table 21: Study Groups

| Group | Route of Administration* | LDP-02 | | Placebo |
|---|---|---|---|---|
| | | # patients | Dose | # patients |
| 1 | SC | 5 | 0.15 mg/kg | 2 |
| 2 | IV | 5 | 0.15 mg/kg | 2 |
| 3 | IV | 5 | 0.5 mg/kg | 2 |
| 4 | IV | 5 | 2.0 mg/kg | 2 |

Study medication (LDP-02 or placebo) was administered on Day 1 either SC into the thigh or via a 30 minute IV infusion. Safety assessments included recording of adverse events, physical examinations, vital signs, clinical laboratories (i.e., hematology, blood chemistries, and urinalysis), plasma cytokine levels, and ECGs. Blood was drawn at various time points to measure LDP-02 serum concentrations and to assess the effectiveness of LDP-02 to saturate and block $\alpha4\beta7$ binding receptors on peripheral blood lymphocytes. The effectiveness of LDP-02 to reduce inflammation in the colon was measured by clinical disease observations, endoscopic appearance, histopathology, and immunohistochemistry.

Study Results

Patient Disposition and Demography

Twenty-nine patients with moderately-severe ulcerative colitis were entered into the study and 28 completed the trial. One patient was found to be *Clostridium difficile* toxin positive at screening, but due to a delay in reporting laboratory results the patient was admitted into the trial and received 2.0 mg/kg IV of LDP-02. Once the laboratory results were obtained, the patient was treated with antibiotics and replaced by another patient. There were no other patients discontinued from the study. As patients were recruited into the study over time, there was no attempt to balance the treatment groups with regard to baseline ulcerative colitis history. As such, severity and duration of ulcerative colitis disease and prior medications for ulcerative colitis varied from patient to patient and from treatment group to treatment group. These data are presented in Table 22.

Table 22: Ulcerative Colitis History by Treatment Group

| Treatment Group | Time Since Onset of UC Symptoms (yrs)[1] | Time Since Diagnosis of UC (yrs)[1] | # of Acute Exacerbations in past 12 months[1] | Weeks on continuous oral 5-ASA in past 6 months[1] | Weeks on continuous oral steroids in past 6 months[1] |
|---|---|---|---|---|---|
| 0.15 mg/kg SC (n=5) | 5.32 (4.8,6.4) | 4.6 (4.3,6.4) | 3 (1,12) | 24.0 (3,26) | 0 (0,6) |
| 0.15 mg/kg IV (n=5) | 9.58 (2.6,14.2) | 4.9 (2.1,14.0) | 1 (1,3) | 24.0 (6,26) | 10 (0,24) |
| 0.5 mg/kg IV (n=5) | 10.8 (0.4,11.8) | 9.0 (0.3,11.8) | 1 (1,2) | 26.0 (0,26) | 0 (0,15) |
| 2.0 mg/kg IV (n=6) | 9.34 (3.4,58.8) | 7.65 (3.2,19.4) | 2 (1,5) | 25.0 (0,26) | 5 (0,26) |
| All LDP-02 (n=21) | 5.99 (0.4,58.8) | 4.9 (0.3,19.4) | 2 (1,12) | 26.0 (0,26) | 0 (0,26) |
| Placebo (n=8) | 5.27 (0.4,11.0) | 4.85 (0.3,9.7) | 1.5 (1,4) | 24.0 (0,26) | 16 (0,26) |
| [1]Median values | | | | | |

Disease Measurements

Although,this was primarily a dose-ranging safety and pharmacokinetics study, various parameters were measured to assess effectiveness of treatment. Effectiveness assessments included recording changes from baseline using a modified Baron's (endoscopy) Scoring System, the Mayo Clinic Disease Activity Index Score, the Powell-Tuck Disease Activity Index Score, stool frequency, and the Inflammatory Bowel Disease Questionnaire. Changes from baseline to Day 30 for these parameters are shown in Table 23. For patients in which there was no Day 30 evaluation, the last post-baseline observation obtained was carried forward to Day 30.

Table 23: Change from Baseline to Day 30 in Disease Parameters

| Treatment Group | Change from baseline to Day 30[1] | | | | |
|---|---|---|---|---|---|
| | Endoscopic Severity Score | Mayo Clinic Activity Index | Powell-Tuck Activity Index | Stool Frequency | Total IBDQ |
| 0.15 mg/kg SC (n=5) | 0 (-2,0) | -3.0 (-9,0) | -3.0 (-6,-2) | -1.0 (-7,1) | 14.0 (14,72) |
| 0.15 mg/kg IV (n=5) | 0 (0,1) | -1.0 (-3,2) | 0 (-3,3) | -0.4 (-5,2) | 8.0 (-3,95) |
| 0.5 mg/kg IV (n=5) | -2.0 (-3,0) | -10 (-11,0) | -6.0 (-13,-2) | -5.3 (-6,0) | 37.0 (14,80) |

(continued)

| Treatment Group | Change from baseline to Day 30[1] | | | | |
|---|---|---|---|---|---|
| | Endoscopic Severity Score | Mayo Clinic Activity Index | Powell-Tuck Activity Index | Stool Frequency | Total IBDQ |
| 2.0 mg/kg IV (n=6) | -0.5 (-2,1) | -2.0 (-6,3) | -1.5 (-5,-5) | -3.2 (-8,2) | -2.5 (-59,95) |
| All LDP-02 (n=21) | 0 (-3,1) | -3.0 (-11,3) | -3.0 (-13,5) | -2.4 (-8,2) | 14.0 (-59,95) |
| Placebo (n=8) | -1.0 (-3,2) | -5.0 (-8,4) | -6.0 (-9,-4) | -3.2 (-12,2) | 53.5 (-30,82) |
| [1]Median values and range. For patients without a Day 30 evaluation the last post-baseline evaluation was carried forward to Day 30. | | | | | |

As seen from the results presented in Table 23, there was variability in response among the different treatment groups. The patients receiving 0.5 mg/kg IV appeared to have the best responses; the median endoscopic severity score was reduced by two grades and the Mayo Clinic score was reduced by 10 points with a decrease in stool frequency. Three of the five patients receiving 0.5 mg/kg IV had a two point improvement in the modified Baron sigmoidoscopy score which is considered an endoscopic response; only one patient (compared with a total of five treated per group) in both the 2.0 mg/kg IV and 0.15 mg/kg SC groups had an endoscopic response. The placebo group also experienced an improvement in sigmoidoscopic score and Mayo Clinic score, although both were less in magnitude when compared to the 0.5 mg/kg IV group. Two of the eight patients experienced an endoscopic response.

The number of patients with a complete remission, defined as a zero on the modified Baron sigmoidoscopic score and on the Mayo Clinic score at Day 30, are reported in Table 24.

Table 24: Patients in Complete Remission at Day 30

| Treatment Group | Measured at Day 30[1] | |
|---|---|---|
| | Number of Patients with Complete Remission | Percentage of Patients in Complete Remission |
| 0.15 mg/kg SC (n=5) | 0 | 0 |
| 0.15 mg/kg IV (n=5) | 0 | 0 |
| 0.5 mg/kg IV (n=5) | 2 | 40% |
| 2.0 mg/kg IV (n=6) | 0 | 0 |
| All LDP-02 (n=21) | 2 | 9.5% |
| Placebo (n=8) | 0 | 0 |
| [1]Zero on the modified Baron Score and the Mayo Clinic Score in Day 30 results | | |

None of the patients in the placebo group experienced a complete remission while two patients among those receiving LDP-02 had complete remissions. The two patients both were in the same group; both patients received a single administration of 0.5 mg/kg of LDP-02. One of the patients was receiving concurrent mesalamine therapy, while the other was receiving concurrent low dose corticosteroid (20 mg prednisone per day orally).

Pharmacokinetics

The assay of LDP-02 in serum was performed by Cytometry Associates, Inc. as previously described (Study L297-007). Blood samples were collected prior to and immediately following the completion of infusion (Day 1) and on Days 2, 3, 5, 10, 14, 21, 30 and 60 to assess the pharmacokinetic profile of LDP-02.

LDP-02 concentrations over time by individual patient and mean pharmacokinetic parameters by LDP-02 dose are presented in the Appendix to study L296-006.

As seen in FIG. 8, serum levels of LDP-02 for the 0.15 mg/kg IV and SC groups fall to <1.0 $\mu$g/mL approximately 20 days post-dose. For the 2.0 mg/kg dose group, LDP-02 levels remain elevated out to approximately Day 60. Table 25 presents the key pharmacokinetic parameters by treatment group.

Table 25: Pharmacokinetic Parameters of LDP-02

| Pharmacokinetic Parameter[1] | Dose and Route of Administration of LDP-02 (number of subjects with data)[2] | | | |
|---|---|---|---|---|
| | 0.15 mg/kg SC (n=5) | 0.15 mg/kg IV (n=5) | 0.5 mg/kg IV (n=5) | 2.0 mg/kg IV (n=4)[3] |
| $C_{max}$ ($\mu$g/mL) | 1.44 (0.33) | 3.602 (0.958) | 10.544 (2.582) | 32.933 (3.360) |
| $t_{max}$ (days) (median & range) | 5 (3-10) | 0.13 (0.13-0.13) | 0.13 (0.13-0.13) | 0.13 (0.13-2) |
| $t_{\frac{1}{2}z}$ (days) | 15.63 (15.92) | 18.91 (20.97) | 10.62 (5.23) | 15.0 (5.36) |
| $AUC_{all}$ ($\mu$g·day/mL) | 25 (16) | 27 (11) | 91 (32) | 515 (93) |
| $\lambda_z$ (1/day) | 0.1226 (0.1064) | 0.0879 (0.0757) | 0.0927 (0.0775) | 0.0542 (0.0298) |
| AUC(INF) ($\mu$g·day/mL) | 31 (23) | 34 (18) | 100 (39) | 553 (116) |
| $CL^4$ (mLday/kg) | 9.21 (9.54) | 7.75 (1.93) | 6.06 (1.32) | 2.31 (1.19) |
| $V_z{}^4$ (mL/kg) | 95.08 (54.19) | 101.05 (62.87) | 77.63 (30.90) | 76.64 (20.03) |

[1]All values are mean +/- SD unless otherwise indicated. The SD appears in parenthesis.
[2]Two patients, one in the 0.15 mg/kg SC and one in the 0.5 mg/kg IV groups had evaluable data through Study Day 21 with measurement at later times which were not physiologically possible.
[3]One patient in the 2.0 mg/kg IV dosing group was withdrawn at Study Day 10 and had a surgical intervention. The pharmacokinetic results for this patient are not included.
[4]Clearance and volume terms for the SC dose group are the apparent clearance (CL/F) and apparent volume ($V_z$/F).

There does appear to be linearity with dose for the maximum concentration of LDP-02 and the area under the curve measured after IV administration. The clearance and the terminal elimination half life appear to be independent of IV dose administered. The volume of distribution appears to decrease slightly with increasing doses of IV LDP-02.

Assessment of the Pharmacodynamic Effect of LDP-02

FACS analysis to measure the presence of $\alpha 4\beta 7$ sites on blood lymphocytes was previously described (Study L296-007). Serum $\alpha 4\beta 7$ binding over time (i.e., MESF values and percentage of baseline at each post-dose time point) are presented by individual patient and by treatment group in the Appendix to Study L297-006.
Mean percent of baseline MESF over time for all treatments are presented in FIG. 9. As seen in FIG. 9, percent of baseline MESF rapidly falls to approximately 10% after SC and IV administration of LDP-02 with duration of effect dependent upon dose. Starting at about day 10, $\alpha 4\beta 7$ signal started to return to baseline for the 0.15 mg/kg IV and SC dose groups. However, $\alpha 4\beta 7$ signal started to return to baseline between day 30 and day 60 for the 0.5 mg/kg IV and 2.0 mg/kg dose groups.

Conclusions

Administration of LDP-02 at doses of 0.15 mg/kg IV and SC, 0.5 mg/kg IV, and 2.0 mg/kg IV to patients with moderately-severe ulcerative colitis was well-tolerated.
The pharmacokinetic and pharmacodynamic data from patients with ulcerative colitis were consistent with those found in healthy volunteers. There appeared to be linearity with dose for the maximum concentration of LDP-02 and area under the curve measured after IV administration. The clearance and the terminal elimination half life appeared to be independent of IV dose administration. The volume of distribution appeared to decrease slightly with increasing doses of IV LDP-02. The percent of baseline MESF declined to ~10% rapidly after SC and IV administration of LDP-02 with duration of effect dependent upon dose. For the 0.15 mg/kg IV and SC dose groups, percent of baseline MESF started returning to baseline approximately 10 days after dosing whereas this started to occur at ~30 days and -60 days for the 0.5 mg/kg IV and 2.0 mg/kg dose groups, respectively.

Appendix to Study L297-006

LDP-02 Serum Concentration Over Time by Subject by Treatment Group. Data obtained from individual subjects are presented in Tables 26-30. The data presented in Tables 26-30 are in μg/mL.

Table 26 Group 1: 0.15 mg/kg LDP-02 SC

| Time (day) | Subject # 201101 | Subject # 301103 | Subject # 302105 | Subject # 304107 | Subject # 401104 |
|---|---|---|---|---|---|
| Pre-Dose | BQL | BQL | BQL | BQL | BQL |
| 0.125 | BQL | 0.07 | BQL | BQL | NS |
| 2 | 0.61 | 0.91 | 0.94 | 1.01 | 1.29 |
| 3 | 0.90 | 1.10 | 1.29 | 1.49 | 1.65 |
| 5 | 0.76 | 1.48 | NR | 1.66 | 1.74 |
| 10 | 0.15 | 1.12 | 1.40 | 0.92 | 1.44 |
| 14 | BQL | 0.61 | 0.78 | 0.24 | 0.99 |
| 21 | BQL | BQL | NS | 0.11 | 0.65 |
| 30 | BQL | 0.33 | 0.84 | 0.26 | 0.12 |
| 60 | BQL | 0.23 | 0.37 | 0.30 | BQL |
| BQL= reported as non-detectable NS= no sample received from laboratory | | | | | |

Table 27 Group 2: 0.15 mg/kg LDP-02 IV

| Time (Day) | Subject # 101201 | Subject # 102202 | Subject # 305204 | Subject # 402203 | Subject # 403206 |
|---|---|---|---|---|---|
| Pre-Dose | BQL | BQL | BQL | BQL | BQL |
| 0.125 | 4.14 | 4.88 | 3.35 | 2.34 | 3.30 |
| 2 | NR | 2.74 | 1.92 | 1.83 | 2.34 |
| 3 | 3.12 | 3.15 | 1.55 | 1.42 | 2.03 |
| 5 | 1.82 | 1.83 | 1.33 | 0.82 | 1.19 |
| 10 | 0.81 | 0.88 | 0.86 | 0.37 | 0.79 |
| 14 | 0.32 | 0.15 | BQL | 0.23 | 0.26 |
| 21 | 0.38 | 0.12 | 0.10 | BQL | BQL |
| 30 | 0.38 | BQL | 0.40 | BQL | 0.05 |
| 60 | 0.24 | BQL | 0.36 | BQL | 0.14 |
| NR= no sample result reported from laboratory | | | | | |

Table 28 Group 3: 0.5 mg/kg LDP-02 IV

| Time (day) | Subject # 206302 | Subject # 208303 | Subject # 309306 | Subject # 502304 | Subject # 503307 |
|---|---|---|---|---|---|
| Pre-Dose | BQL | BQL | BQL | BQL | BQL |
| 0.125 | 14.06 | 12.33 | 7.90 | 8.67 | 9.76 |
| 2 | 10.01 | 8.51 | 5.73 | 5.84 | 8.26 |
| 3 | 6.56 | 6.45 | 4.96 | 4.67 | 7.27 |
| 5 | 4.15 | 5.52 | 3.59 | 2.94 | 5.61 |
| 10 | 3.17 | 4.46 | 2.81 | 3.11 | 4.21 |
| 14 | 2.51 | 0.14 | 2.46 | 1.14 | 3.01 |
| 21 | BQL | 0.17 | 0.14 | BQL | 2.04 |
| 30 | BQL | 0.48 | BQL | 0.06 | 1.29 |
| 60 | 0.41 | 1.73 | 0.10 | 0.28 | BQL |

Table 29 Group 4: 2.0 mg/kg LDP-02 IV

| Time (day) | Subject # 104403 | Subject # 210402 | Subject # 310415 | Subject# 404401 | Subject # 504405 | Subject # 506407 |
|---|---|---|---|---|---|---|
| Pre-Dose | BQL | BQL | BQL | BQL | BQL | BQL |
| 0.125 | 30.45 | 38.83 | 37.66 | 29.71 | 28.90 | 32.18 |
| 2 | 32.18 | 28.22 | 35.14 | 27.49 | 27.49 | 26.87 |
| 3 | 23.93 | 17.40 | 27.49 | 24.45 | 22.92 | 22.46 |
| 5 | 21.52 | 15.34 | 21.52 | 18.42 | 21.52 | 17.79 |
| 10 | 13:10 | 41.11 | 14.82 | 13.10 | 10.99 | 11.96 |
| 14 | 11.72 | 3.13 | 13.10 | 11.23 | 1.22 | 9.03 |
| 21 | 7.53 | 0.08 | 10.99 | 8.55 | 0.12 | 5.70 |
| 30 | 5.80 | BQL | 8.26 | 7.02 | NR | 4.19 |
| 60 | 1.71 | 0.41 | 2.24 | 1.95 | NR | 0.06 |

Table 30 placebo group

| Time (day) | Subject # 202102 | Subject # 303106 | Subject # 103205 | Subject # 306207 | Subject # 308305 | Subject # 501301 | Subject # 209404 | Subject # 505406 |
|---|---|---|---|---|---|---|---|---|
| Pre-Dose | BQL | BQL | BQL | BQL | BQL | BQL | BQL | BQL |
| 0.125 | BQL | BQL | BQL | BQL | BQL | BQL | BQL | BQL |
| 2 | BQL | BQL | BQL | BQL | BQL | BQL | BQL | BQL |
| 3 | BQL | BQL | BQL | BQL | BQL | BQL | BQL | BQL |
| 5 | BQL | BQL | BQL | BQL | BQL | BQL | BQL | BQL |
| 10 | BQL | BQL | BQL | BQL | BQL | BQL | BQL | BQL |
| 14 | BQL | BQL | BQL | BQL | BQL | BQL | BQL | BQL |
| 21 | BQL | BQL | NR | BQL | BQL | BQL | BQL | BQL |
| 30 | BQL | BQL | BQL | BQL | BQL | BQL | BQL | BQL |
| 60 | BQL | BQL | BQL | BQL | BQL | BQL | BQL | BQL |

BQL = below quantitation limit.

Mean Pharmacokinetic Parameters by Treatment Group. Data obtained from individual subjects are presented in Tables 31-34.

Table 31 Group 1: 0.15 mg/kg LDP-02 SC

| Subject | $C_{max}$ ($\mu$g/mL) | $t_{max}$ (days) | $t_{\frac{1}{2}z}$ (days) | $AUC_{all}$ ($\mu$g·day/mL) | $\lambda_z$ (1/day) | AUC ($\mu$g·day/mL) | CL (mL/day/kg) | $V_z$ (mL/kg) |
|---|---|---|---|---|---|---|---|---|
| 201101 | 0.90 | 3 | 2.58 | 5.30 | 0.2692 | 5.86 | 25.61 | 95.15 |
| 301103 | 1.48 | 5 | 34.61 | 30.39 | 0.0200 | 41.87 | 3.58 | 178.87 |
| 302105 | 1.40 | 10 | 31.35 | 46.94 | 0.0221 | 63.68 | 2.36 | 106.55 |
| 304107 | 1.66 | 5 | 3.88 | 15.41 | 0.1788 | 16.02 | 9.36 | 52.37 |
| 401104 | 1.74 | 5 | 5.72 | 28.17 | 0.1212 | 29.16 | 5.14 | 42.45 |
| Mean | 1.436 | 5.6 | 15.628 | 25.242 | 0.1223 | 31.318 | 9.21 | 95.078 |

(continued)

| Subject | $C_{max}$ ($\mu$g/mL) | $t_{max}$ (days) | $t_{\frac{1}{2}z}$ (days) | $AUC_{all}$ ($\mu$g·day/mL) | $\lambda_z$ (1/day) | AUC ($\mu$g·day/mL) | CL (mL/day/kg) | $V_z$ (mL/kg) |
|---|---|---|---|---|---|---|---|---|
| SD | 0.329 | 2.607 | 15.921 | 15.813 | 10.1064 | 22.613 | 9.54 | 54.190 |

| |
|---|
| $C_{max}$ = maximum concentration<br>$t_{max}$ = time to maximum concentration<br>$\lambda_z$ = a measure of elimination<br>$t_{1/2z}$ = terminal half-live<br>$AUC_t$ = $AUC_{all}$ = area under the curve using all time points<br>AUC = $AUC_{ext}$ = area under curve extrapolated<br>AUC ext (%) = % of area under curve attributed to extrapolation extrapolation<br>$V_z$ = apparent volume of distribution<br>CL = Clearance |

Table 32 Group 2: 0.15 mg/kg LDP-02 IV

| Subject | $C_{max}$ ($\mu$g/mL) | $t_{max}$ (days) | $t_{\frac{1}{2}z}$ (days) | $AUC_{all}$ ($\mu$g·day/mL) | $\lambda_z$ (1/day) | AUC ($\mu$g·day/mL) | CL (mL/day/kg) | $V_z$ (mL/kg) |
|---|---|---|---|---|---|---|---|---|
| 101201 | 4.14 | 0.13 | 54.69 | 39.64 | 0.0127 | 58.58 | 2.56 | 202.06 |
| 102202 | 4.88 | 0.13 | 3.62 | 25.15 | 0.1914 | 25.78 | 5.82 | 30.39 |
| 305204 | 3.35 | 0.13 | 19.37 | 34.17 | 0.0358 | 44.23 | 3.39 | 94.77 |
| 402203 | 2.34 | 0.13 | 4.88 | 12.10 | 0.1420 | 13.72 | 10.94 | 77.03 |
| 403206 | 3.30 | 0.13 | 11.99 | 23.28 | 0.0578 | 25.70 | 5.84 | 100.99 |
| Mean | 3.602 | 0.13 | 18.91 | 26.868 | 0.0879 | 33.602 | 5.71 | 101.05 |
| SD | 0.9579 | 0 | 20.97 | 10.611 | 0.0757 | 17.718 | 3.27 | 62.87 |

Table 33 Group 3: 0.5 mg/kg LDP-02 IV

| Subject | $C_{max}$ ($\mu$g/mL) | $T_{max}$ (days) | $t_{\frac{1}{2}z}$ (days) | $AUC_{all}$ ($\mu$g·day/mL) | $\lambda_z$ (1/day) | AUC ($\mu$g·day/mL) | CL (mL/day/kg) | $V_z$ (mL/kg) |
|---|---|---|---|---|---|---|---|---|
| 206302 | 14.06 | 0.13 | 17.21 | 139.26 | 0.0403 | 149.44 | 3.35 | 83.08 |
| 208303 | 12.33 | 0.13 | 3.02 | 74.99 | 0.2293 | 75.73 | 6.60 | 28.79 |
| 309306 | 7.90 | 0.13 | 9.22 | 67.49 | 0.0751 | 68.82 | 7.27 | 96.69 |
| 502304 | 8.67 | 0.13 | 10.52 | 65.34 | 0.0659 | 69.59 | 7.19 | 109.09 |
| 503307 | 9.76 | 0.13 | 13.11 | 109.80 | 0.0529 | 134.20 | 3.73 | 70.48 |
| Mean | 10.544 | 0.13 | 10.616 | 91.376 | 0.0927 | 99.556 | 5.628 | 77.626 |
| SD | 2.582 | 0 | 5.229 | 32.207 | 0.0775 | 39.048 | 1.928 | 30.90 |

Table 34 Group 4: 2.0 mg/kg LDP-02 IV

| Subject | $C_{max}$ ($\mu$g/mL) | $t_{max}$ (days) | $t_{\frac{1}{2}z}$ (days) | $AUC_{all}$ ($\mu$g·day/mL) | $\lambda_z$ (1 day) | AUC ($\mu$g·day/mL) | CL (mL/day/kg) | $V_z$ (mL/kg) |
|---|---|---|---|---|---|---|---|---|
| 104403 | 32.18 | 2.00 | 17.92 | 510.32 | 0.0387 | 554.52 | 3.61 | 93.22 |
| 310415 | 37.66 | 0.13 | 16.72 | 626.06 | 0.0415 | 680.08 | 2.94 | 70.92 |
| 404401 | 29.71 | 0.13 | 18.34 | 525.63 | 0.0378 | 577.22 | 3.46 | 91.68 |

(continued)

| Subject | $C_{max}$ (µg/mL) | $t_{max}$ (days) | $t_{\frac{1}{2}z}$ (days) | $AUC_{all}$ (µg·day/mL) | $\lambda_z$ (1 day) | AUC (µg·day/mL) | CL (mL/day/kg) | $V_z$ (mL/kg) |
|---------|------------|------------|------------|------------|------------|------------|------------|------------|
| 506407 | 32.18 | 0.13 | 7.02 | 398.45 | 0.0988 | 399.06 | 5.01 | 50.75 |
| Mean | 32.933 | 0.13 | 15.0 | 515.12 | 0.0542 | 552.72 | 3.755 | 76.643 |
| SD | 3.360 | 0.935 | 5.364 | 93.19 | 0.0298 | 116.10 | 0.885 | 20.034 |

Serum $\alpha4\beta7$ Binding Over Time by Subject by Treatment Group. Data obtained from individual subjects are presented in Tables 35-40. For each subject the time of blood sampling, MESF of the sample and % of baseline (pre-dose) MESF is presented.

Table 35 Group 1: 0.15 mg/kg LDP-02 SC

| Time Days | Subject # 201101 | | Subject # 301103 | | Subject # 302105 | | Subject # 304107 | | Subject # 401104 | | Mean | |
|---|---|---|---|---|---|---|---|---|---|---|---|---|
| Pre-Dose | 10046 | 100% | 7326 | 100% | 12684 | 100% | 13117 | 100% | 3369 | 100% | 9308 | 100% |
| 0.125 | 951 | 9% | 762 | 10% | 1700 | 13% | 857 | 7% | 1105 | 33% | 1075 | 12% |
| 3 | 797 | 8% | 383 | 5% | 707 | 6% | 853 | 7% | 575 | 17% | 663 | 17% |
| 5 | 845 | 8% | 723 | 10% | | | 815 | 6% | 1052 | 31% | 859 | 9% |
| 10 | 675 | 7% | 717 | 10% | 862 | 7% | 865 | 7% | 941 | 28% | 812 | 9% |
| 14 | 4197 | 42% | 754 | 10% | 830 | 7% | 905 | 7% | 1058 | 31% | 1549 | 17% |
| 21 | 9610 | 96% | 803 | 11% | 834 | 7% | 3443 | 26% | 948 | 28% | 3128 | 34% |
| 30 | 9462 | 94% | 1142 | 16% | 1275 | 10% | 1587 | 12% | 1113 | 33% | 2916 | 31% |
| 60 | 9839 | 98% | 752 | 10% | 849 | 7% | 1262 | 10% | 2849 | 85% | 3110 | 33% |

Table 36 Group 2: 0.15 mg/kg LDP-02 IV

| Time Days | Subject # 101201 | | Subject # 102202 | | Subject # 305204 | | Subject # 402203 | | Subject # 403206 | | Mean | |
|---|---|---|---|---|---|---|---|---|---|---|---|---|
| Pre-Dose | 2588 | 100% | 2712 | 100% | 8394 | 100% | 10016 | 100% | 8342 | 100% | 6410 | 100% |
| 0.125 | 701 | 27% | 827 | 30% | 848 | 10% | 642 | 6% | 875 | 10% | 779 | 12% |
| 3 | 760 | 29% | 784 | 29% | 820 | 10% | 679 | 7% | 875 | 10% | 784 | 12% |
| 5 | 677 | 26% | 884 | 33% | 1012 | 12% | 639 | 6% | 859 | 10% | 814 | 13% |
| 10 | 671 | 26% | 753 | 28% | 943 | 11% | 690 | 7% | 856 | 10% | 783 | 12% |
| 14 | 1008 | 39% | 1515 | 56% | 1377 | 16% | 608 | 6% | 744 | 9% | 1050 | 16% |
| 21 | 953 | 37% | 4220 | 156% | 1860 | 22% | 2044 | 20% | 1606 | 19% | 2137 | 33% |
| 30 | 988 | 38% | 328 | 12% | 2332 | 28% | 3302 | 33% | 2560 | 31% | 1902 | 30% |
| 60 | 1680 | 65% | 3670 | 135% | 3275 | 39% | 6851 | 68% | 1168 | 14% | 3329 | 52% |

Table 37 Group 3: 0.5 mg/kg LDP-02 IV

| Time Days | Subject # 206302 | | Subject # 208303 | | Subject # 309306 | | Subject # 502304 | | Subject # 503307 | | Mean | |
|---|---|---|---|---|---|---|---|---|---|---|---|---|
| Pre-Dose | 3830 | 100% | 11267 | 100% | 5084 | 100% | 5615 | 100% | 9400 | 100% | 7039 | 100% |
| 0.125 | 1322 | 35% | 1577 | 14% | 887 | 17% | 879 | 16% | 1021 | 11% | 1137 | 16% |
| 3 | 1189 | 31% | 2012 | 18% | 914 | 18% | 775 | 14% | 982 | 10% | 1174 | 17% |
| 5 | 1054 | 28% | 1717 | 15% | 962 | 19% | 809 | 14% | 1147 | 12% | 1138 | 16% |
| 10 | 1195 | 31% | 2108 | 19% | 965 | 19% | 829 | 15% | 732 | 8% | 1166 | 17% |
| 14 | 1339 | 35% | 2405 | 21% | 1106 | 22% | 610 | 11% | 801 | 9% | 1252 | 18% |
| 21 | 1296 | 34% | 2085 | 19% | 671 | 13% | 636 | 11% | 733 | 8% | 1084 | 15% |
| 30 | 1483 | 39% | 1706 | 15% | 1203 | 24% | 860 | 15% | 611 | 7% | 1173 | 17% |
| 60 | 985 | 26% | 1038 | 9% | 1611 | 32% | 764 | 14% | 7611 | 81% | 2402 | 34% |

Table 38 Group 4: 2.0 mg/kg LDP-02 IV*

| Time Days | Subject # 104403 | | Subject # 210402 | | Subject # 310415 | | Subject # 404401 | | Subject # 506407 | | Mean | |
|---|---|---|---|---|---|---|---|---|---|---|---|---|
| Pre-Dose | 6714 | 100% | 5026 | 100% | 4642 | 100% | 4235 | 100% | 7418 | 100% | 5607 | 100% |
| 0.125 | 695 | 10% | 666 | 13% | 736 | 16% | 671 | 16% | 738 | 10% | 701 | 13% |
| 3 | 659 | 10% | 671 | 13% | 632 | 14% | 760 | 18% | 683 | 9% | 681 | 12% |
| 5 | 633 | 9% | 659 | 13% | 663 | 14% | 730 | 17% | 665 | 9% | 670 | 12% |
| 10 | 703 | 10% | 636 | 13% | 556 | 12% | 778 | 18% | 734 | 10% | 681 | 12% |
| 14 | 681 | 10% | 590 | 12% | 640 | 14% | 658 | 16% | 755 | 10% | 665 | 12% |
| 21 | 528 | 8% | 621 | 12% | 568 | 12% | 586 | 14% | 756 | 10% | 612 | 11% |
| 30 | 639 | 10% | 1218 | 24% | 599 | 13% | 682 | 16% | 740 | 10% | 776 | 14% |
| 60 | | | | | | | | | | | | |
| *No data for Subject # 505405 | | | | | | | | | | | | |

Table 39 Placebo Group

| Time Days | Subject # 202102 | | Subject # 303106 | | Subject # 103205 | | Subject # 306207 | | Subject # 308305 | | Subject # 501301 | |
|---|---|---|---|---|---|---|---|---|---|---|---|---|
| Pre-Dose | 7657 | 100% | 21074 | 100% | 4935 | 100% | 8070 | 100% | 15162 | 100% | 5274 | 100% |
| 0.125 | 5643 | 74% | 23312 | 111% | 4935 | 100% | 6837 | 85% | 15162 | 100% | 6424 | 122% |
| 3 | 8831 | 115% | 19528 | 93% | 4593 | 93% | 7162 | 89% | 13876 | 92% | 6022 | 114% |
| 5 | 7158 | 93% | 16567 | 79% | 4452 | 90% | 5044 | 63% | 13094 | 86% | 5530 | 105% |
| 10 | 7413 | 97% | 17575 | 83% | 5499 | 111% | 4750 | 59% | 14531 | 96% | 8201 | 155% |
| 14 | 6092 | 80% | 17827 | 85% | 3222 | 65% | 4169 | 52% | 10294 | 68% | 6740 | 128% |
| 21 | 8463 | 111% | 18048 | 86% | | | 4491 | 56% | 12700 | 84% | 7205 | 137% |
| 30 | 7353 | 96% | 15817 | 75% | 2317 | 47% | 11458 | 142% | 9328 | 62% | 5745 | 109% |
| 60 | 3385 | 44% | 11810 | 56% | | | 4771 | 59% | 9648 | 64% | 3262 | 62% |

EP 3 167 902 B1

Table 40 Placebo group

| Time Days | Subject # 209404 | | Subject # 505406 | | Mean | |
|---|---|---|---|---|---|---|
| Pre-Dose | 11012 | 100% | 7579 | 100% | 10095 | 100% |
| 0.125 | 11826 | 107% | 9025 | 119% | 10396 | 103% |
| 3 | 10549 | 96% | 8792 | 116% | 9919 | 98% |
| 5 | 11614 | 105% | 6217 | 82% | 8710 | 86% |
| 10 | 8238 | 75% | 7150 | 94% | 9170 | 91% |
| 14 | 8382 | 76% | 4787 | 63% | 7689 | 76% |
| 21 | 7031 | 64% | 7160 | 94% | 9300 | 92% |
| 30 | 6817 | 62% | 8166 | 108% | 8375 | 83% |
| 60 | | | | | | |

**Claims**

1. A humanized immunoglobulin or antigen-binding fragment thereof having binding specificity for α4β7 integrin, for use in treating inflammatory bowel disease, said use comprising administering the immunoglobulin or antigen-binding fragment thereof in multiple doses, each of the doses independently comprising up to 8 mg immunoglobulin or antigen-binding fragment per kg body weight, wherein the minimum interval between any two doses is a period of at least 7 days and the minimum interval before a subsequent dose is administered is a period of one month when a dose comprises 8 mg immunoglobulin or fragment thereof per kg body weight, and wherein said immunoglobulin or antigen-binding fragment has binding specificity for the α4β7 complex wherein said humanized immunoglobulin or antigen-binding fragment thereof comprises three complementarity determining regions (CDR1, CDR2 and CDR3) of a light chain variable region and three complementarity determining regions (CDR1, CDR2 and CDR3) of a heavy chain variable region of the amino acid sequence set forth below:

|  |  |
|---|---|
| light chain: | CDR1 SEQ ID NO:9 |
| | CDR2 SEQ ID NO:10 |
| | CDR3 SEQ ID NO:11 |
| heavy chain: | CDR1 SEQ ID NO:12 |
| | CDR2 SEQ ID NO:13 |
| | CDR3 SEQ ID NO:14, |

and wherein the immunoglobulin or fragment thereof is administered parenterally.

2. The humanized immunoglobulin or antigen-binding fragment thereof for use of claim 1, wherein said humanized immunoglobulin or antigen-binding fragment thereof comprises a heavy chain and a light chain, the light chain comprising complementarity determining regions derived from an antibody of nonhuman origin which binds α4β7 and a framework region derived from a light chain of human origin, wherein each of said complementarity determining regions (CDR1, CDR2 and CDR3) comprises the amino acid sequence set forth below:

light chain:    CDR1 SEQ ID NO:9

(continued)

CDR2 SEQ ID NO:10
CDR3 SEQ ID NO:11

the heavy chain comprising complementarity determining regions derived from an antibody of nonhuman origin which binds α4β7 and a framework region derived from a heavy chain of human origin, wherein each of said complementarity determining regions (CDR1, CDR2 and CDR3) comprises the amino acid sequence set forth below:

heavy chain:  CDR1 SEQ ID NO:12
CDR2 SEQ ID NO:13
CDR3 SEQ ID NO:14.

3. The humanized immunoglobulin or antigen-binding fragment thereof for use in claim 2, wherein said humanized immunoglobulin or antigen-binding fragment thereof comprises the heavy chain variable region of SEQ ID NO:6.

4. The humanized immunoglobulin or antigen-binding fragment thereof for use in claim 2 or 3, wherein said humanized immunoglobulin or antigen-binding fragment thereof comprises the light chain variable region of SEQ ID NO:8.

5. The humanized immunoglobulin or antigen-binding fragment thereof having binding specificity for α4β7 integrin, for use in any one of the preceding claims wherein each of said doses independently comprise an amount of immunoglobulin or fragment which is sufficient to achieve:

a) 50% or greater saturation of α4β7 integrin binding sites on circulating lymphocytes, or
b) 50% or greater inhibition of α4β7 integrin expression on the cell surface of circulating lymphocytes, or
c) 50% or greater of α4β7 integrin binding sites on circulating lymphocytes and 50% or greater inhibition of α4β7 integrin expression on the cell surface of circulating lymphocytes

wherein (i) said saturation is maintainable for a period of at least 10 days following administration of said doses; (ii) said inhibition is maintainable for a period of at least 10 days following administration of said doses; or (iii) said saturation and inhibition are each maintainable for a period of at least 10 days following administration of said doses.

6. The humanized immunoglobulin or antigen-binding fragment thereof for use in claim 5, wherein each of said doses independently comprise an amount of immunoglobulin or fragment which is sufficient to achieve and maintain said saturation and/or inhibition for a period of at least 14 days, or at least 20 days, or at least 25 days, or at least 30 days, or at least 60 days following administration of said dose.

7. The humanized immunoglobulin or antigen-binding fragment thereof for use in claim 5, wherein each of said doses comprises an amount of immunoglobulin which is sufficient to achieve and maintain a serum concentration of immunoglobulin of at least 1 ug/mL for a period of at least 10 days, or at least 14 days, or at least 20 days, or at least 25 days, or at least 30 days, or at least 60 days following administration of said dose.

8. The humanized immunoglobulin or antigen-binding fragment thereof for use in any preceding claim, wherein each of said doses independently comprise 0.1 to 8 mg immunoglobulin or antigen-binding fragment thereof per kg body weight.

9. The humanized immunoglobulin or antigen-binding fragment thereof for use in any preceding claim, wherein each of said doses independently comprise 0.15, 0.5, 1.0, 1.5 or 2.0 mg immunoglobulin or fragment per kg body weight.

10. The humanized immunoglobulin or antigen-binding fragment thereof for use in any preceding claim, wherein the interval between doses is at least 14 days, or at least 21 days, or at least 28 days, or at least 30 days.

11. The humanized immunoglobulin or antigen-binding fragment thereof for use in claim 1, wherein the inflammatory bowel disease is selected from ulcerative colitis, Crohn's disease, ileitis, Celiac disease, nontropical Sprue, enteropathy associated with seronegative arthropathies, microscopic or collagenous colitis, eosinophilic gastroenteritis, or pouchitis resulting after proctocolectomy, and ileoanal anastomosis.

12. The humanized immunoglobulin or antigen-binding fragment thereof for use in any of the preceding claims, wherein said interval between any two doses is a period of at least 14 days.

13. A humanized immunoglobulin or antigen-binding fragment thereof having binding specificity for α4β7 integrin, for use in maintenance therapy of inflammatory bowel disease wherein said use comprises administering said immunoglobulin or antigen-binding fragment thereof in doses and the minimum interval between any two doses is a period of at least 7 days, and wherein no more than 8 mg immunoglobulin or antigen-binding fragment thereof per kg body weight are administered during a period of 30 days,
wherein said humanized immunoglobulin or antigen-binding fragment thereof comprises three complementarity determining regions (CDR1, CDR2 and CDR3) of a light chain variable region and three complementarity determining regions (CDR1, CDR2 and CDR3) of a heavy chain variable region of the amino acid sequence set forth below:

| | |
|---|---|
| light chain: | CDR1 SEQ ID NO:9 |
| | CDR2 SEQ ID NO:10 |
| | CDR3 SEQ ID NO:11 |
| heavy chain: | CDR1 SEQ ID NO:12 |
| | CDR2 SEQ ID NO:13 |
| | CDR3 SEQ ID NO:14. |

14. The humanized immunoglobulin or antigen-binding fragment thereof for use in claim 13, wherein the interval between doses is at least 14 days, or at least 21 days, or at least 28 days, or at least 30 days.

15. The humanized immunoglobulin or antigen-binding fragment thereof for use in claim 13 or claim 14, wherein the interval between doses is at least 40, 50, 60, 70, 80, 90, 100, 110 or 120 days.

16. The humanized immunoglobulin or antigen-binding fragment thereof for use in any preceding claim wherein said humanized immunoglobulin or antigen-binding fragment thereof is administered subcutaneously or intravenously.

**Patentansprüche**

1. Humanisiertes Immunglobulin oder antigenbindendes Fragment davon, das eine Bindungsspezifität für a4β7-Integrin aufweist, zur Verwendung bei der Behandlung von entzündlichen Darmerkrankungen, wobei die Verwendung die Verabreichung des Immunglobulins oder antigenbindenden Fragments davon in Wiederholungsdosen umfasst, wobei jede der Dosen unabhängig voneinander bis zu 8 mg Immunglobulin oder antigenbindendes Fragment pro kg Körpergewicht umfasst, wobei das Mindestintervall zwischen zwei beliebigen Dosen ein Zeitraum von mindestens 7 Tagen ist und das Mindestintervall vor der Verabreichung einer nachfolgenden Dosis ein Zeitraum von einem Monat ist, wenn eine Dosis 8 mg Immunglobulin oder ein Fragment davon pro kg Körpergewicht umfasst, und wobei das Immunglobulin oder antigenbindende Fragment Bindungsspezifität für den a4β7 Komplex aufweist, wobei das humanisierte Immunglobulin oder antigenbindende Fragment davon drei komplementaritätsbestimmende Regionen (CDR1, CDR2 und CDR3) einer variablen Region einer leichten Kette und drei komplementaritätsbestimmende Regionen (CDR1, CDR2 und CDR3) einer variablen Region einer schweren Kette der unten dargelegten Aminosäuresequenz umfasst:

| | |
|---|---|
| Leichte Kette: | CDR1 SEQ ID NR:9 |
| | CDR2 SEQ ID NR:10 |
| | CDR3 SEQ ID NR:11 |
| Schwere Kette: | CDR1 SEQ ID NR:12 |
| | CDR2 SEQ ID NR:13 |
| | CDR3 SEQ ID NR:14, |

und wobei das Immunglobulin oder Fragment davon parenteral verabreicht wird.

2. Humanisiertes Immunglobulin oder antigenbindendes Fragment davon zur Verwendung nach Anspruch 1, wobei das humanisierte Immunglobulin oder antigenbindende Fragment davon eine schwere Kette und eine leichte Kette umfasst, wobei die leichte Kette komplementaritätsbestimmende Regionen, die von einem Antikörper nicht-humanen Ursprungs, der a4β7 bindet, abgeleitet sind, und eine Gerüstregion, die von einer leichten Kette menschlichen Ursprungs abgeleitet ist, umfasst, wobei jede der komplementaritätsbestimmenden Regionen (CDR1, CDR2 und CDR3) die unten dargestellte Aminosäuresequenz umfasst:

Leichte Kette:  CDR1SEQ ID NR:9
CDR2 SEQ ID NR:10
CDR3 SEQ ID NR:11

wobei die schwere Kette komplementaritätsbestimmende Regionen, die von einem Antikörper nicht-menschlichen Ursprungs abgeleitet sind, der a4β7 bindet, und eine Gerüstregion umfasst, die von einer schweren Kette menschlichen Ursprungs abgeleitet ist, wobei jede der komplementaritätsbestimmenden Regionen (CDR1, CDR2 und CDR3) die unten dargestellte Aminosäuresequenz umfasst:

Schwere Kette:  CDR1SEQ ID NR:12
CDR2 SEQ ID NR:13
CDR3 SEQ ID NR:14.

3. Humanisiertes Immunglobulin oder antigenbindendes Fragment davon zur Verwendung nach Anspruch 2, wobei das humanisierte Immunglobulin oder antigenbindende Fragment davon die variable Region der schweren Kette der SEQ ID NR:6 umfasst.

4. Humanisiertes Immunglobulin oder antigenbindendes Fragment davon zur Verwendung nach Anspruch 2 oder 3, wobei das humanisierte Immunglobulin oder antigenbindende Fragment davon die variable Region der leichten Kette von SEQ ID NR:8 umfasst.

5. Humanisiertes Immunglobulin oder antigenbindendes Fragment davon, das eine Bindungsspezifität für a4β7-Integrin aufweist, zur Verwendung nach einem der vorhergehenden Ansprüche, wobei jede der genannten Dosen unabhängig voneinander eine Menge an Immunglobulin oder Fragment umfasst, die ausreicht, um Folgendes zu erreichen:

a) 50 % oder größere Sättigung der a4β7-Integrin-Bindungsstellen auf zirkulierenden Lymphozyten, oder
b) 50 % oder größere Hemmung der a4β7-Integrin-Expression auf der Zelloberfläche von zirkulierenden Lymphozyten, oder
c) 50 % oder mehr der a4β7-Integrin-Bindungsstellen auf zirkulierenden Lymphozyten und 50 % oder größere Hemmung der a4β7-Integrin-Expression auf der Zelloberfläche der zirkulierenden Lymphozyten

wobei (i) die Sättigung für einen Zeitraum von zumindest 10 Tagen nach Verabreichung der Dosen aufrechterhaltbar ist; (ii) die Hemmung für einen Zeitraum von zumindest 10 Tagen nach Verabreichung der Dosen aufrechterhaltbar ist; oder (iii) die Sättigung und die Hemmung jeweils für einen Zeitraum von zumindest 10 Tagen nach Verabreichung der Dosen aufrechterhaltbar sind.

6. Humanisiertes Immunglobulin oder antigenbindendes Fragment davon zur Verwendung nach Anspruch 5, wobei jede der Dosen unabhängig voneinander eine Menge an Immunglobulin oder Fragment umfasst, die ausreicht, um die Sättigung und/oder Hemmung für einen Zeitraum von zumindest 14 Tagen oder zumindest 20 Tagen oder zumindest 25 Tagen oder zumindest 30 Tagen oder zumindest 60 Tagen nach Verabreichung der Dosis zu erreichen und aufrechtzuerhalten.

7. Humanisiertes Immunglobulin oder antigenbindendes Fragment davon zur Verwendung nach Anspruch 5, wobei jede der Dosen eine Menge an Immunglobulin umfasst, die ausreicht, um eine Serumkonzentration von Immunglobulin von zumindest 1 ug/ml für einen Zeitraum von zumindest 10 Tagen oder zumindest 14 Tagen oder zumindest 20 Tagen oder zumindest 25 Tagen oder zumindest 30 Tagen oder mindestens 60 Tagen nach Verabreichung der Dosis zu erreichen und aufrechtzuerhalten.

8. Humanisiertes Immunglobulin oder antigenbindendes Fragment davon zur Verwendung nach einem der vorhergehenden Ansprüche, wobei jede der genannten Dosen unabhängig voneinander 0,1 bis 8 mg Immunglobulin oder antigenbindendes Fragment davon pro kg Körpergewicht umfasst.

9. Humanisiertes Immunglobulin oder antigenbindendes Fragment davon zur Verwendung nach einem der vorhergehenden Ansprüche, wobei jede der genannten Dosen unabhängig voneinander 0,15, 0,5, 1,0, 1,5 oder 2,0 mg Immunglobulin oder Fragment pro kg Körpergewicht umfasst.

10. Humanisiertes Immunglobulin oder ein antigenbindendes Fragment davon zur Verwendung nach einem der vorhergehenden Ansprüche, wobei das Intervall zwischen den Dosen zumindest 14 Tage oder zumindest 21 Tage oder zumindest 28 Tage oder zumindest 30 Tage beträgt.

11. Humanisiertes Immunglobulin oder antigenbindendes Fragment davon zur Verwendung nach Anspruch 1, wobei die entzündliche Darmerkrankung aus Colitis ulcerosa, Morbus Crohn, Ileitis, Zöliakie, nicht-tropischer Sprue, Enteropathie, die mit seronegativen Arthropathien assoziiert ist, mikroskopischer oder kollagener Colitis, eosinophiler Gastroenteritis oder Pouchitis, die nach Proktokolektomie entsteht, und ileoanaler Anastomose ausgewählt ist.

12. Humanisiertes Immunglobulin oder ein antigenbindendes Fragment davon zur Verwendung nach einem der vorhergehenden Ansprüche, wobei das Intervall zwischen zwei beliebigen Dosen einen Zeitraum von zumindest 14 Tagen beträgt.

13. Humanisiertes Immunglobulin oder antigenbindendes Fragment davon mit Bindungsspezifität für a4β7-Integrin zur Verwendung in der Erhaltungstherapie von entzündlichen Darmerkrankungen, wobei die Verwendung die Verabreichung des Immunglobulins oder antigenbindenden Fragments davon in Dosen umfasst und das Mindestintervall zwischen zwei beliebigen Dosen einen Zeitraum von zumindest 7 Tagen umfasst, und wobei nicht mehr als 8 mg Immunglobulin oder antigenbindendes Fragment davon pro kg Körpergewicht während eines Zeitraums von 30 Tagen verabreicht werden, wobei das humanisierte Immunglobulin oder antigenbindende Fragment davon drei komplementaritätsbestimmende Regionen (CDR1, CDR2 und CDR3) einer variablen Region einer leichten Kette und drei komplementaritätsbestimmende Regionen (CDR1, CDR2 und CDR3) einer variablen Region einer schweren Kette der unten dargelegten Aminosäuresequenz umfasst:

| | | |
|---|---|---|
| Leichte Kette: | CDR1 | SEQ ID NR:9 |
| | CDR2 | SEQ ID NR:10 |
| | CDR3 | SEQ ID NR:11 |
| Schwere Kette: | CDR1 | SEQ ID NR:12 |
| | CDR2 | SEQ ID NR:13 |
| | CDR3 | SEQ ID NR:14. |

14. Humanisiertes Immunglobulin oder ein antigenbindendes Fragment davon zur Verwendung nach Anspruch 13, wobei das Intervall zwischen den Dosen zumindest 14 Tage oder zumindest 21 Tage oder zumindest 28 Tage oder mindestens 30 Tage beträgt.

15. Humanisiertes Immunglobulin oder ein antigenbindendes Fragment davon zur Verwendung nach Anspruch 13 oder 14, wobei das Intervall zwischen den Dosen zumindest 40, 50, 60, 70, 80, 90, 100, 110 oder 120 Tage beträgt.

16. Humanisiertes Immunglobulin oder antigenbindendes Fragment davon zur Verwendung nach einem der vorhergehenden Ansprüche, wobei das humanisierte Immunglobulin oder antigenbindende Fragment davon subkutan oder intravenös verabreicht wird.

**Revendications**

1. Immunoglobuline humanisée ou fragment de liaison à un antigène de celle-ci ayant une spécificité de liaison pour l'intégrine α4β7, pour une utilisation dans le traitement d'une maladie inflammatoire de l'intestin, ladite utilisation comprenant l'administration de l'immunoglobuline ou du fragment de liaison à un antigène de celle-ci en multi-dose, chacune des doses comprenant indépendamment jusqu'à 8 mg d'immunoglobuline ou de fragment de liaison à un antigène par kg de poids corporel, dans laquelle l'intervalle minimal entre deux doses quelconques est une période d'au moins 7 jours et l'intervalle minimal avant l'administration d'une dose suivante est une période d'un mois lorsqu'une dose comprend 8 mg d'immunoglobuline ou de fragment de celle-ci par kg de poids corporel, et dans laquelle ladite immunoglobuline ou ledit fragment de liaison à un antigène a une spécificité de liaison pour le complexe a4β7, dans laquelle ladite immunoglobuline humanisée ou ledit fragment de liaison à un antigène de celle-ci comprend trois régions de détermination de complémentarité (CDR1, CDR2 et CDR3) d'une région variable de chaîne légère et trois régions de détermination de complémentarité (CDR1, CDR2 et CDR3) d'une région variable de chaîne lourde de la séquence d'acides aminés indiquée ci-après :

chaîne légère :  CDR1 SEQ ID N° : 9
CDR2 SEQ ID N° : 10
CDR3 SEQ ID N° : 11
chaîne lourde :  CDR1 SEQ ID N° : 12
CDR2 SEQ ID N° : 13
CDR3 SEQ ID N° : 14,

et dans laquelle l'immunoglobuline ou le fragment de celle-ci étant administré par voie parentérale.

2. Immunoglobuline humanisée ou fragment de liaison à un antigène de celle-ci pour une utilisation selon la revendication 1, dans laquelle ladite immunoglobuline humanisée ou ledit fragment de liaison à un antigène de celle-ci comprend une chaîne lourde et une chaîne légère, la chaîne légère comprenant des régions de détermination de complémentarité dérivées d'un anticorps d'origine non humaine qui se lie à α4β7 et une région cadre dérivée d'une chaîne légère d'origine humaine, dans laquelle chacune desdites régions de détermination de complémentarité (CDR1, CDR2 et CDR3) comprend la séquence d'acides aminés indiquée ci-après :

chaîne légère :  CDR1 SEQ ID N° : 9
CDR2 SEQ ID N° : 10
CDR3 SEQ ID N° : 11

la chaîne lourde comprenant des régions de détermination de complémentarité dérivées d'un anticorps d'origine non humaine qui se lie à α4β7 et une région cadre dérivée d'une chaîne lourde d'origine humaine, dans laquelle chacune desdites régions de détermination de complémentarité (CDR1, CDR2 et CDR3) comprend la séquence d'acides aminés indiquée ci-après :

chaîne lourde :  CDR1 SEQ ID N° : 12
CDR2 SEQ ID N° : 13
CDR3 SEQ ID N° : 14

3. Immunoglobuline humanisée ou fragment de liaison à un antigène de celle-ci pour une utilisation selon la revendication 2, dans laquelle ladite immunoglobuline humanisée ou ledit fragment de liaison à un antigène de celle-ci comprend la région variable de chaîne lourde de SEQ ID N° : 6.

4. Immunoglobuline humanisée ou fragment de liaison à un antigène de celle-ci pour une utilisation selon la revendication 2 ou 3, dans laquelle ladite immunoglobuline humanisée ou ledit fragment de liaison à un antigène de celle-ci comprend la région variable de chaîne légère de SEQ ID N° : 8.

5. Immunoglobuline humanisée ou fragment de liaison à un antigène de celle-ci ayant une spécificité de liaison pour l'intégrine α4β7, pour une utilisation selon l'une quelconque des revendications précédentes, dans laquelle chacune desdites doses comprend indépendamment une quantité d'immunoglobuline ou de fragment qui est suffisante pour obtenir :

a) une saturation de 50 % ou plus de sites de liaison d'intégrine α4β7 sur des lymphocytes en circulation, ou
b) une inhibition de 50 % ou plus de l'expression d'intégrine α4β7 sur la surface cellulaire des lymphocytes en circulation, ou

c) 50 % ou plus de sites de liaison d'intégrine $\alpha4\beta7$ sur des lymphocytes en circulation et une inhibition de 50 % ou plus de l'expression d'intégrine $\alpha4\beta7$ sur la surface cellulaire des lymphocytes en circulation

dans laquelle (i) ladite saturation peut être maintenue pendant une période d'au moins 10 jours après l'administration desdites doses ; (ii) ladite inhibition peut être maintenue pendant une période d'au moins 10 jours après l'administration desdites doses ; ou (iii) lesdites saturation et inhibition peuvent chacune être maintenues pendant une période d'au moins 10 jours après l'administration desdites doses.

6. Immunoglobuline humanisée ou fragment de liaison à un antigène de celle-ci pour une utilisation selon la revendication 5, dans laquelle chacune desdites doses comprend indépendamment une quantité d'immunoglobuline ou de fragment qui est suffisante pour obtenir et maintenir lesdites saturation et/ou inhibition pendant une période d'au moins 14 jours, ou d'au moins 20 jours, ou d'au moins 25 jours, ou d'au moins 30 jours, ou d'au moins 60 jours après l'administration de ladite dose.

7. Immunoglobuline humanisée ou fragment de liaison à un antigène de celle-ci pour une utilisation selon la revendication 5, dans laquelle chacune desdites doses comprend une quantité d'immunoglobuline qui est suffisante pour obtenir et maintenir une concentration sérique d'immunoglobuline d'au moins 1 pg/ml pendant une période d'au moins 10 jours, ou d'au moins 14 jours, ou d'au moins 20 jours, ou d'au moins 25 jours, ou d'au moins 30 jours, ou d'au moins 60 jours après l'administration de ladite dose.

8. Immunoglobuline humanisée ou fragment de liaison à un antigène de celle-ci pour une utilisation selon l'une quelconque des revendications précédentes, dans laquelle chacune desdites doses comprend indépendamment 0,1 à 8 mg d'immunoglobuline ou de fragment de liaison à un antigène de celle-ci par kg de poids corporel.

9. Immunoglobuline humanisée ou fragment de liaison à un antigène de celle-ci pour une utilisation selon l'une quelconque des revendications précédentes, dans laquelle chacune desdites doses comprend indépendamment 0,15 ; 0,5 ; 1,0 ; 1,5 ou 2,0 mg d'immunoglobuline ou de fragment par kg de poids corporel.

10. Immunoglobuline humanisée ou fragment de liaison à un antigène de celle-ci pour une utilisation selon l'une quelconque des revendications précédentes, dans laquelle l'intervalle entre des doses est d'au moins 14 jours, ou d'au moins 21 jours, ou d'au moins 28 jours, ou d'au moins 30 jours.

11. Immunoglobuline humanisée ou fragment de liaison à un antigène de celle-ci pour une utilisation selon la revendication 1, dans laquelle la maladie inflammatoire de l'intestin est sélectionnée parmi la rectocolite hémorragique, la maladie de Crohn, l'iléite, la maladie cœliaque, la sprue non tropicale, l'entéropathie associée à des arthropathies séronégatives, la colite microscopique ou collagénique, la gastro-entérite éosinophile ou la pochite résultant d'une proctocolectomie et une anastomose iléo-anale.

12. Immunoglobuline humanisée ou fragment de liaison à un antigène de celle-ci pour une utilisation selon l'une quelconque des revendications précédentes, dans laquelle ledit intervalle entre deux doses quelconques est une période d'au moins 14 jours.

13. Immunoglobuline humanisée ou fragment de liaison à un antigène de celle-ci ayant une spécificité de liaison pour l'intégrine $\alpha4\beta7$, pour une utilisation dans une extension de traitement d'une maladie inflammatoire de l'intestin, dans laquelle ladite utilisation comprend l'administration de ladite immunoglobuline ou dudit fragment de liaison à un antigène de celle-ci en doses, et l'intervalle minimal entre deux doses quelconques est une période d'au moins 7 jours, et dans laquelle pas plus de 8 mg d'immunoglobuline ou de fragment de liaison à un antigène de celle-ci par kg de poids corporel sont administrés au cours d'une période de 30 jours, dans laquelle ladite immunoglobuline humanisée ou ledit fragment de liaison à un antigène de celle-ci comprend trois régions de détermination de complémentarité (CDR1, CDR2 et CDR3) d'une région variable de chaîne légère et trois régions de détermination de complémentarité (CDR1, CDR2 et CDR3) d'une région variable de chaîne lourde de la séquence d'acides aminés indiquée ci-après :

chaîne légère :   CDR1 SEQ ID N° : 9
                  CDR2 SEQ ID N° : 10
                  CDR3 SEQ ID N° : 11

(suite)

chaîne lourde : CDR1 SEQ ID N° : 12
CDR2 SEQ ID N° : 13
CDR3 SEQ ID N° : 14.

14. Immunoglobuline humanisée ou fragment de liaison à un antigène de celle-ci pour une utilisation selon la revendication 13, dans laquelle l'intervalle entre des doses est d'au moins 14 jours, ou d'au moins 21 jours, ou d'au moins 28 jours, ou d'au moins 30 jours.

15. Immunoglobuline humanisée ou fragment de liaison à un antigène de celle-ci pour une utilisation selon la revendication 13 ou la revendication 14, dans laquelle l'intervalle entre des doses est d'au moins 40, 50, 60, 70, 80, 90, 100, 110 ou 120 jours.

16. Immunoglobuline humanisée ou fragment de liaison à un antigène de celle-ci pour une utilisation selon l'une quelconque des revendications précédentes, dans laquelle ladite immunoglobuline humanisée ou ledit fragment de liaison à un antigène de celle-ci est administrée par voie sous-cutanée ou intraveineuse.

```
     ATGAAGTTGCCTGTTAGGCTGTTGGTGCTTCTGTTGTTCTGGATTCCTGTTTCCGGAGGT
1    ------------------------------------------------------------ 60
     TACTTCAACGGACAATCCGACAACCACGAAGACAACAAGACCTAAGGACAAAGGCCTCCA
     [M   K   L   P   V   R   L   L   V   L   L   L   F   W   I   P   V   S   G   G]

                          Signal Peptide

     GATGTTGTGGTGACTCAAACTCCACTCTCCCTGCCTGTCAGCTTTGGAGATCAAGTTTCT
61   ------------------------------------------------------------ 120
     CTACAACACCACTGAGTTTGAGGTGAGAGGGACGGACAGTCGAAACCTCTAGTTCAAAGA
     [D   V   V   V   T   Q   T   P   L   S   L   P   V   S   F   G   D   Q   V   S

                          Framework 1

     ATCTCTTGCAGGTCTAGTCAGAGTCTTGCAAAGAGTTATGGGAACACCTATTTGTCTTGG
121  ------------------------------------------------------------ 180
     TAGAGAACGTCCAGATCAGTCTCAGAACGTTTCTCAATACCCTTGTGGATAAACAGAACC
      I   S   C][R   S   S   Q   S   L   A   K   S   Y   G   N   T   Y   L   S][W

                   CDR 1

     TACCTGCACAAGCCTGGCCAGTCTCCACAGCTCCTCATCTATGGGATTTCCAACAGATTT
181  ------------------------------------------------------------ 240
     ATGGACGTGTTCGGACCGGTCAGAGGTGTCGAGGAGTAGATACCCTAAAGGTTGTCTAAA
      Y   L   H   K   P   G   Q   S   P   Q   L   L   I   Y][G   I   S   N   R   F

              Framework 2                                CDR 2

     TCTGGGGTGCCAGACAGGTTCAGTGGCAGTGGTTCAGGGACAGATTTCACACTCAAGATC
241  ------------------------------------------------------------ 300
     AGACCCCACGGTCTGTCCAAGTCACCGTCACCAAGTCCCTGTCTAAAGTGTGAGTTCTAG
      S][G   V   P   D   R   F   S   G   S   G   S   G   T   D   F   T   L   K   I

              Framework 3

     AGCACAATAAAGCCTGAGGACTTGGGAATGTATTACTGCTTACAAGGTACACATCAGCCG
301  ------------------------------------------------------------ 360
     TCGTGTTATTTCGGACTCCTGAACCCTTACATAATGACGAATGTTCCATGTGTAGTCGGC
      S   T   I   K   P   E   D   L   G   M   Y   Y   C][L   Q   G   T   H   Q   P

                                                          CDR 3

     TACACGTTCGGAGGGGGGACCAAGCTGGAAATAAAA
361  ------------------------------------
     ATGTGCAAGCCTCCCCCCTGGTTCGACCTTTATTTT
      Y   T][F   G   G   G   T   K   L   E   I   K]

                   Framework 4
```

FIG. 1

```
    ATGGGATGGAGCTGTATCATCCTCTTCTTGGTATCAACAGCTACAAGTGTCCACTCCCAG
1   ------------------------------------------------------------  60
    TACCCTACCTCGACATAGTAGGAGAAGAACCATAGTTGTCGATGTTCACAGGTGAGGGTC
    M   G   W   S   C   I   I   L   F   L   V   S   T   A   T   S   V   H   S] [Q
```

<div align="center">

Signal Peptide

</div>

```
    GTCCAACTGCAGCAGCCTGGGGCTGAGCTTGTGAAGCCTGGGACTTCAGTGAAGCTGTCC
61  ------------------------------------------------------------  120
    CAGGTTGACGTCGTCGGACCCCGACTCGAACACTTCGGACCCTGAAGTCACTTCGACAGG
    V   Q   L   Q   Q   P   G   A   E   L   V   K   P   G   T   S   V   K   L   S
```

<div align="center">

Framework 1

</div>

```
    TGCAAGGGTTATGGCTACACCTTCACCAGCTACTGGATGCACTGGGTGAAGCAGAGGCCT
121 ------------------------------------------------------------  180
    ACGTTCCCAATACCGATGTGGAAGTGGTCGATGACCTACGTGACCCACTTCGTCTCCGGA
    C   K   G   Y   G   Y   T   F   T] [S   Y   W   M   H] [W   V   K   Q   R   P
```

<div align="center">

CDR 1

</div>

```
    GGACAAGGCCTTGAGTGGATCGGAGAGATTGATCCTTCTGAGAGTAATACTAACTACAAT
181 ------------------------------------------------------------  240
    CCTGTTCCGGAACTCACCTAGCCTCTCTAACTAGGAAGACTCTCATTATGATTGATGTTA
    G   Q   G   L   E   W   I   G] [E   I   D   P   S   E   S   N   T   N   Y   N
```

<div align="center">

Framework 2                CDR 2

</div>

```
    CAAAAATTCAAGGGCAAGGCCACATTGACTGTAGACATTTCCTCCAGCACAGCCTACATG
241 ------------------------------------------------------------  300
    GTTTTTAAGTTCCCGTTCCGGTGTAACTGACATCTGTAAAGGAGGTCGTGTCGGATGTAC
    Q   K   F   K   G] [K   A   T   L   T   V   D   I   S   S   S   T   A   Y   M
```

<div align="center">

Framework 3

</div>

```
    CAGCTCAGCAGCCTGACATCTGAGGACTCTGCGGTCTACTATTGTGCAAGAGGGGGTTAC
301 ------------------------------------------------------------  360
    GTCGAGTCGTCGGACTGTAGACTCCTGAGACGCCAGATGATAACACGTTCTCCCCCAATG
    Q   L   S   S   L   T   S   E   D   S   A   V   Y   Y   C   A   R] [G   G   Y
```

```
    GACGGATGGGACTATGCTATTGACTACTGGGGTCAAGGCACCTCAGTCACCGTCTCCTCA
361 ------------------------------------------------------------  420
    CTGCCTACCCTGATACGATAACTGATGACCCCAGTTCCGTGGAGTCAGTGGCAGAGGAGT
    D   G   W   D   Y   A   I   D   Y] [W   G   Q   G   T   S   V   T   V   S   S]
```

<div align="center">

CDR 3                Framework 4

</div>

<div align="center">

FIG. 2

</div>

```
              10           20           30            40
   *      *        *      *     *      *       *       *        *
ATG AAA TGC ACC TGG GTC ATT CTC TTC TTG GTA TCA ACA GCT ACA AGT
 M   K   C   T   W   V   I   L   F   L   V   S   T   A   T   S>
                    Single peptide cleavage site
 50          60              70          80          90
  *       *      |  *       * | Spe I*    *      *       *       *
GTC CAC TCC|CAG GTC CAA CTA GTG CAG TCT GGG GCT GAG GTT AAG AAG
 V   H   S   Q   V   Q | L   V   Q   S   G   A   E   V   K   K
                       |
                       |_____
      100         110         120          130         140
    *       *       *       *       *       *       *       *   *
CCT GGG GCT TCA GTG AAG GTG TCC TGC AAG GGT TCT GGC TAC ACC TTC
 P   G   A   S   V   K   V   S   C   K   G   S   G   Y   T   F>
                                                           Xba I
      150         160         170         180          190
  *       *      *       *      *       *      *      *       *  |   *
ACC AGC TAC TGG ATG CAT TGG GTG AGG CAG GCG CCT GGC CAA CGT CTA
 T   S   Y   W   M   H   W   V   R   Q   A   P   G   Q   R|  L>
    *       200         210         220         230         240
  __*       *       *       *       *       *       *       *      *     *
GAG TGG ATC GGA GAG ATT GAT CCT TCT GAG AGT AAT ACT AAC TAC AAT
 E   W   I   G   E   I   D   P   S   E   S   N   T   N   Y   N>
              250         260         270         280    Nhe I
       *       *      *       *      *       *      *       *      *
CAA AAA TTC AAG GGA CGC GTC ACA TTG ACT GTA GAC ATT TCC|GCT AGC|
 Q   K   F   K   G   R   V   T   L   T   V   D   I   S | A   S> |
    290         300         310         320         330
  *       *      *       *      *       *      *       *      *      *
ACA GCC TAC ATG GAG CTC AGC AGC CTG AGA TCT GAG GAC ACT GCG GTC
 T   A   Y   M   E   L   S   S   L   R   S   E   D   T   A   V>
  340         350         360         370         380
      *       *      *       *      *      *      *      *      *
TAC TAT TGT GCA AGA GGG GGT TAC GAC GGA TGG GAC TAT GCT ATT GAC
 Y   Y   C   A   R   G   G   Y   D   G   W   D   Y   A   I   D>
  390             400  BstE II 410            420  ___Constant430
  *      *       *       *      *_____*      *      * |   *region*
TAC TGG GGT CAA GGC ACC CTG GTC ACC|GTC TCC TCA|GCC TCC ACC AAG
 Y   W   G   Q   G   T   L|  V   T | V   S   S | A   S   T   K>
          440         450          460         470         480
   *       *        *      *          *      *      *       *      *     *
GGC CCA TCG GTC TTC CCC CTG GCA CCC TCC TCC AAG AGC ACC TCT GGG
 G   P   S   V   F   P   L   A   P   S   S   K   S   T   S   G>
      490         500         510         520
      *       *      *      *       *      *       *      *    *Age I
GGC ACA GCG GCC CTG GGC TGC CTG GTC AAG GAC TAC TTC CCC GAA CCG
 G   T   A   A   L   G   C   L   V   K   D   Y   F   P   E|  P>
530          540
 *|      *      *
GTG ACG GTG TCG
 V|  T   V   S>
```

FIG. 3

57

```
                10              20              30              40
        *        *        *        *        *        *        *        *        *        *
ATG AAG TTG CCT GTT AGG CTG TTG GTG CTT CTG TTG TTC TGG ATT CCT
 M   K   L   P   V   R   L   L   V   L   L   L   F   W   I   P>
                        Signal peptide cleavage site
50      BspE I  60|              70              80              90
 *      |        *        *|      *        *        *        *        *        *        *
GTT|TCC GGA|GGT↓GAT GTT GTG ATG ACT CAA AGT CCA CTC TCC CTG CCT
 V | S   G |  G   D   V   V   M   T   Q   S   P   L   S   L   P>

        100             110             120             130             140
        *        *        *        *        *        *        *        *        *
GTC ACC CCT GGA GAA CCA GCT TCT ATC TCT TGC AGG TCT AGT CAG AGT
 V   T   P   G   E   P   A   S   I   S   C   R   S   S   Q   S>

        150             160             170             180 Asp 718    190
 *        *        *        *        *        *        *        *        *        *
CTT GCA AAG AGT TAT GGG AAC ACC TAT TTG TCT TGG TAC CTG CAG AAG
 L   A   K   S   Y   G   N   T   Y   L   S  |W   Y |L   Q   K>

            200             210             220             230             240
     *Msc I *        *        *        *        *        *        *        *        *
CC|T GGC CA|G TCT CCA CAG CTC CTC ATC TAT GGG ATT TCC AAC AGA TTT
 P   G   Q   S   P   Q   L   L   I   Y   G   I   S   N   R   F>

            250             260             270             280
        *        *        *        *        *        *        *        *        *
TCT GGG GTG CCA GAC AGG TTC AGT GGC AGT GGT TCA GGG ACA GAT TTC
 S   G   V   P   D   R   F   S   G   S   G   S   G   T   D   F>

        290             300 NruI        310             320             330
        *        *        *        *        *        *        *        *        *        *
ACA CTC AAG ATC|TCG CGA|GTA GAG GCT GAG GAC GTG GGA GTG TAT TAC
 T   L   K   I | S   R | V   E   A   E   D   V   G   V   Y   Y>

        340             350             360             370             380
        *        *        *        *        *        *        *        *        *
TGC TTA CAA GGT ACA CAT CAG CCG TAC ACG TTC GGA CAG GGG ACC AAG
 C   L   Q   G   T   H   Q   P   Y   T   F   G   Q   G   T   K>

        390             400             410 Kas I
 *        *        *        *        *        *
GTG GAA ATA AAA CGG GCT GAT GCG GCG CC|
 V   E   I   K   R   A   D   A| A   P>|
```

FIG. 4

Light Chain

CDR1        R S S Q S L A K S Y G N T Y L S

CDR2        G I S N R F S

CDR3        L Q G T H Q P Y T

Heavy Chain

CDR1        S Y W M H

CDR2        E I D P S E S N T N Y N Q K F K G

CDR3        G G Y D G W D Y A I D Y

FIG. 5

**Mean Pharmacokinetic Data: L297-007**

FIG. 6

Mean Serum LDP-02 Profiles
Phase Ib/IIa Study L297-006

FIG. 8

FIG. 9

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 4816567 A, Cabilly
- EP 0125023 B1
- US 4816397 A, Boss
- EP 0120694 B1
- WO 8601533 A, Neuberger, M.S.
- EP 0194276 B1
- US 5225539 A
- EP 0239400 B1
- EP 0451216 B1
- EP 0519596 A1
- US 4946778 A, Ladner
- US 5476786 A, Huston
- US 4172124 A, Koprowski
- WO 9824893 A, Abgenix
- US 5939598 A, Kucherlapate, R. and Jakobovits, A.
- US 5545806 A, Lonberg
- US 5545807 A, Surani
- WO 9713852 A, Lonberg
- GB 2209757 B, Winter
- WO 8907142 A, Morrison
- WO 9429351 A, Morgan
- WO 9404679 A, Carter
- WO 9806248 A
- US 70073796
- US 5514548 A, Krebber
- WO 9306213 A, Hoogenboom
- US 0112234 W
- US 09748960 B
- US 09550082 B

## Non-patent literature cited in the description

- **CARLOS, T.M. ; HARLAN, J.M.** *Blood,* 1994, vol. 84, 2068-2101
- **BERLIN, C. et al.** *Cell,* 1993, vol. 74, 185-195
- **ERLE, D.J. et al.** *J. Immunol.,* 1994, vol. 153, 517-528
- **STREETER, P.R. et al.** *Nature,* 1988, vol. 331, 41-46
- **SCHWEIGHOFFER, T. et al.** *J. Immunol.,* 1993, vol. 151, 717-729
- **PODOLSKY.** *New Engl. J. Med.,* 1991, vol. 325, 928-937
- **PODOLSKY.** *New Engl. J. Med.,* 1991, vol. 325, 1008-1016
- **GORDON F. H. et al.** *Gastroenterology,* April 1999, vol. 116 (4), A726
- **LAZAROVITS, A.I. et al.** *J. Immunol.,* 1984, vol. 133, 1857-1862
- **KABAT, E.A. et al.** Sequences of Proteins of Immunological Interest. U.S. Department of Health and Human Services, 1991
- Cellular and Molecular Immunology. W. B. Saunders Co, 1991, 41-50
- **NISONOFF, A.** Introduction to Molecular Immunology. Sinauer Associates, Inc, 1984, 45-65
- **BIRD, R.E. et al.** *Science,* 1988, vol. 242, 423-426
- **LAZAROVITS, A.I. et al.** *J. Immunol.,* 1984, vol. 133 (4), 1857-1862
- **KOHLER et al.** *Nature,* 1975, vol. 256, 495-497
- **HARLOW et al.** Antibodies: A Laboratory Manual. Cold Spring Harbor, 1988
- Current Protocols in Molecular Biology. John Wiley & Sons, 1991, vol. 2
- *Eur. J. Immunol.,* 1976, vol. 6, 511-519
- **MILSTEIN et al.** *Nature,* 1977, vol. 266, 550-552
- **HARLOW, E. ; D. LANE.** Antibodies: A Laboratory Manual. Cold Spring Harbor Laboratory, 1988
- Current Protocols In Molecular Biology. John Wiley & Sons, 1991, vol. 2
- **JAKOBOVITS et al.** *Proc. Natl. Acad. Sci. USA,* 1993, vol. 90, 2551-2555
- **JAKOBOVITS et al.** *Nature,* 1993, vol. 362, 255-258
- **KETTLEBOROUGH, C.A. et al.** *Protein Engineering,* 1991, vol. 4, 773-783
- **KAMMAN, M. et al.** *Nucl. Acids Res.,* 1989, vol. 17, 5404
- **SATO, K. et al.** *Cancer Research,* 1993, vol. 53, 851-856
- **KOLBINGER, F.** *Protein Engineering,* 1993, vol. 8, 971-980
- **KETTLEBOROUGH, C.A.** *Protein Engineering,* 1991, vol. 4, 773-783
- **HANNINEN, A.** *J. Clin. Invest.,* 1993, vol. 92, 2509-2515

- **HANNINEN, A. et al.** *J. Clin. Invest.,* 1993, vol. 92, 2509-2515
- Remington's Pharmaceutical Sciences. Mack Publishing Company
- **BAKER et al.** Controlled Release of Biological Active Agents. John Wiley and Sons, 1986
- *Br Med J,* 1955, vol. 2, 1042-1048